# NEUE EUROPÄISCHE PATENTSCHRIFT

(11) **EP 1 976 479 B2**
(45) Veröffentlichungstag und Bekanntmachung des Hinweises auf die Entscheidung über den Einspruch: **02.10.2019**
(45) Hinweis auf die Patenterteilung: 16.11.2011
(21) Anmeldenummer: 07702603.7
(22) Anmeldetag: 05.01.2007
(51) Int. Cl.: A61K 6/10

(54) **DENTALABFORMMASSEN UND DARAUS HERGESTELLTE GEHÄRTETE PRODUKTE**
DENTAL IMPRESSION MATERIALS, CURED PRODUCTS MADE THEREFROM, AND USE OF SURFACTANTS FOR PRODUCING DENTAL IMPRESSION MATERIALS
MATIÈRES DE MOULAGE DENTAIRE, PRODUITS DURCIS RÉALISÉS À PARTIR DE CES MATIÈRES ET UTILISATION DE TENSIOACTIFS POUR FABRIQUER DES MATIÈRES DE MOULAGE DENTAIRE

(30) Priorität: 09.01.2006 DE 102006001126
(43) Veröffentlichungstag der Anmeldung: 08.10.2008
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 58540 Meinerzhagen (DE)
(74) Vertreter: Keil & Schaafhausen Patent- und Rechtsanwälte PartGmbB
(86) Internationale Anmeldenummer: PCT/EP2007/000066
(87) Internationale Veröffentlichungsnummer: WO 2007/080071

(56) Entgegenhaltungen:
- EP-A2- 0 613 926
- WO-A1-87/03001
- US-A- 4 657 959
- NORLING BARRY K ; BRUKL CHARLES E: "SURFACE WETTABILITY MODIFICATION OF POLY(VINYLSILOXANES) WITH NONIONIC SURFACTANTS" COLLOIDS AND SURFACES, Bd. 20, Nr. 4, 1986, Seiten 277-288, XP002451249

## Beschreibung

Beschrieben werden neue Dentalabformmassen, die sich durch ein verbessertes Benetzungsverhalten auszeichnen.

Dentalabformmassen sind an sich bekannt und werden bereits seit langem eingesetzt. Diese Massen müssen eine Vielzahl von Eigenschaften aufweisen, wie rasches Abbindeverhalten, ausgezeichnete Detailgenauigkeit und gleichzeitig gute Lagerfähigkeit der Vorprodukte. Üblicherweise werden Zweikomponenten-Mischungen eingesetzt, die unmittelbar vor dem Gebrauch miteinander vermischt werden und die sodann rasch verarbeitet werden müssen. Für das Erzielen einer möglichst guten Detailgenauigkeit ist es wichtig, dass die zwischen Zahn bzw. Zahnfleisch einerseits und dem sich ausbildenden Abdruck andererseits möglichst wenig Flüssigkeit, wie Speichel oder Blut, befindet und dass diese Flüssigkeit möglichst wenig in den sich ausbildenden Formkörper aufgenommen wird.

Dentalabformmassen enthalten üblicherweise hydrophobe Materialien, wie Polyorganosiloxane. Zum Minimieren der Flüssigkeitsschicht zwischen Abdruck und Zahn bzw. Zahnfleisch hat man bereits den Einsatz von Tensiden in Dentalabdruckmassen vorgeschlagen. Diese bewirken ein Verteilen des Wasserfilms und daher wird die Ausbildung eines Flüssigkeitsfilms erschwert.

Aus der US-A-4,657,959 ist eine aushärtbare und als dentales Abformmaterial einsetzbare Siliconzusammensetzung bekannt, welche ein aushärtbares Siliconprepolymer und ein Tensid enthält. Bei dem einzusetzenden Tensid handelt es sich vorzugsweise um ein ethoxyliertes nichtionisches oberflächenaktives Agens, enthaltend eine oder mehrere Siloxan- oder Perfluoralkylgruppen. Das Tensid wird in einer Menge eingesetzt, daß die Zusammensetzung drei Minuten nach Aufgabe des Wassertropfens auf die ausgehärtete Zusammensetzung besonders bevorzugt einen Kontaktwinkel kleiner 10° aufweist. Diese Kontaktwinkeleigenschaften an einem bereits ausgehärteten Abformmaterial nach drei Minuten entsprechen aber nicht den Praxisanforderungen. Weiterhin sind in den Beispielen dieser US-A-4,657,959 amphotere oder ionische Fluortenside aufgeführt und im Falle der nichtionischen Fluortenside sind die Perfluoralkylgruppen über eine SO₂NR-Gruppe mit den Polyethergruppen verknüpft. Diese SO₂NR-Gruppen sind für einen Einsatz in einem Platin-katalysierten additionsvernetzenden Silicon ungeeignet, da sie starke Metall-Chelat-Komplexe am Platinkatalysator ausbilden und diesen auf diese Weise inhibieren. Darüber hinaus ist in der US-A-4,657,959 weder der Zusatz eines nicht reaktiven Polyetherpolymers noch eines reaktiv einpolymerisierbaren Polyetherpolymers, z.B. mit Vinyl-, Allyl- oder SiH-Gruppen, offenbart.

In der EP-A-1,290,998 wird eine additionsvernetzende Siliconabformmassen-Zusammensetzung beschrieben, die ein Organopolysiloxan mit wenigstens zwei aliphatisch gesättigten Kohlerwasserstoffgruppen, einem Polyether mit wenigstens einer Alkylgruppe, ein Organohydrogensiloxan, einen löslichen Platinkatalysator, einen anorganischen Füllstoff, sowie ein nichtionisches Tensid und/oder ein Polyether-modifiziertes Siliconöl enthält.

Die EP-A-847,745 beschreibt Dentalsiliconabdruckmaterialien, die neben Siliconpolymeren mit SiH-Gruppen und Siliconpolymeren mit Si-Alkenylgruppen, Katalysator und anorganischen Füllstoffen ein Polysiloxan-Polyester-Polymer mit mindestens einem auf Dimethicon basierenden Polyalkylenoxidsubstituenten und mit einem fluorierten Alkylsubstituenten enthalten. Gemäß dieser Offenbarung wird mit einem ausgehärteten, festen Probekörper ein Kontaktwinkel von weniger als 73° über die Wilhelmi-Methode bestimmt. Bei dem eingesetzten fluorhaltigen Polysiloxan-Polyester handelt es sich um ein hochmolekulares Polymer, an dessen Kettenenden jeweils fluorierte Kohlenwasserstoffe sitzen. Die damit erzielbaren Kontaktwinkel sind eher gering. Das fluorierte Polysiloxan-Polyester stellt aufgrund der polymeren Struktur, der hohen Molmasse der Polymere und der an beiden Kettenenden sitzenden Fluorkohlenwasserstoffgruppen kein klassisches niedermolekulares Tensid mit einem hydrophilen Kopf und einem hydrophoben Schwanz dar.

Die WO-A-2004/058,196 beschreibt dentale Abdruckmaterialien umfassend ein Polyvinylsiloxan und ein Tensid, wobei das Tensid der Zusammensetzung eine derartige Benetzbarkeit verleiht, daß das Material 15 Minuten nach dem Aushärten nach ungefähr 15 Sekunden einen Oberflächenkontaktwinkel mit Wasser von weniger als ungefähr 10° aufweist. Insbesondere werden diese Kontaktwinkel durch Einsatz des Tensides PEG8-Methicone erreicht.

Die EP-A-1,165,016 beschreibt ein additionsvernetzendes Abformmaterial auf Siliconbasis mit einem Polyalkylenoxid, und/oder dessen Derivate mit einer Molmasse > 3000 g/mol mit einer Konzentration zwischen 0,001 bis 1,0 Gew.%. Der eingesetzte Polyether soll die Standfestigkeit des Abformmaterials verbessern. Gleichzeitig wurden die Kontaktwinkel mit > 80° bestimmt. Aus dieser Druckschrift ist zu entnehmen, daß nur durch einen Polyetherzusatz keine guten Kontaktwinkel und damit keine guten Hydrophilieeigenschaften zu erzielen sind.

EP-A-729,341 offenbart zur Hydrophilierung von Zahnabdruckmassen den Einsatz von Polyethercarbosilanen. Die Kontaktwinkel-/Randwinkelmessung erfolgt 30 Minuten nach Aushärtung des Abformmaterials und die Kontaktwinkel liegen bei minimal 42°.

Die EP-A-613,926 beschreibt Polyetherabformmaterialien, die wenigstens ein hydrophiles Mittel aus der Gruppe bestehend aus hydrophilen Silikonölen, fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern enthalten. Die Kontaktwinkelmessung erfolgt 30 Minuten nach Aushärtung des Abformmaterials und die Kontaktwinkel liegen zwischen 18 und 65°.

Aus der WO-A-00/48,553 ist eine additionsvernetzende Siliconabformmaterial-Zusammensetzung bekannt, die einen einpolymerisierbaren Siliconpolyether mit kammartigen Polyethergruppen enthält. In den Beispielen wurden Kombinationen dieses einpolymerisierbaren Siliconpolyethers mit Nonylphenylethoxylat-Tensiden beschrieben.

EP-A-231,420 offenbart ein additionsvernetzendes Siliconabformmaterial mit einem Siliconpolyether.

DE-A-4,010,281 offenbart additionsvernetzende Polyetherabformzusammensetzungen, bei denen der Polyether endständige Vinyldimethylsiloxygruppen oder Allylgruppen enthält. Die über eine SiOC-Bindung an dem Polyether gebundenen ungesättigten Gruppen sind hydrolyseanfällig und nicht lagerstabil. Im anderen Fall sind die Polyether durch Pt-katalysierte Hydrosilylierung mit Vinyldimethylsiloxangruppen verknüpft. Der hochaktive Katalysator kann nicht durch gebräuchliche ständige Reinigungsmethoden abgetrennt werden. Selbst eine Reinigung des Polymers über eine Hochvakuumdestillation ist aufgrund der hohen Molmassen des Polymers und Pt-Katalysators nicht möglich. Die auf diese Weise synthetisierten und mit Restplatin verunreinigten Polyetherpolymere sind nicht lagerstabil und somit für eine Dentalabformmassenzusammensetzung ungeeignet. Aus diesem Grund erfolgte bis zum heutigen Tag keine Kommerzialisierung dieser Produkte.

US-A-5,064,891 beschreibt eine additionsvernetzende Siliconzusammensetzung mit einem Silicontensid. Die Kontaktwinkelmessungen wurden an ausgehärteten Probekörpern und drei Minuten nach Aufbringen der Wassertropfen durchgeführt. Die beschriebenen Kontaktwinkel liegen zwischen 60 und 65°.

EP-885,932 beschreibt additionsvernetzende Organopolysiloxanzusammensetzungen mit einem hydrophilen ungesättigten Polysiloxan-Polyether-Copolymer mit 2 bis 5 Siliciumatomen und mindestens einer aliphatischen ungesättigten Funktionalität und mindestens einer Polyether-Funktionalität.

Aus der US-A-5,907,002 ist ein additionsvernetzendes Abformsiliconmaterial beschrieben, das in der Kombination eines nichtionischen Tensids mit einem Methylphenylpolysiloxan formuliert wird. Das nichtionische Tensid kann neben der hydrophilen Gruppe eine lipophile Gruppe haben, wobei diese eine Alkylgruppe oder eine Fluorcarbon-Gruppe sein kann. Der Einsatz von Mischungen aus Silicontensid mit Fluortensid wird nicht offenbart. Die erreichten Kontaktwinkel liegen zwischen 28 und 60°.

Kombinationen von Fluortensiden und Silicontensiden sind aus der DE 699 17 384 T2 (entsprechend EP-B-974,626) bekannt. Dieses Dokument beschreibt wässrige pigmentierte Tintenstrahldrucktinten enthaltend solche Tensidgemische.

Aus der DE-A-199 22 929 schließlich sind härtbaren dentale Abformmaterialien enthaltend Tenside oder Kombinationen von Tensiden bekannt. Diese Abformmaterialien sind für die Abdrucknahme der Mundschleimhaut bestimmt und fließen bei sehr geringem Druck; sie fließen jedoch nicht, wenn kein Druck angewendet wird. Das Abdruckmaterial soll auf die Mundschleimhaut nur eine sehr geringe Reizung ausüben. Der Einsatz von Gemischen aus Silicontensiden und Fluortensiden wird nicht offenbart.

US-A-6,861,457 beschreibt hydrophile dentale Abformaterialien auf der Basis additionsvernetzender Polysiloxane. Diese enthalten neben einem Polyether mit ungesättigten Gruppen ein nichtionisches Tensid und/oder ein Polyether-modifiziertes Siliconöl. Kombinationen von Fluortensiden mit Silicontensiden werden nicht beschrieben.

In der DE-A-43 06 997 werden hydrophilierte Polyether offenbart. Diese können unterschiedlichste Tenside enthalten; als mögliche Substanzklassen werden neben anderen Tensiden hydrophile Silikonöle oder fluorierte Kohlenwasserstoffverbindungen offenbart. In den Beispielen werden härtbare Zusammensetzungen enthaltend mit Aziridinogruppen verkappte Polyether und ein nichtionisches Fluortensid (Fluorad FC430) beschrieben. Diese Verbindung enthält gemäß dem 3M Material Safety Data Sheet, Ausgabe 30.08.2002, Perfluoroctylsulfonatgruppen. Härtbare Zusammensetzungen enthaltend Gemische aus Silicontensiden und Fluortenside mit (Poly)alkylenoxidresten, Kohlenhydratresten oder aliphatischen Polyhydroxyresten werden in DE-A-43 06 997 nicht offenbart.

EP-B-244,478 beschreibt die Verwendung von hydrophilen Silikonen als Zahnabdruckmaterialien. In diesem Dokument wird unter anderem der Einsatz von Netzmitteln aus ethoxylierten nicht ionischen grenzflächenaktiven Stoffen mit solubilisierenden Siloxan- oder Perfluoralkylresten offenbart. Härtbare Zusammensetzungen enthaltend Gemische aus Silicontensiden und Fluortenside mit (Poly)alkylenoxidresten, Kohlenhydratresten oder aliphatischen Polyhydroxyresten werden auch in diesem Dokument nicht beschrieben.

WO-A-2005/016289 beschreibt Dentalabformmassen auf der Basis von Polyorganosiloxanen. Diese zeichnen sich durch die Anwesenheit eines Netzmittels aus, welches die Benetzbarkeit der Masse durch Wasser verbessert, so dass sich nach drei Minuten ein Kontaktwinkel von weniger als 50° einstellt. Als Netzmittel werden beispielsweise ethoxylierte Nonylphenole oder PEG-8 Methicone vorgeschlagen.

In den genannten Patentdokumenten werden die Kontaktwinkelmessungen nicht auf die Praxisanforderungen bezogen ausgeführt, d. h. die Kontaktwinkel werden an ausgehärteten Probekörpern gemessen. Die Praxisanforderung aber sieht so aus, daß zum einen nicht die Kontaktwinkeleigenschaften des ausgehärteten Abformmaterials relevant, sondern ganz im Gegenteil die Kontaktwinkeleigenschaften im nicht ausgehärteten plastischen Zustand entscheidend sind und zum anderen die Kontaktwinkeleigenschaften nicht erst nach einer Zeitspanne von 3 Minuten relevant sind, sondern sofort bei der Abdrucknahme, d. h. zwischen größer 0 Sekunden und kleiner 10 Sekunden nach dem initialen Kontakt zwischen dem plastischen Abformmaterial und der Zahnsubstanz bzw. der Mundschleimhaut. Dies erklärt sich dadurch, daß das Abformmaterial bei der Zahnabdrucknahme während der Verarbeitungszeit, also im noch nicht ausgehärteten Zustand, in der plastischen Phase initial, d.h. zwischen > 0 und < 10 s, die feuchte mit Speichel benetzte Zahnsubstanz und Mundschleimhaut benetzen und unmittelbar anfließen muß. Alles was sich zeitlich danach an Kontaktwinkeleigenschaften einstellt, ist für eine detailgetreue Abdrucknahme somit nicht mehr relevant.

In der unter Praxisbedingungen für das Anfließen des Abformmaterials an den feuchten Zahn wichtigen Phase nach Mischen der beiden Komponenten, in der sogenannten Verarbeitungszeit, in der das Abdruckmaterial noch plastisch verformbar ist und mit feuchten Zähnen, Speichel und Blut in Kontakt kommt, weisen die im vorstehend beschriebenen Stand der Technik offenbarten Materialien keine guten Kontaktwinkel von <=10° auf. Ein Spreiten des Wassertropfens auf der Materialoberfläche ist nicht zu beobachten. Die in diesen Dokumenten beschriebenen Kontaktwinkel sind lediglich beim Ausgießen der ausgehärteten Abformung außerhalb des Mundes mit flüssigem Gipsbrei zur Modellerstellung relevant, jedoch nicht bei der entscheidenden Abdrucknahme im Mund. Ferner beschreibt keines dieser Dokumente den Einsatz einer synergistisch wirkenden Tensidmischung oder einer Kombination dieser Mischung mit einem Polyether.

Eine sehr aktuelle und wissenschaftliche Untersuchung in Deutsche Zahnärztliche Zeitschrift 60 (2005) 10, S. 587-592 von Rupp et al. beschreibt die Anforderungen, die an ein Dentalabformmaterial hinsichtlich Benetzungsverhalten gestellt werden.

Hier wurden Untersuchungen zur initialen Hydrophilie und zur Gleichgewichtshydrophilie durchgeführt. Als Praxisanforderung ergibt sich daraus, dass zu jedem Zeitpunkt während der plastischen Phase der Verarbeitungszeit das Abformmaterial eine niedrige initiale Hydrophilie und zusätzlich eine zu jedem Zeitpunkt gleich niedrige Gleichgewichts-Hydrophilie aufweisen soll.

Das Applizieren und Anfließen des Abformmaterials im Patientenmund erfolgt unter Praxisbedingungen zu verschiedenen Zeitpunkten, abhängig von der Abformtechnik und von der Anzahl der abzuformenden Zähne. So ist z.B. bei einer Einzelkronenabformung zum Applizieren und Anfließen eine relativ kurze Zeit, z.B. 40 Sekunden, erforderlich, während bei umfangreicheren Inlets mit 4 oder 5 abzuformenden Zähnen das Applizieren und Anfließen erst nach 2 Minuten erfolgt ist.

Nach der Untersuchung von Rupp et al. weisen die heute verwendeten Abformmaterialien, vor allem die Siliconabfommaterialien, eine starke Drift in hydrophober Richtung bei zunehmender Verarbeitungszeit auf, was gegen die o.g. Praxisbedürfnisse und Praxisanforderungen der Abformmaterials läuft.

Weiterhin sind die dort gemessenenen initialen Hydrophilie-Kontaktwinkel sowohl bei den Polyether- bzw. Siliconabformmaterialen ebenfalls verbesserungswürdig.

Ausgehend von diesem Stand der Technik war es Aufgabe der vorliegenden Erfindung eine Dentalabdruckmasse bereitzustellen, die zu jedem Zeitpunkt während der Verarbeitungszeit (sowohl am Anfang als auch am Ende) einen niedrigen initialen Kontaktwinkel und einen konstanten und niedrigen Gleigewichts-Kontaktwinkel (Hydrophile) liefert, so dass unter Praxisbedingungen ein außerordentlich gutes Anfließen an den feuchten Zahn bzw. Zahngewebe erfolgt und dies wiederum zur Ausbildung eines äußerst detailgetreuen Abdrucks führt.

Es wurde in überraschender Weise gefunden, dass der Einsatz einer synergistischen Mischung ausgewählter Tenside zu einer initialen Spreitung des Wasserfilms zwischen Zahn bzw. Zahnfleisch und noch plastischer Formmasse führt und dass sich dadurch sehr detailgetreue Abdrücke herstellen lassen.

Die vorliegende Erfindung betrifft eine Dentalabformmasse enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzenden Esterreste einer ethylenisch ungesättigten Carbonsäure enthaltenden Polyether oder der durch ringöffnende Metathesereaktion vernetzenden Polyether, Silicone oder Kautschuke, und enthaltend ferner ein mindestens einen (Poly)alkylenoxidrest sowie eine Silizium enthaltende Gruppe aufweisendes nichtionisches Tensid mit einer Molmasse von weniger als 6000 g/mol (nachstehend auch Silizium aufweisendes Tensid), und ein nichtionisches Fluortensid-, (nachstehend auch Fluortensid genannt). Als nichtionisches Fluortensid wird eine Verbindung gemäß Anspruch 1 eingesetzt.

Bevorzugte Dentalabformmassen weisen 40 Sekunden nach Mischbeginn, vorzugsweise zu jedem Zeitpunkt während der Verarbeitungszeit zwischen >0 und 3 Minuten, einen niedrigen initialen Wassertropfen-Kontaktwinkel von < 10° auf (gemessen bei 50 % Luftfeuchtigkeit in der Klimakammer) und bei einem Tropfenalter von 10 Sekunden.

Der dynamische Verlauf des Wassertropfen-Kontaktwinkels bevorzugter Dentalabformmassen, gemessen 40 Sekunden nach Mischbeginn, nimmt vorzugsweise folgende Werte an: nach einem Tropfenalter von 0,25 Sekunden einen Wassertropfen-Kontaktwinkel von <75°, vorzugsweise von <40°; nach einem Tropfenalter von 0,5 Sekunden einen Wassertropfen-Kontaktwinkel von <55°, vorzugsweise <30°; nach einem Tropfenalter von 1 Sekunde einen Wassertropfen-Kontaktwinkel von <35°, vorzugsweise < 25°; nach einem Tropfenalter von 2 Sekunden: einen Wassertropfen-Kontaktwinkel von <20°; und nach einem Tropfenalter von 3 Sekunden einen Wassertropfen-Kontaktwinkel von <10°.

Auf der noch nicht ausgehärteten erfindungsgemäßen Dentalabformmasse spreitet ein Wassertropfen und bildet nach kurzer Zeit, typischerweise nach spätestens 3 Sekunden, einen Tropen mit einem sehr geringen Kontaktwinkel von < 10° aus oder er verläuft vollständig unter Ausbildung eines Wasserfilms.

Die erfindungsgemäßen Dentalabformmassen sind durch den Gehalt einer synergistischen Mischung ausgewählter Tenside gekennzeichnet.

Fluortenside sind die folgenden Verbindungen der Formeln Ic

F-(CF₂-CF₂)₁₋₇-CH₂-CH₂-(O-CH₂-CH₂)₁₋₁₅-OH (Ic)

Nicht erfindungsgemäß eingesetzte Fluortenside sind Verbindungen der Formel Id:

F-(CF₂-CF₂)₁₋₉-CH₂-CH₂-(O-CH₂-CH₂)₁₋₂₅-OH (Id).

Besonders bevorzugt werden Fluortenside eingesetzt, die biologisch abbaubar sind und die beim Einsatz in Dental-Abformmassen keine oder akzeptable Befunde gemäß ISO 10993-1 aufweisen.

CF₃-(CF₂)₆-(CH₂)₂-O-(C₂H₄O)ₘH

mit m = 2 bis 10

Das erfindungsgemäß eingesetzte Silizium aufweisende Tensid enthält mindestens einen (Poly)alkylenoxidrest und besitzt eine Molmasse von weniger als 6000 g/mol, vorzugsweise von weniger als 4000 g/mol, insbesondere von 350 bis 2000 g/mol.

Das erfindungsgemäß eingesetzte Silizium aufweisende Tensid enthält neben dem mindestens einen (Poly)alkylenoxidrest mindestens einen Rest, der Organosiloxanreste oder der Organosilanreste enthält. Die organischen Gruppen sind Kohlenwasserstoffreste, die gegebenenfalls teil- oder perfluoriert sind.

Derartige Organosiloxantenside oder Organocarbonsilantenside sind an sich bekannt.

Bevorzugt werden Silicium aufweisende Tenside eingesetzt, die Organosiloxantenside der Formeln II oder III sind oder die Organocarbonsilantenside der Formel IV, V oder VI sind

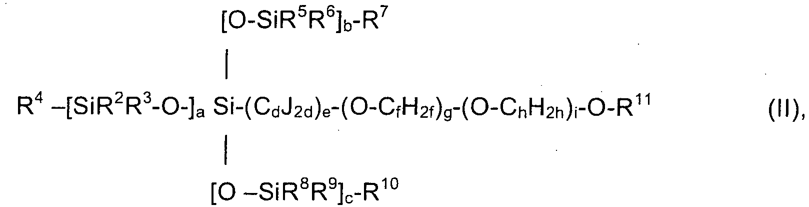

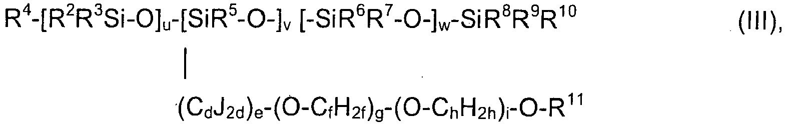

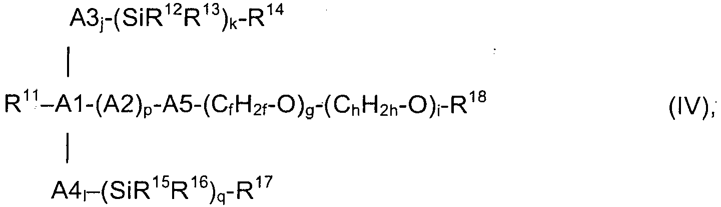

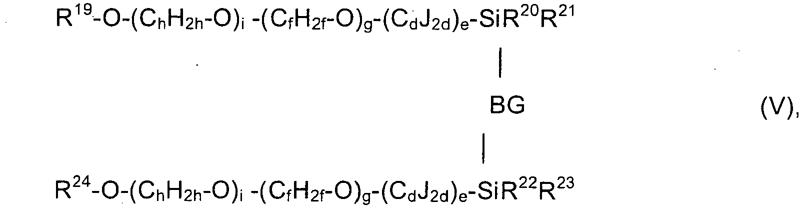

R¹⁹-O-(CₕH₂ₕ-O)ᵢ-(C_{f}H_{2f}-O)_{g}-(C_{d}J_{2d})ₑ-SiR²⁰R²¹R²² (VI),

worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
a, b, c und w unabhängig voneinander ganze Zahlen von 0 bis 100 bedeuten, vorzugsweise 0 bis 75, insbesondere 0 bis 35 und ganz besonders bevorzugt 0 bis 15, v eine ganze Zahl von 1 bis 100 bedeutet, vorzugsweise 1 bis 15 und ganz besonders bevorzugt 1 bis 6,
wobei die Summe von a, b und c zwischen 1 und 300 beträgt, vorzugsweise 1 bis 50, insbesondere 1 bis 10 und ganz besonders bevorzugt 1 bis 3,
und die Summe von v und w zwischen 1 und 200 beträgt, vorzugsweise 2 bis 90,
u 0 oder 1 ist,
d eine ganze Zahl von 1 bis 10 ist, vorzugsweise 1 bis 6 und insbesondere 1 bis 3,
J Wasserstoff oder Fluor bedeutet, vorzugsweise Wasserstoff,
e 0 oder 1 ist,
f und h unabhängig voneinander ganze Zahlen von 2 bis 6 bedeuten,
g und i unabhängig voneinander ganze Zahlen von 0 bis 30 sind, vorzugsweise 0 bis 15, wobei die Summe von g und i 1 bis 60 bedeutet, vorzugsweise 2 bis 30, insbesondere 2 bis 15,
R¹¹ Wasserstoff, Alkyl, Alkenyl oder Aryl ist, das gegebenenfalls teilweise oder vollständig fluoriert ist, vorzugsweise Wasserstoff oder Methyl,
R¹², R¹³, R¹⁴, R¹⁵, R¹⁶ und R¹⁷ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
k und q unabhängig voneinander 0 oder 1 bedeuten,
A1 Kohlenstoff oder Silizium bedeutet,
A2, A3 und A4 unabhängig voneinander eine Gruppe C_{d}J_{2d} ist, worin J und d die oben definierten Bedeutungen aufweisen,
j, p und l unabhängig voneinander 0 oder 1 sind,
A5 eine zweiwertige Brückengruppe, insbesondere -O-, -CO-O- oder -CO- bedeutet,
R¹⁸ Wasserstoff, Alkyl, Alkenyl oder Aryl ist, das gegebenenfalls teilweise oder vollständig fluoriert ist, vorzugsweise Wasserstoff oder Methyl,
R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
BG eine zweiwertige Brückengruppe ist, und
R¹⁹ und R²⁴ unabhängig voneinander Wasserstoff, Alkyl, Alkenyl oder Aryl sind, die gegebenenfalls teilweise oder vollständig fluoriert sind, vorzugsweise Wasserstoff oder Methyl,
mit der Massgabe, dass von den Resten R², R³ und R⁴ und/oder den Resten R⁵, R⁶ und R⁷ und/oder den Resten R⁸, R⁹ und R¹⁰ und/oder den Resten R¹⁵, R¹⁶ und R¹⁷ und/oder den Resten R²⁰ und R²¹ und/oder den Resten R²² und R²³ und/oder den Resten R²⁰, R²¹ und R²² nur einer Wasserstoff sein kann,
wobei f und h innerhalb eines Moleküls im Rahmen der gegebenen Definition unterschiedliche Werte annehmen können.

Unterschiedliche Indizes f und h bedeuten, dass Alkylenoxideinheiten unterschiedlicher Kohlenstoffanzahl vorliegen können, die in statistischer Verteilung oder in Form von Blöcken auftreten können.

Besonders bevorzugt werden Organosiloxantenside der Formel IIIa eingesetzt 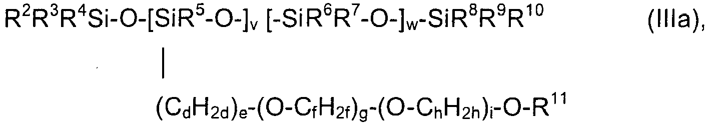
worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
v eine ganze Zahl von 1 bis 100 bedeutet,
w eine ganze Zahl von 0 bis 100 bedeutet,
d eine ganze Zahl von 1 bis 10 ist,
e 0 oder 1 ist,
f und h unabhängig voneinander ganze Zahlen von 2 bis 6 bedeutet,
g und i unabhängig voneinander ganze Zahlen von 0 bis 30 sind, wobei die Summe von g und i 1 bis 60 bedeutet, und
R¹¹ Wasserstoff, Alkyl, Alkenyl oder Aryl ist.

Weitere besonders bevorzugte Organosiloxantenside sind Verbindungen der Formel IIIb oder IIIc 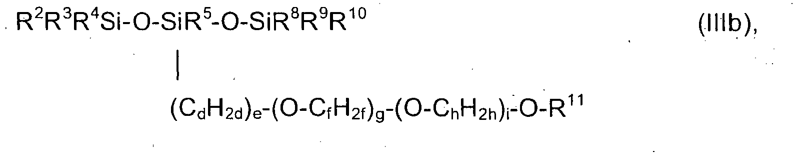 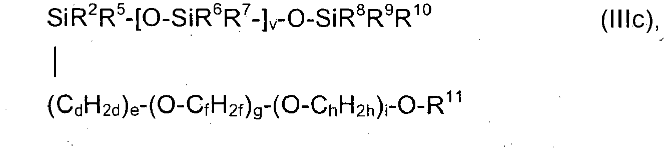
worin R², R³, R⁴, R⁵, R⁶, R⁷, R⁸, R⁹ und R¹⁰ unabhängig voneinander Wasserstoff, Alkyl oder Alkenyl sind, vorzugsweise Wasserstoff, Methyl, insbesondere Butyloxy und Methoxy, Vinyl oder Allyl,
v eine ganze Zahl von 1 bis 100 ist,
d, e, f, g, h und i die weiter oben definierten Bedeutungen besitzen, und
R¹¹ Wasserstoff, Alkyl, Alkenyl oder Aryl ist.

Ganz besonders bevorzugte Organosiloxantenside sind Verbindungen der Formel IIId 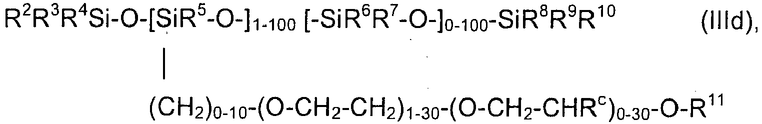
worin R², R³, R⁴, R⁵, R⁶, R⁷, R⁸, R⁹ und R¹⁰ unabhängig voneinander Alkyl, Alkenyl, Alkinyl, Aryl, Aryloxy, Aralkyl, Aralkyloxy, Alkylaryl oder Alkylaryloxy bedeuten, vorzugsweise Methyl,
R^{c} C₁-C₆ Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Weitere ganz besonders bevorzugte Organosiloxantenside sind Verbindungen der Formel IIIe 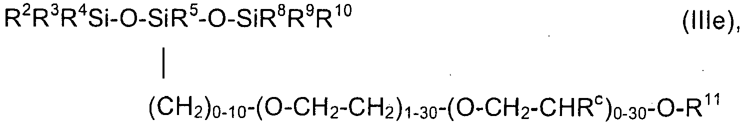
worin R², R³, R⁴, R⁵, R⁸, R⁹ und R¹⁰ unabhängig voneinander Alkyl, Alkenyl, Alkinyl, Aryl, Aryloxy, Aralkyl, Aralkyloxy, Alkylaryl oder Alkylaryloxy bedeuten, vorzugsweise Methyl,
R^{c} C₁-C₆ Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Weitere ganz besonders bevorzugte Organosiloxantenside sind Verbindungen der Formel IIIf 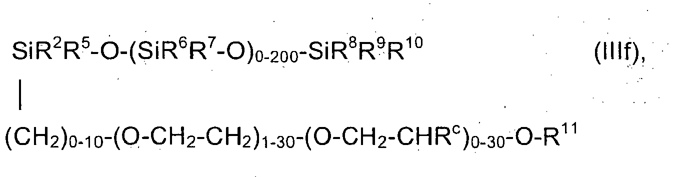
worin R², R⁵, R⁶, R⁷, R⁸, R⁹ und R¹⁰ unabhängig voneinander Alkyl, Alkenyl, Alkinyl, Aryl, Aralkyl oder Alkylaryl bedeuten, vorzugsweise Methyl,
R^{c} C₁-C₆ Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Ganz besonders bevorzugt werden Organosiloxantenside der Formel IIIg eingesetzt

[R²R³R⁴Si-O]₂-SiR⁵-(CH₂)₁₋₁₀-(O-CH₂-CH₂)₁₋₃₀-O-R¹¹ (IIIg)

worin R², R³, R⁴ und R⁵ unabhängig voneinander Alkyl und/oder Alkenyl bedeuten, vorzugsweise Methyl,
R¹¹ Wasserstoff, Alkyl, Alkenyl oder Aryl ist, vorzugsweise Wasserstoff oder Methyl.

Ganz besonders bevorzugte Silicium aufweisende Tenside sind Verbindungen der Formeln VII, VIII, IX und X 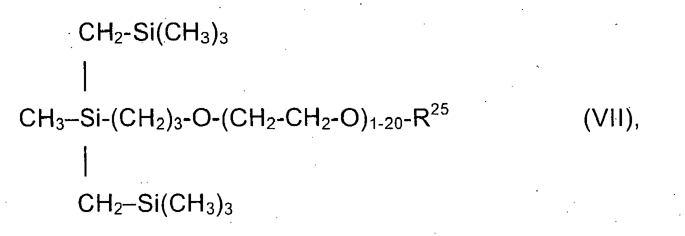 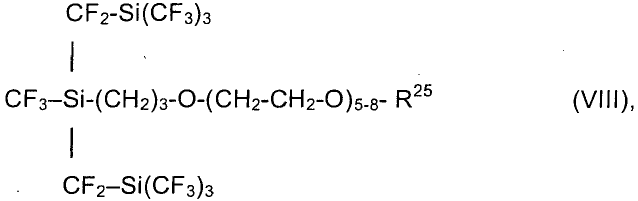 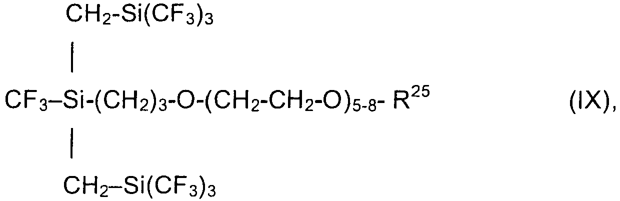 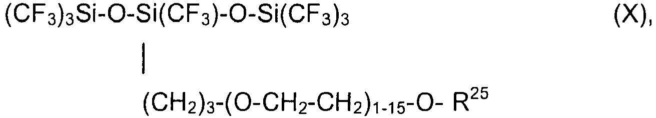 worin R²⁵ Wasserstoff, Methyl, Ethyl, Propyl oder Butyl ist, vorzugsweise Wasserstoff oder Methyl.

Diese bevorzugten Silcontenside oder Carbonsilantenside sind z.B. unter der Bezeichung Masil SF 19 (Fa. Lubrizol), Silwet L77 (Fa. GE-Bayer-Silicones) kommerziell erhältlich.

Besonders bevorzugt werden Carbosilantenside der Formel IVa eingesetzt 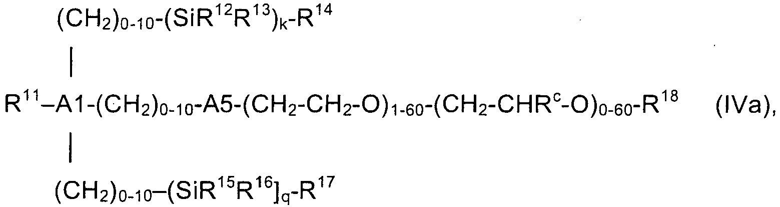
worin R¹¹, R¹², R¹³, R¹⁴, R¹⁵, R¹⁶ und R¹⁷ unabhängig voneinander Wasserstoff, Alkyl, Alkenyl, Alkinyl, Aryl, Aralkyl oder Alkylaryl, sind,
die Summe von k und q 1 oder 2 ist,
A5 ein Rest -O-, -CO-, -CO-O- , -S-, -NR¹⁹-, -CO-NR¹⁹-, -SO₂- oder -SO₂-NR¹⁹- ist,
R¹⁹ Wasserstoff, Alkyl, Alkenyl oder Aryl bedeutet,
R^{c} C₁-C₆ Alkyl, vorzugsweise Methyl oder Ethyl, oder Phenyl ist, und
R¹⁸ Wasserstoff, Alkyl, Alkenyl oder Aryl bedeutet.

Ebenfalls besonders bevorzugt werden Carbosilantenside der Formel Va eingesetzt 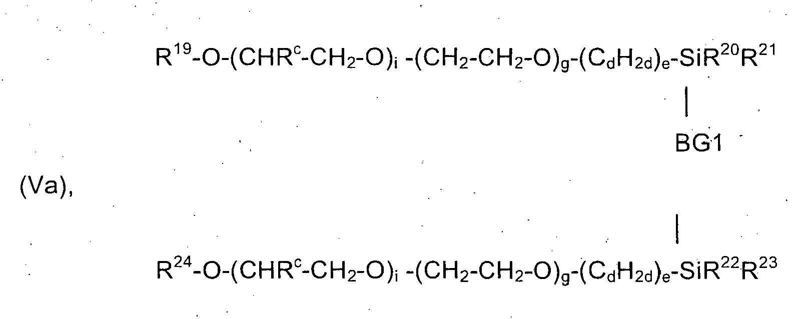
worin R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet,
BG1 eine Brückengruppe ist, die ausgewählt wird aus den Gruppen der Formeln -O-, Alkylen, Polyoxyalkylen, Phenylen, Cycloalkylen, Bicycloalkadiendiyl und -C₆H₅-K-C₆H₅-,
K eine direkte C-C-Bindung, -O-, -SO₂-, Alkylen oder Haloalkylen ist, und
R¹⁹ und R²⁴ unabhängig voneinander Wasserstoff, Alkyl, Alkenyl oder Aryl bedeuten.

Ganz besonders bevorzugt werden Verbindungen der Formel Va eingesetzt, worin BG1 eine Brückengruppe ist, die ausgewählt wird aus der Gruppe bestehend aus Formeln -O-, C₁-C₁₀-Alkylen, -(O-CH₂-CHR^{c})₂₋₆₀-, -(CH₂)₁₋₁₀-(O-CH₂-CHR^{c})₂₋₆₀-(CH₂)₁₋₁₀-, Phenylen, Cyclohexylen, Cyclopentylen, Norbonen-diyl, Bis-cyclopentadienyl-diyl, -C₆H₅-O-C₆H₅-, -C₆H₅-SO₂-C₆H₅-, -C₆H₅-C(CH₃)₂-C₆H₅- und -C₆H₅-C(CF₃)₂-C₆H₅-.

Ebenfalls besonders bevorzugt werden Carbosilantenside der Formel VIa eingesetzt

R¹⁹-O-(CHR^{c}-CH₂-O)ᵢ -(CH₂-CH₂-O)_{g}-(C_{d}H_{2d})ₑ-SiR²⁰R²¹R²² (VIa),

worin R²⁰, R²¹ und R²² unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet, und
R¹⁹ und R²⁴ unabhängig voneinander Wasserstoff, Alkyl, Alkenyl oder Aryl bedeuten.

Besonders bevorzugt werden Fluor-Carbosilantenside der Formel IVb eingesetzt 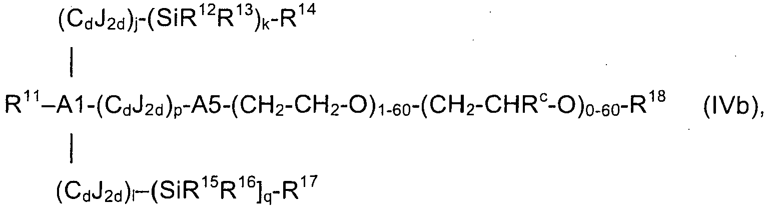
worin R¹¹, R¹², R¹³, R¹⁴, R¹⁵, R¹⁶ und R¹⁷ unabhängig voneinander Wasserstoff, Fluor, Alkyl, Alkyloxy, Alkenyl, Alkenyloxy, Alkinyl, Alkinyloxy, Aryl, Aryloxy, Aralkyl, Alkylaryl, Fluoralkyl, Fluoralkyloxy, Fluoralkenyl, Fluoralkenyloxy, Fluoralkinyl, Fluoralkinyloxy, Fluoraryl, Fluoraryloxy, Flouraralkyl oder Fluoralkylaryl sind, vorzugsweise Wasserstoff, Fluor, Alkyl, Fluoralkyl, Alkoxy, Fluoralkoxy, Alkenyl und Fluoralkenyl, wobei mindestens einer dieser Reste Fluor enthält und die Fluor enthaltenden Reste teil- oder perfluoriert sein können,
d eine ganze Zahl von 1 bis 10 bedeutet,
j, p und l 0 oder 1 sind,
die Summe von k und q 1 oder 2 ist,
A5 ein Rest -O-, -CO-O- , -S-, -NR¹⁹-, -CO-NR¹⁹-, -SO₂- oder -SO₂-NR¹⁹- ist,
R¹⁹ Wasserstoff, Alkyl, Alkenyl oder Aryl bedeutet,
R^{c} C₁-C₆ Alkyl, vorzugsweise Methyl oder Ethyl, oder Phenyl ist, und
R¹⁸ Wasserstoff, Alkyl, Alkenyl oder Aryl bedeutet.

Ganz besonders bevorzugt werden Fluor-Silicontenside der Formel IVc eingesetzt, 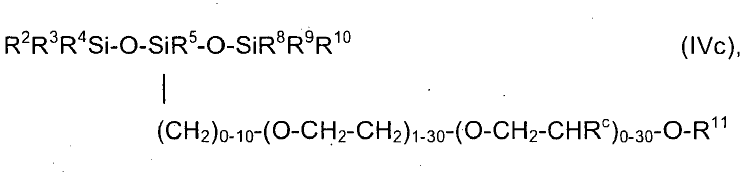
worin R², R³, R⁴, R⁵, R⁸, R⁹ und R¹⁰ unabhängig voneinander Alkyl, Alkyloxy, Alkenyl, Alkenyloxy, Alkinyl, Alkinyloxy, Aryl, Aryloxy, Aralkyl, Aralkyloxy, Alkylaryl oder Alkylaryloxy, Fluoralkyl, Fluoralkyloxy, Fluoralkenyl, Fluoralkenyloxy, Fluoralkinyl, Fluoralkinyloxy, Fluoraryl, Fluoraryloxy, Fluoraralkyl, Fluoraralkyloxy, Fluoralkylaryl oder Fluoralkylaryloxy bedeuten, vorzugsweise Methyl, Trifluormethyl, C₁-C₄Alkoxy, C₁-C₄Fluoralkoxy, insbesondere Methoxy oder Trifluormethyl, und/oder Vinyl und/oder Allyl, wobei mindestens einer dieser Reste Fluor enthält und die Fluor enthaltenden Reste teil- oder perfluoriert sein können,
R^{c} C₁-C₆ Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Ebenfalls besonders bevorzugt werden Fluor-Carbosilantenside der Formel Vc eingesetzt 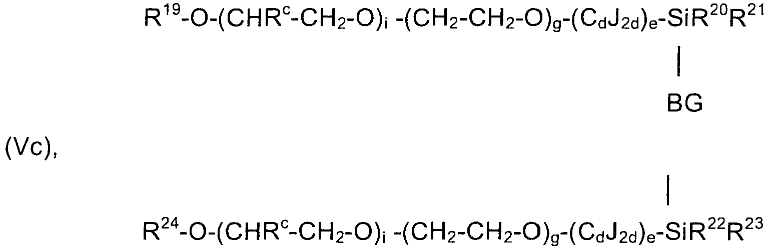
worin R²⁰, R²¹, R²² und R²³ unabhängig voneinander Wasserstoff, Fluor, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy, Fluoralkyl, Fluoralkyloxy, Fluoralkenyl, Fluoralkenyloxy, Fluoralkinyl, Fluoralkinyloxy, Fluoraryl, Fluoraryloxy, Fluoraralkyl, Fluoraralkyloxy, Fluoralkylaryl und/oder Fluoralkylaryloxy bedeuten, vorzugsweise Wasserstoff, Fluor, Alkyl, Fluoralkyl, Alkyloxy, Fluoralkyloxy, Alkenyl oder Fluoralkenyl, wobei mindestens einer dieser Reste Fluor enthält und die Fluor enthaltenden Reste teil- oder perfluoriert sein können,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet,
BG eine Brückengruppe ist, die ausgewählt wird aus den Gruppen der Formeln -O-, Alkylen, Polyoxyalkylen, Phenylen, Cycloalkylen, Bicycloalkadiendiyl und -C₆H₅-K-C₆H₅-,
K eine direkte C-C-Bindung, -O-, -SO₂-, Alkylen oder Haloalkylen ist, und
R¹⁹ und R²⁴ unabhängig voneinander Wasserstoff, Alkyl, Alkenyl oder Aryl bedeuten.

Ganz besonders bevorzugt werden Verbindungen der Formel Vc eingesetzt, worin BG eine Brückengruppe ist, die ausgewählt wird aus der Gruppe bestehend aus Formeln -O-, C₁-C₁₀-Alkylen, -(O-CH₂-CHR^{c})₂₋₆₀-, -(CH₂)₁₋₁₀₋(O-CH₂-CHR^{c})₂₋₆₀-(CH₂)₁₋₁₀-, Phenylen, Cyclohexylen, Cyclopentylen, Norbonen-diyl, Bis-cyclopentadienyl-diyl, -C₆H₅-O-C₆H₅-, -C₆H₅-SO₂-C₆H₅-, -C₆H₅-C(CH₃)₂-C₆H₅- und -C₆H₅-C(CF₃)₂-C₆H₅-.

Ebenfalls besonders bevorzugt werden Fluor-Carbosilantenside der Formel VIb eingesetzt

R¹⁹-O-(CHR^{c}-CH₂-O)ᵢ -(CH₂-CH₂-O)_{g}-(C_{d}H_{2d})ₑ-SiR²⁰R²¹R²² (VIb),

worin R²⁰, R²¹ und R²² unabhängig voneinander Wasserstoff, Fluor, Alkyl, Alkenyl, Alkinyl, Aryl, Aralkyl und/oder Alkylaryl, Fluoralkyl, Fluoralkenyl, Fluoralkinyl, Fluoraryl, Fluoraralkyl und/oder Fluoralkylaryl bedeuten, vorzugsweise Wasserstoff, Fluor, Alkyl, Fluoralkyl, Alkenyl oder Fluoralkenyl, wobei mindestens einer dieser Reste Fluor enthält und die Fluor enthaltenden Reste teil- oder perfluoriert sein können,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet, und
R¹⁹ und R²⁴ unabhängig voneinander Wasserstoff, Alkyl, Alkenyl oder Aryl bedeuten.

Die nichtionischen Silcontenside oder nichtionischen Carbonsilantenside der Formeln II bis X werden einzeln oder in Kombination besonders bevorzugt zusammen mit den nichtionischen Fluortensiden der Formeln I oder Ia bis Id oder Gemischen von zwei oder mehreren dieser nichtionischen Fluortenside eingesetzt.

Der Anteil an Silizium aufweisendem nichtionischem Tensid an der erfindungsgemäßen Dentalabformmasse beträgt üblicherweise 0,05 bis 5,0 Gew.-%, vorzugsweise 0,1 bis 5,0 Gew.-%, bezogen auf die Dentalabformmasse.

Der Anteil an nichtionischem Fluortensid an der erfindunsgemäßen Dentalabformmasse beträgt üblicherweise 0,001 bis 5,0 Gew.-%, vorzugsweise 0,01 bis 5,0 Gew.-%, bezogen auf die Dentalabformmasse.

In der erfindungsgemäß eingesetzten synergistischen Tensidmischung beträgt das Gewichtsverhältnis von Silizium aufweisendem nichtionischem Tensid zu nichtionischem Fluortensid 100 : 1 bis 1 : 100, bevorzugt 50 : 1 bis 1 : 50, besonders bevorzugt 10 : 1 bis 1 : 10 und insbesondere bevorzugt 4 : 1 bis 1 : 5.

Die in den erfindungsgemäßen Dentalabformmassen eingesetzten synergistischen Mischungen aus Silizium aufweisendem nichtionischem Tensid und nichtionischem Fluortensid enthalten vorzugsweise als weitere Komponente einen Alkenylreste und/oder Alkinylreste enthaltenden Polyether und/oder einen hydroxyl- und/oder aryloxy- und/oder arylalkyloxy- und/oder alkoxy-terminierten Polyether.

Diese zusätzliche Komponente bewirkt eine weitere Verbesserung der Konaktwinkeleigenschaften des nicht ausgehärteten plastischen Materials und somit eine weitere Verbesserung der Formtreue des entstehenden Abdrucks.

Beispiele für bevorzugte Zusätze dieses Typs sind die Verbindungen der Formel XI

R³⁰-A-(R³⁰)ₙ₁ (XI),

worin R³⁰ ein ethylenisch ungesättigter Kohlenwasserstoffrest, vorzugsweise Allyl, Styryl, Acryloyl oder Methacryloly, oder Wasserstoff oder Alkyl bedeutet, und mehrere Reste R³⁰ eines Moleküls im Rahmen der gegebenen Definition unterschiedlich sein können,
n1 eine ganze Zahl von 1 bis 5 bedeutet, vorzugsweise 1, 2 oder 3, insbesondere 1,
A ein Rest der Formel Z-(O-(Cₙ₂H₂ₙ₂-O)ₘ₁-)ₙ₁₊₁ bedeutet,
Z ein n1-wertiger Kohlenwasserstoffrest ist, vorzugsweise ein von einem zwei-, drei-, vier- fünf- oder sechswertigen aliphatischen Alkohol abgeleiteter Rest, insbesondere ein von Alkylenglykol, Trimethylolpropan, Pentaerythrit oder Sorbit abgeleiteter Rest,
n2 eine ganze Zahl von 2 bis 8, vorzugsweise von 2 bis 4 bedeutet, und
m1 eine ganze Zahl von 1 bis 35000, vorzugsweise von 50 bis 1500 ist, wobei n2 und m1 innerhalb eines Moleküls im Rahmen der gegebenen Definition unterschiedlich sein können.

Die Indizes n2 und/oder m1 können innerhalb eines Moleküls sowie innerhalb einer Alkylenoxidkette im Rahmen der gegebenen Definition unterschiedliche Werte annehmen. Unterschiedliche Indizes n2 und/oder m1 bedeuten, dass Alkylenoxideinheiten unterschiedlicher Kohlenstoffanzahl vorliegen können, die in statistischer Verteilung oder in Form von Blöcken auftreten können bzw. die dass die einzelnen Blöcke innerhalb eines Moleküls unterschiedliche Längen aufweisen können.

Ganz besonders bevorzugt eingesetzte Polyether sind Verbindungen der Formeln XII und XIII

CH₂=CH-CH₂-O-(Cₙ₂H₂ₙ₂-O)ₘ₉-CH₂-CH=CH₂ (XII),

R³¹-O-(Cₙ₂H₂ₙ₂-O)ₘ₉-R³² (XIII),

worin n2 eine ganze Zahl von 2 bis 8, vorzugsweise von 2 bis 4 bedeutet,
m9 eine ganze Zahl von 3 bis 70000, vorzugsweise von 10 bis 2500 ist, wobei n2 und m9 innerhalb eines Moleküls im Rahmen der gegebenen Definition unterschiedlich sein können, und
R³¹ und R³² unabhängig voneinander Wasserstoff oder C₁-C₆-Alkyl, insbesondere Wasserstoff und/oder Methyl, Ethyl oder Propyl bedeuten.

Beispiele für besonders bevorzugte Alkylenoxideinheiten sind -CH₂-CH₂-O-, -CH₂-CH₂-CH₂-O-,-CH₂-CH(CH₃)-O- und -CH₂-CH₂-CH₂-CH₂-O-.

Besonders bevorzugt werden Polyether der Formeln XII und XIII, insbesondere solche, die Ethylenoxideinheiten oder Ethylenoxideinheiten und Propylenoxideinheiten oder Ethylenoxideinheiten und Butylenoxideinheiten aufweisen.

Diese Polyether kommen in der Regel als Gemische von Polymeren unterschiedlicher Kettenlänge zum Einsatz. Typische Molekulargewichte (Zahlenmittel) bewegen sich im Bereich von 150 bis 3000000, vorzugsweise von 250 bis 100000 und besonders bevorzugt von 250 bis 50000.

Der Anteil dieser Polyether an der erfindungsgemäßen Dentalabformmasse beträgt üblicherweise 0,1 bis 25,0 Gew.-%, vorzugsweise 0,1 bis 10,0 Gew.-%, und besonders bevorzugt 0,5 bis 2,5 Gew.-%, bezogen auf die Dentalabformmasse.

Die in den erfindungsgemäßen Dentalabformmassen eingesetzten synergistischen Mischungen aus Silizium aufweisendem nichtionischem Tensid und nichtionischem Fluortensid enthalten vorzugsweise als weitere Komponente ein Polyol.

Diese zusätzliche Komponente bewirkt eine weitere Verbesserung der Konaktwinkeleigenschaften des nicht ausgehärteten plastischen Materials und somit eine weitere Verbesserung der Detailgenauigkeit des entstehenden Abdrucks.

Bei dieser zusätzlichen Komponente kann es sich um monomere, oligomere oder polymere Polyole handeln. Diese können primäre, sekundäre und/oder tertiäre Hydroxylgruppen aufweisen. Die Hydroxylgruppen können an aromatische Reste gebunden sein, bevorzugt jedoch an aliphatische Reste.

Der Anteil dieser Polyole an der erfindungsgemäßen Dentalabformmasse beträgt üblicherweise 0,1 bis 25,0 Gew.-%, vorzugsweise 0,1 bis 10,0 Gew.-%, und besonders bevorzugt 0,5 bis 2,5 Gew.-%, bezogen auf die Dentalabformmasse.

Bevorzugte Polyole werden ausgewählt aus der Gruppe der Kohlehydrate, der Polyvinylalkohole, der aliphatischen Di-, Tri-, Tetra-, Penta- und/oder Hexaole und Mischungen von zwei oder mehreren dieser Polyole.

Besonders bevorzugte Polyole werden ausgewählt aus der Gruppe der Polyvinylalkohole, der Polysaccharide, Trimethylolpropan, Pentaerythritol, Dipentaerythritol, Glycerin, Allyloxy-1,2-propandiol, 2-Methyl-2,4-pentandiol, Trimethylolpropanallylether, Decandiol, Nonandiol, Octandiol, Heptandiol, Hexandiol, Pentandiol, Butandiol, Propandiol, Ethandiol, Fructose, Glucose und Mischungen von zwei oder mehreren dieser Polyole, insbesondere Glycerin.

In einer weiteren bevorzugten Ausführungsform enthält die erfindungsgemäße Dentalabformmasse neben dem Polyol als weitere Komponente einen Alkenylreste und/oder Alkinylreste enthaltenden Polyether und/oder einen aryloxy- und/oder arylalkyloxy- und/oder hydroxyl- und/oder alkoxy-terminierten Polyether.

Durch die Anwesenheit dieser beiden Komponenten lassen sich die Kontaktwinkeleigenschaften des nicht ausgehärteten plastischen Materials verbessern.

Bei den härtbaren Polymersystemen können unterschiedliche Typen eingesetzt werden. In Abhängigkeit vom jeweiligen Polymersystem können die erfindungsgemäßen Dentalabformmassen als Einkomponentensysteme oder als Mehrkomponentensysteme, vorzugsweise als Zweikomponentensysteme, vorliegen. Die härtbaren Polymersysteme und deren zusätzliche Komponenten, wie Katalysatoren und/oder Initiatoren, sind dem Fachmann an sich bekannt.

Der Anteil der härtbaren Polymeren an der erfindungsgemäßen Dentalabformmasse beträgt üblicherweise 5 bis 80 Gew.-%, vorzugsweise 20 bis 70 Gew.-%, bezogen auf die Dentalabformmasse.

Der Anteil der Katalysatoren und/oder der Photoinitiatoren an der erfindungsgemäßen Dentalabformmasse beträgt üblicherweise 0,00005 bis 10 Gew.-%, vorzugsweise 0,0001 bis 5 Gew.-%, bezogen auf die Dentalabformmasse.

Bevorzugt werden Dentalabformmassen enthaltend einen vernetzbaren Polyether, der Alkoxysilylreste, Aziridinoreste, von einer ethylenisch ungesättigten Carbonsäure abgeleitete Reste, Alkenylreste als vernetzbare Gruppen oder über ringöffnende Metathesereaktion vernetzbare Gruppen aufweist, einen Vernetzungskatalysator und/oder Photoinitiator sowie ein Silizium aufweisendes nichtionisches Tensid und ein nichtionisches Fluortensid, wobei das nichtionische Fluortensid aus einem teil- oder perfluorierten Kohlenwasserstoffrest besteht, der über ein Sauerstoffatom oder eine Estergruppe als Brückengruppe mit einem (Poly)alkylenoxidrest verbunden ist.

Besonders bevorzugt werden Dentalabformmasse enthaltend Organopolysiloxane als härtbare Polymersysteme eingesetzt. Derartige Zusammensetzungen sind z.B. aus DE 3410646 A1 bekannt. Bekanntermaßen unterscheidet man zwischen durch Additionsreaktion vernetzenden Organopolysiloxanen, durch Kondensationsreaktion vernetzenden Organopolysiloxanen; und durch ringöffnende Metathesereaktion vernetzenden Organopolysiloxanen. Erfindungsgemäß können alle diese Polymersysteme eingesetzt werden.

Bevorzugt werden durch Additionsreaktion vernetzende Organopolysiloxane.

Durch Additionsreaktion härtbare Organopolysiloxane sind beispielsweise aus DE 3410646 A1, DE 10017154 A1 bekannt.

Diese kommen üblicherweise in Form einer Mehrkomponenten Dentalabformmasse enthaltend Komponenten A und B zum Einsatz. Darin enthält
a) Komponente A ein Organopolysiloxan mit mindestens zwei ethylenisch ungesättigten Gruppen und einen Hydrosilylierungskatalysator,
b) Komponente B enthält ein Organohydrogenpolysiloxan, und
c) mindestens eine der Komponenten A und/oder B enthält ein Silizium aufweisendes nichtionisches Tensid und/oder ein nichtionisches Fluortensid nach Anspruch 1, und Komponenten A und B weisen eine solche Zusammensetzung auf, so dass nach dem Vermischen der Komponenten A und B eine Kombination von Silizium aufweisendem nichtionischem Tensid und nichtionischem Fluortensid nach Anspruch 1 vorliegt.

Als Organopolysiloxan mit mindestens zwei ethylenisch ungesättigten Gruppen kommen üblicherweise Organopolysiloxane zum Einsatz, die mindestens zwei an Si-Atome gebundene Allyl- oder insbesondere Vinylgruppen aufweisen.

Typischerweise handelt es sich dabei um Verbindungen der folgenden Formeln XIV oder XV

CH₂=CH-SiR₂-B-SiR₂-CH=CH₂ (XIV),

oder

CH₂=CH-CH₂-SiR₂-B-SiR₂-CH₂-CH=CH₂ (XV),

worin B ein Rest der Formel -O-(SiR₂-O)ₘ₂- bedeutet,
die einzelnen R innerhalb der Polymerkette unabhängig voneinander Alkyl, Alkenyl, Alkinyl, Cycloalkyl, Aryl und/oder Aralkyl bedeuten, die gegebenenfalls substituiert sind, und m2 eine ganze Zahl von 10 bis 6000 ist, vorzugsweise von 20 bis 2000.

Organopolysiloxane mit mindestens zwei ethylenisch ungesättigten Gruppen kommen in der Regel als Gemische von Polymeren unterschiedlicher Kettenlänge zum Einsatz. Typische Molekulargewichte (Zahlenmittel) bewegen sich im Bereich von 900 bis 500000, vorzugsweise von 1500 bis 150000.

Beispiele für Alkylgruppen sind geradkettige oder verzweigte Alkylgruppen mit ein bis sechs Kohlenstoffatomen, wie Methyl, Ethyl, n-Propyl, Isopropyl oder n-Butyl. Beispiele für substituierte Alkylgruppen sind geradkettige oder verzweigte Alkylgruppen mit ein bis sechs Kohlenstoffatomen, die mit ein oder mehreren Halogenatomen substituiert sind, z.B. Trifluormethyl.

Beispiele für Cycloalkylgruppen sind Reste mit fünf bis sechs Ringkohlenstoffatomen, wie Cyclohexyl.

Beispiele für Arylgruppen sind ein der zweikernige aromatische Kohlenwasserstoffreste, wie Phenyl oder Naphthyl.

Beispiele für substituierte Arylgruppen sind mit Alkyl- oder Halogen substituierte Phenyle, wie Tolyl, Xylyl oder Chlorphenyl.

Ein Beispiel für eine Aralkylgruppe ist Benzyl.

Besonders bevorzugt werden Organopolysiloxane der Formeln I und/oder II, worin R Methyl ist.

Als Organohydrogenpolysiloxane kommen die üblicherweise als Vernetzer eingesetzten Verbindungen zum Einsatz. Dabei kann es sich um Polyalkyl-, Polyaryl-, Polyalkylaryl-, Polyhalogenalkyl-, Polyhalogenaryl- und Polyhalogenalkylaryl-Siloxane handeln, welche im Molekül mindesten zwei an Siliciumatome gebundene Wasserstoffatome aufweisen.

Ein typisches Beispiel sind Verbindungen der Formel RₐH_{b}SiO_{(4-(a+b)/2)}, worin R die oben für die Verbindungen der Formel XIV oder XV definierte Bedeutung besitzt, a eine reelle Zahl mit 1<a<2, b eine reelle Zahl mit 0<b<=1 bedeutet, mit der Massgabe, dass 1<a+b<2,7 ist und dass die Verbindungen mindestens zwei, vorzugsweise mindestens drei Si-H-Bindungen aufweisen.

Bevorzugte Beispiele für Organohydrogenpolysiloxane sind die Verbindungen der Formel XVIa, XVIb, XVIc, XVId und XVIe

H-SiR₂-O-(SiR₂-O)_{c}-(SiHR-O)_{d}-SiR₂H (XVIa),

R₃Si-O-(SiR₂-O)_{c}-(SiHRO)_{d}-SiR₃ (XVIb),

Rₙ₃Si-(O-SiR₂-H)₄₋ₙ₃ (XVIc),

(SiO_{4/2})_{q}(R₂HSiO_{1/2})ₚ (XVId),

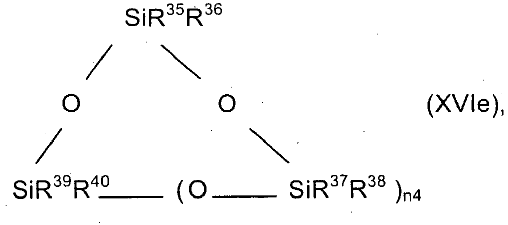

worin R die für Verbindungen der Formeln XIV oder XV definierten Bedeutungen aufweisen, und innerhalb eines Moleküls unterschiedliche Reste R im Rahmen der gegebenen Definition unterschiedliche Bedeutungen annehmen können, insbesondere Alkylreste, vorzugsweise Methyl bedeuten,
R³⁵ bis R⁴⁰ unabhängig voneinander Wasserstoff, Alkyl, Cycloalkyl, Aryl oder Aralkyl bedeuten, wobei mindestens zwei dieser Reste Wasserstoff bedeuten und von den Resten R³⁵ und R³⁶, oder R³⁷ und R³⁸ oder R³⁹ und R⁴⁰ nur jeweils einer Wasserstoff ist,
c, d und e unabhängig voneinander ganze Zahlen von 0 bis 100 sind, wobei die Summe von c, d und e 2 bis 300 beträgt,
n3 eine ganze Zahl von 0 bis 3 ist,
q und p unabhängig voneinander reelle Zahlen größer als 0 sind, mit der Massgabe, dass die Summe von q und p 1 ist und
n4 eine ganze Zahl von 1 bis 5 ist, vorzugsweise 1 oder 2.

Bevorzugt werden Organohydrogenpolysiloxane eingesetzt, die einen SiH-Gehalt von 0,01 bis 15 mmol/g, vorzugsweise von 0,1 bis 10 mmol/g, aufweisen.

Weitere bevorzugt eingesetzte Organohydrogenpolysiloxane sind Methylhydrogenpolysiloxane.

Als Katalysatoren für die Hydrosilylierung werden üblicherweise Salze, Komplexe oder kollodial vorliegende Formen der Übergangsmetalle der 8. Nebengruppe eingesetzt. Vorzugsweise kommen Platin, Palladium oder Rhodium in Form von Metallen oder als Komplexe zum Einsatz. Besonders bevorzugt werden Platin-komplexe eingesetzt, die z.B. aus Hexachloroplatinsäure oder aus Platinsalzen hergestellt worden sind, z.B. Tris(divinyltetramethyldisiloxane)di-platinum(o)-complex, Platinum-Divinyltetramethyldisiloxan Complex.

Besonders bevorzugte Dentalabformmassen enthalten in Komponente A ein Polydialkylsiloxan mit mindestens zwei Vinylgruppen und eine Platinverbindung als Hydrosilylierungskatalysator.

Weitere besonders bevorzugte Dentalabformmassen enthalten in Komponente B eine Mischung aus Silizium aufweisendem nichtionischem Tensid und nichtionischem Fluortensid.

Weitere bevorzugte Dentalabformmassen enthalten durch Kondensationsreaktion vernetzende Organopolysiloxane.

Durch Kondensationsreaktion härtbare Organopolysiloxane sind beispielsweise aus DE 4137698 A1 bekannt.

Bevorzugt werden Dentalabformmasse, deren Komponente C ein Polydialkylsiloxan mit mindestens zwei Hydroxylgruppen enthält und deren Komponente D ein Polydialkylsiloxan und/oder ein Silan mit mindestens zwei Di- oder Trialkoxysilylgruppen sowie einen Kondensationskatalystor, vorzugsweise eine Zinnverbindung, enthält.

Ebenfalls bevorzugt werden Dentalabformmassen, deren Komponente D eine Mischung aus Silizium aufweisendem nichtionischem Tensid und nichtionischem Fluortensid enthält.

Diese kommen üblicherweise in Form einer Mehrkomponenten Dentalabformmasse enthaltend Komponenten C und D zum Einsatz. Darin enthält
d) Komponente C ein Organopolysiloxan mit mindestens zwei Hydroxylgruppen,
e) Komponente D ein Kieselsäureester, Polykieselsäureester und/oder ein Organopolysiloxan mit mindestens zwei Alkoxygruppen sowie einen Kondensationskatalysator, und
f) mindestens eine der Komponenten C und/oder D enthält ein Silizium aufweisendes nichtionisches Tensid und/oder ein nichtionisches Fluortensid, nach Anspruch 1 und Komponenten C und D weisen eine solche Zusammensetzung auf, so dass nach dem Vermischen der Komponenten C und D eine Kombination von Silizium aufweisendem nichtionischem Tensid und nichtionischem Fluortensid nach Anspruch 1 vorliegt.

Als Organopolysiloxan mit mindestens zwei Hydroxylgruppen kommen üblicherweise Organopolysiloxane zum Einsatz, die mindestens zwei an Si-Atome gebundene Hydroxylgruppen aufweisen.

Typischerweise handelt es sich dabei um Verbindungen der folgenden Formel XVII

HO-SiR₂-B-SiR₂-OH (XVII),

worin B die für die Verbindungen der Formeln XIV und XV definierte Bedeutung aufweist.

Besonders bevorzugt werden Organopolysiloxane mit Hydroxyl-Endgruppen, worin R Methyl ist.

Organopolysiloxane mit mindestens zwei Hydroxylgruppen kommen in der Regel als Gemische von Polymeren unterschiedlicher Kettenlänge zum Einsatz. Typische Molekulargewichte (Zahlenmittel) bewegen sich im Bereich von 900 bis 500000, vorzugsweise von 1500 bis 150000.

Als Silane, Kieselsäureester, Polykieselsäureester und/oder Organopolysiloxane mit mindestens zwei Alkoxygruppen kommen üblicherweise Organopolysiloxane oder Organooxysiliziumverbindungen zum Einsatz, die mindestens zwei, vorzugsweise drei oder vier an Si-Atome gebundene Alkoxygruppen aufweisen.

Typischerweise handelt es sich dabei um Verbindungen der folgenden Formel XVIII und/oder XIX und/oder XX

(RO)ₘ-SiR₃₋ₘ-B-SiR₃₋ₘ-(OR)ₘ (XVIII),

SiR_{z}(OR')_{4-z} (XIX),

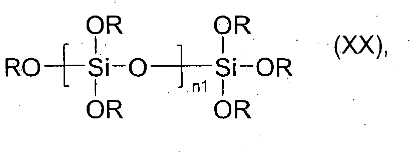

worin B die für Verbindungen der Formel XIV und XV definierte Bedeutung besitzt, die einzelnen R innerhalb der Polymerkette der Formeln XVIII oder XIX oder innerhalb der Verbindung der Formel XX unabhängig voneinander Alkyl, Cycloalkyl, Aryl und/oder Aralkyl bedeuten, die gegebenenfalls substituiert sind,
die einzelnen R' innerhalb der Verbindung der Formel XIX unabhängig voneinander eine der für R definierten Bedeutungen aufweisen, vorzugsweise Alkyl sind,
z eine ganze Zahl von 0 bis 2 ist,
n1 eine ganze Zahl von 1 bis 100 ist, vorzugsweise von 50 bis 70,
und m eine ganze Zahl von 1 bis 3 ist.

Beispiele für Reste R sind bei der Beschreibung der vinyl- bzw. allyl-terminierten Organopolysiloxane der Formeln XIV und XV aufgeführt.

Besonders bevorzugt werden Alkoxysiliziumverbindungen der Formel XIX, worin R und R' Methyl sind und z 0, 1 oder 2 bedeutet.

Organopolysiloxane mit mindestens zwei Alkoxygruppen kommen in der Regel als Gemische von Polymeren unterschiedlicher Kettenlänge zum Einsatz. Typische Molekulargewichte (Zahlenmittel) bewegen sich im Bereich von 400 bis 10000, vorzugsweise von 250 bis 5000.

Als Katalysatoren für die Kondensationsreaktion werden üblicherweise Organozinnverbindungen, Titanate, Zirkonate oder Wismuthate eingesetzt, beispielsweise Tetraethyltitanat, Tetraisopropyltitanat, Tetra-n-propyltitanat, Tetra-n-butyltitanat, n-Butyl-polytitanat, Tetra-2-ethylhexyl-titanat, Tetraisooctyltitanat, Octylenglykoltitanat, Tetra-n-propylzirkonat, Tetra-n-butylzirkonat, Zinn-II-isooctoat, Dioctylzinndicarboxylat, Dioctylzinndilaurat, Dibutylzinndilaurat, Organozinn-carboxylat, Dibutylzinncarboxylat, Dibutylzinnacetylacetonat, Dibutylzinndiacetat und Wismuth-II-ethylhexanoat.

Weitere bevorzugte Dentalabformmassen enthalten Alkenylreste enthaltende Polyether.

Härtbare Systeme dieses Typs sind beispielsweise aus DE-A-4010281 bekannt.

Bevorzugt werden Dentalabformmassen, deren Komponente E ein Polyalkylenether mit mindestens zwei Alkylenresten und eine Platinverbindung als Hydrosilylierungskatalysator enthält.

Ebenfalls bevorzugt werden Dentalabformmassen, deren Komponente F eine Mischung aus Silizium aufweisendem nichtionischem Tensid und nichtionischem Fluortensid enthält.

Diese kommen üblicherweise in Form einer Mehrkomponenten Dentalabformmasse enthaltend Komponenten E und F zum Einsatz. Darin enthält
g) Komponente E einen Vernetzungskatalysator,
h) Komponente F einen vernetzbaren und Alkenylreste aufweisenden Polyether sowie ein Organohydrogenpolysiloxan und/oder SiH-Polyether, und
i) mindestens eine der Komponenten E und/oder F enthält ein Silicium aufweisendes nichtionisches Tensid und/oder ein nichtionisches Fluortensid nach Anspruch 1, und Komponenten E und F weisen eine solche Zusammensetzung auf, so dass nach dem Vermischen der Komponenten E und F eine Kombination von Silizium aufweisendem nichtionischem Tensid und nichtionischem Fluortensid nach Anspruch 1 vorliegt.

Als Alkenylreste aufweisende Polyether kommen üblicherweise von Polyalkylenglykolen abgeleitete und ethylenisch ungesättigten Gruppen, z.B. mit Allyletherresten terminierte Polymere in Frage.

Typischerweise handelt es sich dabei um Verbindungen der folgenden Formel XXI

X-A-(X)ₙ₁ (XXI),

worin A und n1 die für Verbindungen der Formel XI definierte Bedeutung besitzen und X eine Alkenylgruppe mit endständiger Doppelbindung ist.

Beispiele für Alkenylgruppen mit endständiger Doppelbindung X sind -CH₂-CH=CH₂, -SiR₂-CH=CH₂, -CR₂-CH=CH₂ und -C₆H₄-CH=CH₂, worin R Alkylgruppen bedeuten.

Besonders bevorzugt werden Polyether der Formel XXI, die Ethylenoxid- und/oder Propylenoxideinheiten aufweisen, insbesondere solche mit endständigen Allylgruppen.

Alkenylreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3000000, vorzugsweise von 250 bis 100000.

Als Organohydrogenpolysiloxane kommen die bereits weiter oben bei der Beschreibung der durch Addionsreaktion vernetzenden Organopolysiloxane beschriebenen Verbindungen in Frage.

Auch in diesem vernetzbaren Polymersystem werden bevorzugt Organohydrogenpolysiloxane eingesetzt, die einen SiH-Gehalt von 0,01 bis 15 mmol/g, vorzugsweise von 0,1 bis 10 mmol/g, aufweisen.

Weitere bevorzugt eingesetzte Organohydrogenpolysiloxane sind Methylhydrogenpolysiloxane.

Als SiH-Polyether kommen Verbindungen der Formel XXII in Frage

Y-A-(Y)ₙ₁ (XXII),

worin A und n1 die für Verbindungen der Formel XI definierte Bedeutung besitzen und Y eine Gruppe enthaltend einen Siliziumwasserstoffrest ist.

Beispiele für Reste Y sind Gruppen der Formel -R'-SiR₂H oder der Formel XVIf 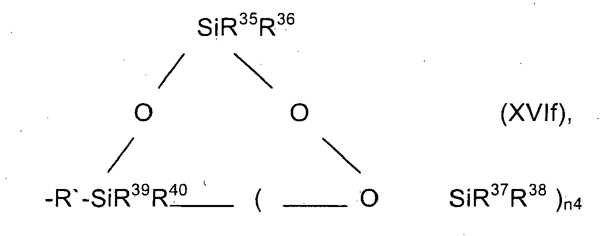
worin R' für einen Alkylenrest, vorzugsweise mit ein bis sechs Kohlenstoffatomen, steht,
R die für Verbindungen der Formeln XIV oder XV definierten Bedeutungen aufweist, und innerhalb eines Moleküls unterschiedliche Reste R im Rahmen der gegebenen Definition unterschiedliche Bedeutungen annehmen können, insbesondere Alkylreste, vorzugsweise Methyl bedeuten,
und R³⁵ bis R⁴⁰ sowie n4 die weiter oben für Reste der Formel XVIe definierte Bedeutung besitzt.

Als Katalysatoren werden auch in diesem härtbaren System die weiter oben beschriebenen Salze, Komplexe oder kollodial vorliegende Formen der Übergangsmetalle der 8. Nebengruppe eingesetzt. Vorzugsweise kommen Platin, Palladium oder Rhodium in Form von Metallen oder als Komplexe zum Einsatz. Besonders bevorzugt werden Platin-komplexe eingesetzt, die z.B. aus Hexachloroplatinsäure oder aus Platinsalzen hergestellt worden sind, z.B. Tris(divinyltetramethyldisiloxane)diplatinum(o)-complex, Platinum-Divinyltetramethyldisiloxan Complex.

Weitere bevorzugte Dentalabformmassen enthalten Alkoxysilylreste enthaltende Polyether.

Härtbare Systeme dieses Typs sind beispielsweise aus PCT/EP2005/001470 und aus EP-A-1,226,808 bekannt.

Bevorzugt werden Dentalabformmassen, die einen Polyalkylenether mit mindestens zwei Alkoxysilylresten und eine Zinnverbindung und/oder organische Säuren und/oder Basen und/oder deren Salze als Kondensationskatalysator enthalten.

Härtbarte Systeme dieses Typs kommen bevorzugt in Form einer Mehrkomponenten Dentalabformmasse enthaltend Komponenten G und H zum Einsatz. Darin enthält
j) Komponente G einen vernetzbaren und Alkoxysilylreste aufweisenden Polyether,
k) Komponente H enthält Wasser,
l) mindestens eine der Komponenten G und/oder H enthalten einen Katalysator sowie ein Silizium aufweisendes nichtionisches Tensid und/oder ein nichtionisches Fluortensid nach Anspruch 1, und Komponenten G und H weisen eine solche Zusammensetzung auf, so dass nach dem Vermischen der Komponenten G und H eine Kombination von Silizium aufweisendem nichtionischem Tensid und nichtionischem Fluortensid nach Anspruch 1 vorliegt.

Als Alkoxysilylreste aufweisende Polyether kommen üblicherweise von Polyalkylenglykolen abgeleitete und mit Alkoxysilylresten, gegebenenfalls über eine Brückengruppe, terminierte Polymere in Frage.

Typischerweise handelt es sich dabei um Verbindungen der Formel XXIII

(RO)₃₋ₙ₅Rₙ₅Si-BG2-A-(BG2-SiRₙ₅(OR)₃₋ₙ₅)ₙ₁ (XXIII),

worin A und n1 die für Verbindungen der Formel XI definierte Bedeutung besitzen, BG2 für eine kovalente Bindung oder eine Brückengruppe ausgenommen -O- steht, n5 eine ganze Zahl von 0 bis 2 bedeutet,
R die für Verbindungen der Formeln XIV oder XV definierten Bedeutungen aufweist, und innerhalb eines Moleküls unterschiedliche Reste R, Br sowie unterschiedliche Indizes n5 im Rahmen der gegebenen Definition unterschiedliche Bedeutungen annehmen können, R insbesondere Alkylreste, vorzugsweise Methyl bedeuten, und BG2 insbesondere eine kovalente Bindung oder eine Brückengruppe -O-CO-NH-R'- ist, worin R' mit dem Siliziumatom verbunden ist und eine Alkylgruppe, vorzugsweise Methyl, Ethyl oder Propyl, bedeutet.

Alkoxysilylreste aufweisende Polyether können auch als Gemische von Polymeren unterschiedlicher Kettenlänge zum Einsatz gelangen. Typische Molekulargewichte (Zahlenmittel) bewegen sich im Bereich von 150 bis 3000000, vorzugsweise von 250 bis 100000 und besonders bevorzugt 250 bis 50000.

Als Katalysatoren können in diesem härtbaren System die weiter oben beschriebenen Organozinnverbindungen eingesetzt, beispielsweise Dibutylzinndilaurat. Es können aber auch organische Säuren, wie Toluolsulfonsäure, oder organische Basen, wie Amine, Guanidine, DBU oder DBN, oder Salze dieser Säuren oder Basen eingesetzt werden.

Weitere bevorzugte Dentalabformmassen enthalten Aziridinoreste enthaltende Polyether.

Härtbare Systeme dieses Typs sind beispielsweise aus US-A-4,353,242 bekannt.

In Zusammensetzungen enthaltend vernetzbare Polyether, die Alkoxysilylreste, Aziridinoreste, von einer ethylenisch ungesättigten Carbonsäure abgeleitete Reste oder Alkenylreste als vernetzbare Gruppen oder über ringöffnende Metathesereaktion vernetzbare Gruppen aufweisen kann gegebenenfalls der Einsatz von Silizium aufweisenden nichtionischen Tensiden entfallen, so dass hier nur ein nichtionisches Fluortensid oder ein Gemisch von nichtionischen Fluortensiden eingesetzt wird.

Dentalabformmassen enthaltend vernetzbare Polyether, die Alkoxysilylreste, Aziridinoreste, von einer ethylenisch ungesättigten Carbonsäure abgeleitete Reste oder Alkenylreste als vernetzbare Gruppen oder über ringöffnende Metathesereaktion vernetzbare Gruppen aufweisen, und ein nichtionisches Fluortensid, das mindestens einen teil- oder perfluorierten Kohlenwasserstoffrest aufweist, der über ein Sauerstoffatom, eine Aminogruppe, eine Ketogruppe, eine Carbonsäureestergruppe, eine Phosphorsäureestergruppe, eine Carbonsäureamid-gruppe und/oder eine Phosphorsäureamidgruppe mit einem (Poly)alkylenoxidrest, einem Kohlenhydratrest, einem aliphatischen Polyhydroxyrest oder einem Stickstoff enthaltenden heterocyclischen Rest verbunden ist oder das mindestens einen teil- oder perfluorierten Kohlenwasserstoffrest und mindestens einen Aminoxidrest aufweist, sind ebenfalls Gegenstand der vorliegenden Erfindung.

Besonders bevorzugte Dentalabformmassen dieses Typs enthalten vernetzbare Polyether, die Alkoxysilylreste oder Aziridinoreste als vernetzbare Gruppen aufweisen.

Auch bei diesen Dentalabformmassen kommen als zusätzliche Komponenten vorzugsweise Polyole und/oder Alkenylreste und/oder Alkinylreste enthaltende Polyether und/oder hydroxyl- und/oder alkoxy-terminierte Polyether zum Einsatz.

Vorzugsweise werden in diesen Dentalabformmassen jedoch Gemische von Silizium aufweisenden nichtionischen Tensiden und nichtionischen Fluortensiden gegebenenfalls in Kombination mit den oben erwähnten zusätzlichen Polyol- und/oder Polyetherkomponenten eingesetzt.

Härtbarte Systeme dieses Typs kommen üblicherweise in Form einer Mehrkomponenten Dentalabformmasse enthaltend Komponenten I und J zum Einsatz. Darin enthält
m) Komponente I einen vernetzbaren und Aziridinoreste oder Alkoxysilylreste aufweisenden Polyether oder einen über ringöffnende Metathesereaktion vernetzbare Gruppen enthaltenden Polyether,
n) Komponente J einen Katalysator, und
o) mindestens eine der Komponenten I und/oder J enthält ein nichtionisches Fluortensid und gegebenenfalls ein Silizium aufweisendes nichtionisches Tensid.

Vorzugsweise weisen Komponenten I und J eine solche Zusammensetzung auf, so dass nach dem Vermischen der Komponenten I und J eine Kombination von Silizium aufweisendem nichtionischem Tensid und nichtionischem Fluortensid nach Anspruch 1 vorliegt.

Als Aziridinoreste oder Alkoxysilylreste aufweisende Polyether kommen üblicherweise von Polyalkylenglykolen abgeleitete und mit Alkoxysilylresten oder mit Aziridinoresten über eine Brückengruppe terminierte Polymere in Frage.

Typischerweise handelt es sich dabei um Verbindungen der folgenden Formel XXVIII 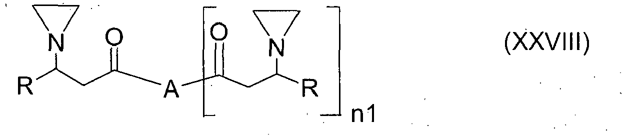 worin A und n1 die für Verbindungen der Formel XI definierte Bedeutung besitzen, R die für Verbindungen der Formeln XIV oder XV definierten Bedeutungen aufweist, und innerhalb eines Moleküls unterschiedliche Reste R im Rahmen der gegebenen Definition unterschiedliche Bedeutungen annehmen können, und R insbesondere Alkylreste, vorzugsweise Methyl bedeuten.

Aziridinoreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3000000, vorzugsweise von 250 bis 100000 und besonders bevorzugt 250 bis 50000.

Als Katalysatoren können in diesem härtbaren System Sulfoniumsalze eingesetzt werden.

Weitere bevorzugte Dentalabformmassen enthalten Esterreste einer ethylenisch ungesättigten Carbonsäure enthaltende Polyether.

Härtbare Systeme dieses Typs sind beispielsweise aus EP 0170219 A2 bekannt.

Bevorzugt werden Dentalabformmassen die einen vernetzbaren Polyalkylenether, der von Acrylsäure und/oder Methacrylsäure abgeleitete Reste aufweist, einen durch Hitze oder durch Strahlung aktivierbaren Initiator sowie ein Silizium aufweisendes nichtionisches Tensid und ein nichtionisches Fluortensid nach Anspruch 1 enthält.

Diese Dentalabformmassen können als Einkomponenten- oder als Zweikomponenten-Formulierungen eingesetzt werden.

Bevorzugt kommen Einkomponenten Dentalabformmasse zum Einsatz, die durch UV-Strahlung und/oder Hitze ausgehärtet werden. Neben dem härtbaren Polymersystem sowie der erfindungsgemäß eingesetzten Tensidmischung sind darin üblicherweise Photoinitiatoren enthalten.

Als vernetzbare Polyether kommen üblicherweise von Polyalkylenglykolen abgeleitete und mit ethylenisch ungesättigten Carbonsäuren terminierte Polymere in Frage.

Typischerweise handelt es sich dabei um Verbindungen der Formeln XXIV und/oder XXVIII

R⁵⁰-A-(CO-R⁵⁰)ₙ₁ (XXIV),

R⁵⁰-CO-A-(CO-R⁵⁰)ₙ₁ (XXVIII),

worin A und n1 die für Verbindungen der Formel XI definierte Bedeutung besitzen, R⁵⁰ ein ethylenisch ungesättigter Rest, vorzugsweise ein Alkenylrest ist und die Reste R⁵⁰ innerhalb eines Moleküls im Rahmen der gegebenen Bedeutung unterschiedlich sein können.

Ganz besonders bevorzugt ist R⁵⁰ CH₂=CH- oder CH₂=C(CH₃)-, also ein von Acrylsäure oder Methacrylsäure abgeleiteter Rest.

Ethylenisch ungesättigte Carbonsäureesterreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3000000, vorzugsweise von 250 bis 100000 und besonders bevorzugt 250 bis 50000.

Diese Polyether werden üblicherweise durch elektromagnetische Strahlung, vorzugsweise UV-Strahlung oder sichtbares Licht gehärtet. Als Photoinitiatoren können in diesem härtbaren System z.B. Campherchinon und/oder Amine eingesetzt werden.

In hitzehärtbaren Systemen werden vorzugsweise Peroxidhärter gegebenenfalls kombiniert mit Aminen eingesetzt.

Weitere bevorzugte Dentalabformmassen enthalten über ringöffnende Metathese Polymerisation (ROMP) vernetzbare Gruppen aufweisende Polyether, Polysiloxane und/oder Synthesekautschuke. Härtbare Systeme dieses Typs sind bekannt, z.B. aus EP 1317917 A1, US 6,649,146 B2, WO 02/32338 A2 und US 6,455,029.

Diese kommen üblicherweise in Form einer Mehrkomponenten Dentalabformmasse enthaltend Komponenten K und L zum Einsatz. Darin enthält
p) Komponente K Polyether, Polysiloxane und/oder Synthesekautschuke, die über ROMP vernetzbare Gruppen aufweisen,
q) Komponente L einen ROMP Vernetzungskatalysator, und
r) mindestens eine der Komponenten K und/oder L enthält ein Silizium aufweisendes nichtionisches Tensid und/oder ein nichtionisches Fluortensid nach Anspruch 1, und Komponenten K und L weisen eine solche Zusammensetzung auf, so dass nach dem Vermischen der Komponenten K und L eine Kombination von Silizium aufweisendem nichtionischem Tensid und nichtionischem Fluortensid nach Anspruch 1 vorliegt.

Als über ROMP vernetzbare Gruppen aufweisende Polyether, Polysiloxane und/oder Synthesekautschuke kommen üblicherweise von Polyalkylenglykolen, Polydialkyl- oder -arylsiloxanen und/oder Polyalkenen oder Polyalkandienen abgeleitete, mit ungesättigten end- und/oder seitenständigen über einen Spacer B gebundenen Resten MT versehene Polymere in Frage.

Bei den Polysiloxanen handelt es sich dabei typischerweise um Verbindungen der folgenden Formel XXV

MT-BG-(SiR₂O)ₘ₅-(SiR(MT)O)ₙ₆-BG-MT XXV,

worin R unabhängig voneinander Alkyl , Cycloalkyl, Aryl und/oder Aralkyl bedeutet, die gegebenenfalls substituiert sind,
m5 eine ganze Zahl von 10 bis 6000, vorzugsweise von 20 bis 2000,
n6 eine ganze Zahl von 0 bis 100, vorzugsweise von 0 bis 10 ist,
BG eine Brückengruppe bedeutet,
MT eine über ROMP vernetzbare Gruppe ist, und
die Reste MT, B2 und R sowie die Indizes m5 und/oder n6 innerhalb eines Moleküls im Rahmen der gegebenen Bedeutung unterschiedlich sein können.

Typische Molekulargewichte (Zahlenmittel) von Verbindungen der Formel XXV bewegen sich im Bereich von 900 bis 500000, vorzugsweise von 1500 bis 150000.

Organopolysiloxane mit mindestens zwei ethylenisch ungesättigten Gruppen kommen in der Regel als Gemische von Polymeren unterschiedlicher Kettenlänge zum Einsatz. Typische Molekulargewichte (Zahlenmittel) bewegen sich im Bereich von 900 bis 500000, vorzugsweise von 1500 bis 150000.

Besonders bevorzugt werden Organopolysiloxane der Formeln XXV, worin R Methyl ist.

Bei den Polyalkylenglycolen handelt es sich dabei typischerweise um Verbindungen der folgenden Formeln XXVI und/oder XXIX

MT-BG3-(Cₙ₆H₂ₙ₆O)ₘ₆-(Cₙ₆H₂ₙ₆₋₁(BG3-MT)O)ₘ₇-BG3-MT (XXVI),

MT-BG3-A-(BG3-MT))ₙ₁ (XXIX),

worin A und n1 die bei Verbindungen der formel XI definierte Bedeutung besitzen,
n6 eine ganze Zahl von 4 bis 8, vorzugsweise von 2 bis 4 bedeutet,
m6 eine ganz Zahl von 2 bis 70000, vorzugsweise von 10 bis 2500 ist,
m7 eine ganz Zahl von 0 bis 70000, vorzugsweise von 10 bis 2500 ist,
die Summe von m6 und m7 3 bis 70000, vorzugsweise 10 bis 2500 ist,
BG3 eine Brückengruppe bedeutet,
MT eine über ROMP vernetzbare Gruppe ist, und
die Reste MT und BG3 sowie die Indizes n6, m6 und/oder m7 innerhalb eines Moleküls im Rahmen der gegebenen Bedeutung unterschiedlich sein können.

Bei den Synthesekautschuken handelt es sich dabei typischerweise um Verbindungen der folgenden Formel XXVII 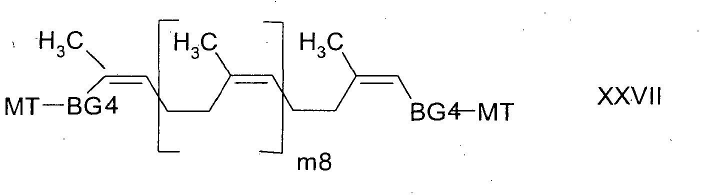
worin BG4 eine Brückengruppe bedeutet,
MT eine über ROMP vernetzbare Gruppe ist, und
m8 eine ganze Zahl von 1500 bis 30000, vorzugsweise 20 bis 500, ist.

Neben 1,4 cis und 1,4 trans Polyisopren und deren Mischformen sind Polymere von 1,3-Dienen, wie z.B. 2,3-Dimethyl-1,3-butadien, Polybutadien und Poly-(2-chlor-1,3-butadien) sowie Synthesekautschuke, die durch Copolymerisation zweier oder dreier verschiedener Monomere entstanden. Die wichtigsten synthetischen Kautschuke sind Styrol-Butadien-Kautschuk, Acrylnitril-Butadien-Kautschuk und Isobuten-Isopren-Kautschuk, Ethylen-Propylen-Copolymere (EPM), Ethylen-Propylen-Dien-Terpolymere (EPDM), ferner Elastomere auf der Basis von Polyurethan, Polysulfiden, Chlorsulfonylpolyethylen, wobei der Synthesekautschuk durch Vulkanisation quervernetzt vorliegen kann.

Die ungesättigten ROMP-vernetzbaren Gruppen MT sind z.B. Cycloalkenylgruppen, wie z.B. Cyclobutenyl-, Cyclopentenyl- oder Gruppen der allgemeinen Formel 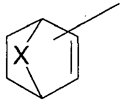 worin X = O, S, NH oder ein gesättigter oder ungesättigter C₁-C₃₀-Kohlenwasserstoffrest ist.

Bei der Brückengruppe BG4 in den oben aufgezählten Formeln handelt es sich vorzugsweise um -O- oder Alkylen, insbesondere um -CH₂-, -CH₂CH₂-, -CH₂CH₂CH₂- oder um -CH(CH₃)-CH₂-.

Als Katalysatoren werden in diesem härtbaren System Salze, Komplexe oder kollodial vorliegende Formen der Übergangsmetalle der 8. Nebengruppe eingesetzt. Vorzugsweise kommen Ruthenium, Osmium, Wolfram oder Molybdän als Komplexe zum Einsatz. Besonders bevorzugt werden Ruthenium-Carben-Komplexe eingesetzt, wie z.B. der Grubbs-Katalysator.

Die erfindungsgemäßen Dentalabformmassen enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzenden Esterreste einer ethylenisch ungesättigten Carbonsäure enthaltenden Polyether oder der durch ringöffnende Metathesereaktion vernetzenden Polyether, Silicone oder Kautschuke, enthalten vorzugsweise als weitere Komponente einen Alkenylreste enthaltenden Polyether und/oder einen hydroxyl- oder alkoxy-terminierten Polyether. Diese zusätzliche Komponente bewirkt eine weitere Verbesserung der Konaktwinkeleigenschaften des nicht ausgehärteten plastischen Materials und somit eine weitere Verbesserung der Detailgenauigkeit des entstehenden Abdrucks.

Beispiele für bevorzugte Zusätze dieses Typs sind die weiter oben beschriebenen Verbindungen der Formel XI, XII und XIII.

Die erfindungsgemäßen Dentalabformmassen können neben den vernetzbaren Polymeren und dem nichtionischen Fluortensid oder der Tensidmischung weitere Komponenten enthalten, die üblicherweise in solchen Massen eingesetzt werden. Beispiele für solche weiteren Komponenten sind Füllstoffe. Dabei kann es sich um verstärkende Füllstoffe oder um nicht-verstärkende Füllstoffe oder um Gemische davon handeln.

Als verstärkende Füllstoffe eignen sich insbesondere hochdisperse, aktive Füllstoffe mit einer BET-Oberfläche von wenigstens 50 m²/g. Besonders geeignet sind solche mit einer Einzelpartikelgröße im Nanometerbereich, welche als Aggregate und/oder Agglomerate vorliegen können. Bevorzugte verstärkende Füllstoffe sind Substanzen, die ausgewählt sind aus der Gruppe bestehend aus Aluminiumhydroxid, Zinkoxid, Titandioxid, Zirkoniumoxid, Siliciumdioxid sowie gefällter und/oder pyrogener Kieselsäure. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff in Form von Nanopartikeln, als faser- oder blättchenförmiger Füllstoff, beispielsweise als mineralischer, faserförmiger Füllstoff, oder als synthetischer, faserförmiger Füllstoff vor.

Der Anteil an verstärkendem Füllstoff in der erfindungsgemäßen Dentalabformmasse beträgt üblicherweise 0 bis 80 Gew.-%, bevorzugt 0,1 bis 50 Gew.-% und besonders bevorzugt 0,1 bis 40 Gew.-%, bezogen auf die gesamte Dentalabformmasse.

Als nichtverstärkende Füllstoffe eignen sich prinzipiell dieselben Substanzen wie für die verstärkende Füllstoffe, wobei die nichtverstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugte nichtverstärkende Füllstoffe sind Substanzen, die ausgewählt werden aus der Gruppe bestehend aus Erdalkalimetalloxiden, Erdalkalimetallhydroxiden, Erdalkalimetallfluoriden, Erdalkalimetallcarbonaten, Calciumapatit (Ca₅[(F, Cl, OH, ½CO₃) | (PO₄)₃], insbesondere Calciumhydroxylapatit (Ca₅[(OH) | (PO₄)₃], Titandioxid, Zirkoniumoxid, Aluminiumhydroxid, Siliciumdioxid, gefällter Kieselsäure und Calciumcarbonat. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe eine mittlere Korngröße von größer als 0,1 µm (Ullmann Encyclopädie der Technischen Chemie, Band 21, Seite 523) auf.

Der Anteil an nichtverstärkendem Füllstoff in der erfindungsgemäßen Dentalabformmasse beträgt üblicherweise 0 bis 80 Gew.-%, bevorzugt 0,1 bis 50 Gew.-% und besonders bevorzugt 0,1 bis 40 Gew.-%, bezogen auf die gesamte Dentalabformmasse.

Der Gesamtanteil an verstärkenden und nichtverstärkenden Füllstoffen in der erfindungsgemäßen Dentalabformmasse beträgt üblicherweise 0 bis 80 Gew.-%, bevorzugt 0,01 bis 80 Gew.-%, besonders bevorzugt 0,05 bis 75 Gew.-%, und ganz besonders bevorzugt 0,1 bis 70 Gew.-%, bezogen auf die gesamte Dentalabformmasse.

Die erfindungsgemäßen Dentalabformmassen können darüber hinaus ein oder mehrere der folgenden Zusatzstoffe enthalten: Puffersalze, Wasserfänger, Pastenbildner, weitere Tenside, Wirkstoffe, Weichmacher, optische Abtastung ermöglichende Substanzen, Geschmacks- und/oder Geruchsstoffe, Diagnostik ermöglichende Substanzen, Fluoridisierungsmittel, Bleichsubstanzen, Desensibilisierungsmittel, Haftverbundvermittler, Farbstoffe, Indikatoren, Stabilisatoren (Antioxidantien) sowie antibakterielle Substanzen.

Liegt die erfindungsgemäße Dentalabformmasse als Mehrkomponentensystem vor, so wird diese vorzugsweise in geeigneten Primärverpackungen, wie Tuben, Dosen, und besonders bevorzugt in Kartuschen und Schlauchbeuteln, wie sie z.B. in der EP-A-723,807, der EP-A-541,972, der WO 98/44860 A1, der EP-A-492,412, der EP-A-492,413 und der EP-A-956,908 beschrieben sind, gelagert und auf die spätere Verwendung zugeschnitten proportioniert ist.

Ein weiterer Gegenstand der vorliegenden Erfindung sind Mischungen, welche durch Vermischen einzelner Komponenten von Mehrkomponentenmassen des zuvor beschriebenen Dentalabformmaterials erhältlich sind. Vorzugsweise wird eine Basiskomponente mit einer Katalysatorkomponente in einem Verhältnis von 1:2 bis 20:1, besonders bevorzugt von 1:1 bis 10:1 und ganz besonders bevorzugt von 10:1, 5:1, 4:1, 2:1 und 1:1, vermischt. Diese Mischungen zeichnen sich durch eine ausgezeichnete Benetzbarkeit und ein hervorragendes Anfließverhalten an feuchte Zahn- und Gewebssubstänz aus. Trotz dieser guten hydrophilen Eigenschaften quillt das Material nicht bei Kontakt mit wässrigen Medien, wie Wasser, Speichel, Blut, Desinfektionsbad oder wässrigem Gipsbrei. Die gute initiale Benetzbarkeit der Mischungen ist wichtig für die detailgetreue Abformung des Abformmaterials in dem Patientenmund während der Verarbeitung und dem ersten Kontakt mit feuchter Mund-/Zahnsubstanz und drückt sich durch einen äußerst niedrigen Kontaktwinkel aus, der sich rasch nach dem Vermischen der Komponenten bzw. nach dem Beginn der Härtung ausbildet. Die erfindungsgemäßen Dentalabformmassen zeichnen sich dadurch aus, dass zwischen 30 Sekunden nach Mischbeginn der Komponenten des härtbaren Polymersystems, z.B. der Katalysatorkomponente und der Basiskomponente, oder des Auslösens der Härtung durch Bestrahlung bis zum Abbinden des härtbaren Polymersystems bei einem Wassertropfenalter in den ersten 10 Sekunden, bevorzugt in den ersten 5 Sekunden nach Aufsetzen des Wassertropfens auf die Oberfläche des Dentalabformmaterials, Kontaktwinkel von kleiner gleich 10° erreicht werden. Die Kontaktwinkel werden dabei mit einem Kontaktwinkelmessgerät G40/G23M der Firma Krüss bei 23°C ± 1°C mit der Messmethode "liegender Tropfen" gemessen. Die Messung erfolgte bei 50 % rel. Luftfeuchtigkeit.

Dazu wurden dynamische Kontaktwinkelbestimmungen am liegenden Tropfen mit dem Kontaktwinkelmessgerät Tropfenkonturanalyse-System DSA100 (Fa. Krüss, D-Hamburg), das mit einem vollautomatischen Tropfendosiersystem kombiniert war, bei Raumtemperatur 23°C ± 1°C durchgeführt. Die Messung erfolgte ebenfalls bei 50 % rel. Luftfeuchtigkeit.

Die Benetzung wurde 40 s, 60 s, 90 s und 120 s nach Mischbeginn des jeweiligen 2-Komponenten-Dentalabformmaterials, d.h. während dessen Verarbeitungszeit, durch Platzieren eines Wassertropfens mit einem Volumen von 2 µl auf die polymerisierende Oberfläche initiiert. Dabei wurde jeweils für die Dauer einer Einzelmessung von 180 s die dynamische Veränderung der Tropfengeometrie mit einer Auflösung von 10 Bildern pro Sekunde erfasst und mit der Herstellersoftware ausgewertet. Die Kontaktwinkel der Einzelbilder wurden nach der Methode "Circle fit" als gemittelte Kontaktwinkel aus den linken und rechten Kontaktwinkeln der einzelnen Tropfenkonturen ermittelt.

Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 10° bei einem Wassertropfenalter in den ersten 10 Sekunden, bevorzugt 5 Sekunden nach Aufsetzen des Wassertropfens auf die Oberfläche des Dentalabformmaterials aus.

Die Erfindung betrifft auch das ausgehärtete Abformmaterial, das sich durch Härten der oben beschriebenen Dentalabformmassen erhalten lässt. Das ausgehärtete Abformmaterial zeichnet sich durch ausgezeichnete mechanische Eigenschaften aus und erfüllt alle Anforderungen nach ISO 4823 an ein elastomeres Dentalabformmaterial.

Die Erfindung betrifft weiterhin die Verwendung einer Mischung aus Silizium aufweisendem nichtionischem Tensid, das mindestens einen (Poly)alkylenoxidrest und eine Molmasse von weniger als 6000 g/mol aufweist, und aus nichtionischem Fluortensid nach Anspruch 1, zur Herstellung von elastomeren Dentalabformmassen.

Die Erfindung betrifft weiterhin die Verwendung einer Mischung enthaltend Silizium aufweisendes nichtionisches Tensid, das mindestens einen (Poly)alkylenoxidrest und eine Molmasse von weniger als 6000 g/mol aufweist, nichtionisches Fluortensid nach Anspruch 1, und einen Alkenyl-, Alkinyl-, Alkyloxy-, Aryloxy-, Arylalkyloxy- und/oder Hydroxy-endgestopptem Polyalkylenether oder ein Gemisch solcher Verbindungen zur Herstellung von elastomeren Dentalabformmassen.

Die Erfindung betrifft außerdem die Verwendung einer Mischung enthaltend Silizium aufweisendes nichtionisches Tensid, das mindestens einen (Poly)alkylenoxidrest und eine Molmasse von weniger als 6000 g/mol aufweist, nichtionisches Fluortensid nach Anspruch 1, und einen Alkenyl-, Alkinyl-, Alkyl- und/oder Hydroxy-endgestopptem Polyalkylenether oder ein Gemisch solcher Verbindungen zur Herstellung von elastomeren Dentalabformmassen.

Die folgenden Beispiele erläutern die Erfindung ohne diese zu beschränken.

In den Beispielen wurden die folgenden Verbindungen eingesetzt:

| | |
|---|---|
| Siloxan I | α, ω-Divinylpolydimethylsiloxan mit einer Viskosität bei 20°C von etwa 1000 mPas |
| Siloxan II | Polymethylhydrogensiloxan mit einer Viskosität bei 20°C von etwa 200 mPas und einem SiH-Gehalt von 1,8 mmol/g |
| Kieselsäure I | pyrogen hergestellte, hochdisperse, hydrophobierte Kieselsäure mit einer BET-Oberfläche von 170 m²/g |
| Quarzmehl I | Quarzmehl mit einer mittleren Korngröße von 10 µm |
| Katalysator I | Platin-Katalysator vom Karstedt-Typ mit einem Gehalt an reinem Platin von 1,0 % |
| Tensid I | nichtionisches alkylverschlossenes Fettalkoholethoxylat-Tensid mit einer Oberflächenspannung von 29 mN/m (in entionisiertem Wasser, bei 20°C, bei einer Konzentration von 1 g/l) und einer Molmasse von etwa 420 g/mol |
| Tensid II | Bestandteil der erfindungsgemäß eingesetzten Mischung; nichtionisches Fluoralkylethoxylat mit einer Oberflächenspannung von 19 dyn/cm (in entionisiertem Wasser, bei 25°C, bei einer Konzentration von 0,01 %), einem HLB-Wert von 11,5 und einer Molmasse von ca. 725 g/mol |
| Tensid III | Bestandteil der erfindungsgemäß eingesetzten Mischung; polyethylenoxid modifiziertes Polydimethylsiloxan (PEG-8-Methicone) mit einer Oberflächenspannung von 20,7 dyn/cm (in entionisiertem Wasser, bei 25°C, bei einer Konzentration von 1 %), einem HLB-Wert von 10,4 und einer Molmasse von ca. 620 g/mol |
| Tensid IV | Bestandteil der erfindungsgemäß eingesetzten Mischung; polyethylenoxid modifiziertes Polydimethylsiloxan (PEG-7-Methicone) mit einer Oberflächenspannung von 20 dyn/cm (in entionisiertem Wasser, bei 25°C, bei einer Konzentration von 0,1 %), einem HLB-Wert von 6,5 und einer Molmasse von ca. 600 g/mol |
| Tensid V | C₁₂-Fettalkoholethoxylat mit vier Ethylenoxid-Einheiten |
| Tensid VI | Nonylphenylethoxylat mit fünf Ethylenoxid-Einheiten |
| Tensid VII | nichtionisches Fluortensid Fluorad 4430 mit einer Oberflächenspannung von 21 dyn/cm (in entionisiertem Wasser, bei 25°C, bei einer Konzentration von 0,1%) |
| Tensid VIII | siloxanbasiertes Geminitensid TEGO® Twin 4000 (Tego Chemie Service GmbH) |
| Tensid IX | polyethylenoxid modifiziertes Polydimethylsiloxan (CAS 68938-54-5) mit einer Oberflächenspannung von 26,6 dyn/cm (in entionisiertem Wasser, bei 25°C, bei einer Konzentration von 0,1%), mit einer Molmasse von ca. 10000 g/Mol |
| Tensid X | nichtionisches Fluortensid mit Perfluoroctylsulfonatgruppe (Fluorad FC430) bekannt aus DE 43 06 997 A1 |
| Polyether I | Bestandteil der erfindungsgemäß eingesetzten Mischung; Polyethylenglycoldimethylether mit einer mittleren Molmasse von 500 g/mol |
| Polyether II | Bestandteil der erfindungsgemäß eingesetzten Mischung; α, ω-Diallylpolyethylenglycol mit einer mittleren Molmasse von 2000 g/mol |
| Polyether III | Bestandteil der erfindungsgemäß eingesetzten Mischung; Polyethylenglycol mit einer mittleren Molmasse von 10000 g/mol |

### Herstellungsbeispiel A1: Katalysatormasse eines additionsvernetzenden Zweikomponenten-Silicon-Dentalabformmaterials

In einem Vakuummischer wurden 50 Teile eines Siloxan I, 6 Teile einer Kieselsäure I, 43 Teile Quarzmehl I un ein Teil eines Katalysators I für 1,5 Stunden homogen gemischt. Es wurde eine dünnfließende Paste (ISO 4823) erhalten. Die Paste stellt eine mögliche Katalysatorkomponente der erfindungsgemäßen Zweikomponenten-Dentalabformmasse dar.

### Herstellungsbeispiel A2: Katalysatormasse eines additionsvernetzenden Zweikomponenten-Silicon-Dentalabformmaterials

In einem Vakuummischer wurden 50 Teile eines Siloxan I, 6 Teile einer Kieselsäure I, 43 Teile Quarzmehl I und ein Teil eines Katalysators I für 1,5 Stunden homogen gemischt. Zu dieser Mischung wurden 0,4 Teile eines Tensids I, wobei das Tensid mit Hydroxyanisol stabilisiert war, gegeben und weitere 15 Minuten im Vakuum gemischt und entgast. Es wurde eine dünnfließende Paste (ISO 4823) erhalten. Die Paste stellt eine mögliche Katalysatorkomponente der erfindungsgemäßen Zweikomponenten-Dentalabformmasse dar.

### Herstellungsbeispiel A3: Grundrezeptur einer Basismasse eines additionsvernetzenden Zweikomponenten-Silicon-Dentalabformmaterials

In einem Vakuummischer wurden 20 Teile eines Siloxan I, 19 Teile eines Siloxan II mit 5 Teilen einer Kieselsäure I und 44 Teilen Quarzmehl I für 1,5 Stunden homogen gemischt und danach 15 Minuten entgast. Die erhaltene Paste stellt eine Grundrezeptur einer Basiskomponente der erfindungsgemäßen Zweikomponenten-Dentalabformmasse dar, auf die in den folgenden Beispielen für die Zugabe der erfindungsgemäßen synergistischen Tensid-(Polyether-)Mischung zurückgegriffen wird.

### Herstellungsbeispiel A4: Basismasse eines additionsvernetzenden Zweikomponenten-Silicon-Dentalabformmaterials mit einer erfindungsgemäßen synergistisch wirkenden Tensid-Polyether-Mischung

93 Teile der Grundrezeptur aus Herstellungsbeispiel A3 wurden in einem Vakuummischer mit 0,75 Teilen eines Tensids II, 2,25 Teilen eines Tensids III, und 4 Teilen eines Polyethers I für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel A5: Basismasse eines additionsvernetzenden Zweikomponenten-Silicon-Dentalabformmaterials mit einer erfindungsgemäßen synergistisch wirkenden Tensid-(Polyether-)Mischung

87 Teile der Grundrezeptur aus Herstellungsbeispiel A3 wurden in einem Vakuummischer mit 0,75 Teilen eines Tensids II, 2,25 Teilen eines Tensids III und 10 Teilen eines Polyethers II für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel A6: Basismasse eines additionsvernetzenden Zweikomponenten-Silicon-Dentalabformmaterials mit einer erfindungsgemäßen synergistisch wirkenden Tensid-Mischung

97 Teile der Grundrezeptur aus Herstellungsbeispiel A3 wurden in einem Vakuummischer mit 0,75 Teilen eines Tensids II und 2,25 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel AA6: Basismasse eines additionsvernetzenden Zweikomponenten-Silicon-Dentalabformmaterials mit einer erfindungsgemäßen synergistisch wirkenden Tensid-Polyol-Mischung

96 Teile der Grundrezeptur aus Herstellungsbeispiel A3 wurden in einem Vakuummischer mit 0,75 Teilen eines Tensids II, 2,25 Teilen eines Tensids III und 1,00 Teilen Glycerin für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel AAA6: Basismasse eines additionsvernetzenden Zweikomponenten-Silicon-Dentalabformmaterials mit einer erfindungsgemäßen synergistisch wirkenden Tensid-Polyol-Polyether-Mischung

94,5 Teile der Grundrezeptur aus Herstellungsbeispiel A3 wurden in einem Vakuummischer mit 0,75 Teilen eines Tensids II, 2,25 Teilen eines Tensids III, 1,50 Teilen Glycerin und 1,00 Teilen α,ω-Diallylpolyethylenglycol mit einer mittleren Molmasse von 1200 g/mol für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel A7: Basismasse eines additionsvernetzenden Zweikomponenten-Silicon-Dentalabformmaterials mit einer erfindungsgemäßen synergistisch wirkenden Tensid-Mischung

97 Teile der Grundrezeptur aus Herstellungsbeispiel A3 wurden in einem Vakuummischer mit 1,5 Teilen eines Tensids II und 1,5 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel A8: Basismasse eines additionsvernetzenden Zweikomponenten-Silicon-Dentalabformmaterials mit einer erfindungsgemäßen synergistisch wirkenden Tensid-Mischung

97 Teile der Grundrezeptur aus Herstellungsbeispiel A3 wurden in einem Vakuummischer mit 0,75 Teilen eines Tensids II und 2,25 Teilen eines Tensids IV für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel A9: Basismasse eines additionsvernetzenden Zweikomponenten-Silicon-Dental-abformmaterials mit einer synergistisch wirkenden Tensid-Polyether-Mischung

94 Teile der Grundrezeptur aus Herstellungsbeispiel A3 wurden in einem Vakuummischer mit 0,75 Teilen eines Tensids II, 2,25 Teilen eines Tensids III und 3 Teilen eines Polyethers III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Beispiel A1: Additionsvernetzende Zweikomponenten-Silicon-Dentalabformmasse mit synergistisch wirkender Tensid-Polyether-Mischung

50 Teile der in Herstellungsbeispiel A1 beschriebenen Katalysatorkomponente und 50 Teile der in Herstellungsbeispiel A4 beschriebenen Basiskomponente wurden aus einer Kartusche (Fa. Mixpac) ausgedrückt und über einen statischen Mischer (Fa. Mixpac) homogen gemischt.

Ergebnis 1A: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 1A und 1 B). Es zeigte sich, dass in jeder Phase während der Verarbeitungszeit der Abformmasse ein aufgebrachter Wassertropfen innerhalb von einer bis zehn Sekunden vollständig auf der Oberfläche der Abformmasse spreitet und diese benetzt. Ein solches Material ist in hervorragender Weise geeignet im Patientenmund trotz Einwirkung von feuchter Zahnsubstanz und Speichel an den abzuformenden Bereichen anzufließen und diese präzise in der Abformung abzubilden. Im Vergleich zu handelsüblichen, nicht mit der erfindungsgemäßen synergistischen Tensidmischung versehenen additionsvernetzenden Silicon-Dentalabformmaterial (Vergleichsbeispiele A9, A10), wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabform-materialien in hervorragender Weise geeignet im Patientenmund trotz Einwirkung von feuchter Zahnsubstanz, Speichel und Blut an die abzuformenden Bereiche anzufließen und diese präzise in der Abformung abzubilden.

### Beispiel A2: Additionsvernetzende Zweikomponenten-Silicon-Dentalabformmasse mit synergistisch wirkender Tensid-Polyether-Mischung

50 Teile der in Herstellungsbeispiel A2 beschriebenen Katalysatorkomponente und 50 Teile der in Herstellungsbeispiel A4 beschriebenen Basiskomponente wurden aus einer Kartusche (Fa. Mixpac) ausgedrückt und über einen statischen Mischer (Fa. Mixpac) homogen gemischt.

### Ergebnis 2A: wie Ergebnis 1A

### Beispiel A3: Additionsvernetzende Zweikomponenten-Silicon-Dentalabformmasse mit synergistisch wirkender Tensid-Polyether-Mischung

50 Teile der in Herstellungsbeispiel A2 beschriebenen Katalysatorkomponente und 50 Teile der in Herstellungsbeispiel A5 beschriebenen Basiskomponente wurden aus einer Kartusche (Fa. Mixpac) ausgedrückt und über einen statischen Mischer (Fa. Mixpac) homogen gemischt.

### Ergebnis 3A: wie Ergebnis 1A

### Beispiel A4: Additionsvernetzende Zweikomponenten-Silicon-Dentalabformmasse mit synergistisch wirkender Tensid-Mischung

50 Teile der in Herstellungsbeispiel A2 beschriebenen Katalysatorkomponente und 50 Teile der in Herstellungsbeispiel A6 beschriebenen Basiskomponente wurden aus einer Kartusche (Fa. Mixpac) ausgedrückt und über einen statischen Mischer (Fa. Mixpac) homogen gemischt.

### Ergebnis 4A: wie Ergebnis 1A

### Beispiel AA4: Additionsvernetzende Zweikomponenten-Silicon-Dentalabformmasse mit synergistisch wirkender Tensid-Polyol-Mischung

50 Teile der in Herstellungsbeispiel A2 beschriebenen Katalysatorkomponente und 50 Teile der in Herstellungsbeispiel AA6 beschriebenen Basiskomponente wurden aus einer Kartusche (Fa. Mixpac) ausgedrückt und über einen statischen Mischer (Fa. Mixpac) homogen gemischt.

### Ergebnis 4AA: wie Ergebnis 1A

### Beispiel AAA4: Additionsvernetzende Zweikomponenten-Silicon-Dentalabformmasse mit synergistisch wirkender Tensid-Polyol-Polyether-Mischung

50 Teile der in Herstellungsbeispiel A2 beschriebenen Katalysatorkomponente und 50 Teile der in Herstellungsbeispiel AAA6 beschriebenen Basiskomponente wurden aus einer Kartusche (Fa. Mixpac) ausgedrückt und über einen statischen Mischer (Fa. Mixpac) homogen gemischt.

### Ergebnis 4AAA: wie Ergebnis 1A

### Beispiel A5: Additionsvernetzende Zweikomponenten-Silicon-Dentalabformmasse mit synergistisch wirkender Tensid-Mischung

50 Teile der in Herstellungsbeispiel A2 beschriebenen Katalysatorkomponente und 50 Teile der in Herstellungsbeispiel A7 beschriebenen Basiskomponente wurden aus einer Kartusche (Fa. Mixpac) ausgedrückt und über einen statischen Mischer (Fa. Mixpac) homogen gemischt.

### Ergebnis 5A: wie Ergebnis 1A

### Beispiel A6: Additionsvernetzende Zweikomponenten-Silicon-Dentalabformmasse mit synergistisch wirkender Tensid-Mischung

50 Teile der in Herstellungsbeispiel A2 beschriebenen Katalysatorkomponente und 50 Teile der in Herstellungsbeispiel A8 beschriebenen Basiskomponente wurden aus einer Kartusche (Fa. Mixpac) ausgedrückt und über einen statischen Mischer (Fa. Mixpac) homogen gemischt.

### Ergebnis 6A: wie Ergebnis 1A

### Beispiel A7: Additionsvernetzende Zweikomponenten-Silicon-Dentalabformmasse mit einer synergistisch wirkenden Tensid-Polyether-Mischung

50 Teile der in Herstellungsbeispiel A2 beschriebenen Katalysatorkomponente und 50 Teile der in Herstellungsbeispiel A9 beschriebenen Basiskomponente wurden aus einer Kartusche (Fa. Mixpac) ausgedrückt und über einen statischen Mischer (Fa. Mixpac) homogen gemischt.

### Ergebnis 7A: wie Ergebnis 1A

### Herstellungs-Vergleichsbeispiel A1: Basiskomponente einer additionsvernetzenden Zweikomponenten-Silicon-Dentalabformmasse mit einer nicht synergistisch wirkenden Tensidmischung

97 Teile der Grundrezeptur aus Herstellungsbeispiel A3 wurden in einem Vakuummischer mit 0,75 Teilen eines Tensids II und 2,25 Teilen eines Tensids V für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A2: Basiskomponente einer additionsvernetzenden Zweikomponenten-Dentalabformmasse mit einer nicht synergistisch wirkenden Tensidmischung

97Teile der Grundrezeptur aus Herstellungsbeispiel A3 wurden in einem Vakuummischer mit 0,75 Teilen eines Tensids II und 2,25 Teilen eines Tensids VI für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A3: Basiskomponente einer additionsvernetzenden Zweikomponenten-Dentalabformmasse ausschließlich mit dem Fluortensid aus den Beispielen A1 bis A7, d.h. nicht in Abmischung mit Silicontensid

98,5 Teile der Grundrezeptur aus Herstellungsbeispiel A3 wurden in einem Vakuummischer mit 1,50 Teilen eines Tensids II für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A4: Basiskomponente einer additionsvernetzenden Zweikomponenten-Dentalabformmasse ausschließlich mit dem Fluortensid aus den Beispielen A1 bis A7, d.h. nicht in Abmischung mit Silicontensid bzw. Silicontensid und Polyetherpolymer

97 Teile der Grundrezeptur aus Herstellungsbeispiel A3 wurden in einem Vakuummischer mit 3 Teilen eines Tensids II für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A5: Basiskomponente einer additionsvernetzenden Zweikomponenten-Dentalabformmasse ausschließlich mit dem Silicontensid aus den Beispielen A1 bis A5, d.h. nicht in synergistischer Mischung mit Fluortensid bzw Fluortensid und Polyetherpolymer

97 Teile der Grundrezeptur aus Herstellungsbeispiel A3 wurden in einem Vakuummischer mit 3 Teilen eines Tensids III für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A6: Basiskomponente einer additionsvernetzenden Zweikomponenten-Dentalabformmasse ausschließlich mit dem Silicontensid aus Beispiel A6, d.h. nicht in synergistischer Mischung mit Fluortensid und Polyetherpolymer

97 Teile der Grundrezeptur aus Herstellungsbeispiel A3 wurden in einem Vakuummischer mit 3 Teilen eines Tensids IV für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A7: Basiskomponente einer additionsvernetzenden Zweikomponenten-Dentalabformmasse mit einem Tensid V

97 Teile der Grundrezeptur aus Herstellungsbeispiel A3 wurden in einem Vakuummischer mit 3 Teilen eines Tensids V für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A8: Basiskomponente einer additionsvernetzenden Zweikomponenten-Dentalabformmasse mit einem Tensid VI

97 Teile der Grundrezeptur aus Herstellungsbeispiel A3 wurden in einem Vakuummischer mit 3 Teilen eines Tensids VI für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A9: Basiskomponente einer additionsvernetzenden Zweikomponenten-Silicon-Dentalabformmasse mit einer nicht synergistisch wirkenden Tensidmischung aus dem besten Silicontensid (run 2) und Fluortensid (run 11) aus Beispiel 1 der US-A-4,657,959

99 Teile der Basiskomponente aus Beispiel 1, run 2, der US-A-4,657,959 wurden in einem Vakuummischer mit 1 Teil eines Tensids VII für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A10: Basiskomponente einer additionsvernetzenden Zweikomponenten-Silicon-Dental-abformmasse mit einer nicht synergistisch wirkenden Tensidmischung aus dem besten Silicontensid (run 2) und Fluortensid (run 11) aus Beispiel 1 der US-A-4,657,959

97 Teile der Basiskomponente aus Beispiel 1, run 2 der US-A-4,657,959 wurden in einem Vakuummischer mit 2 Teilen eines Tensids VII und einem Teil eines Tensids IV für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A11: Basiskomponente einer additionsvernetzenden Zweikomponenten-Silicon-Dentalabformmasse mit einer nicht synergistisch wirkenden Tensidmischung

97 Teile der Grundrezeptur aus Herstellungsbeispiel A3 wurden in einem Vakuummischer mit 0,75 Teilen eines Tensids II und 2,25 Teilen eines Tensid IX für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Herstellungs-Vergleichsbeispiel A12: Basiskomponente einer additionsvernetzenden Zweikomponenten-Silicon-Dentalabformmasse mit einer nicht synergistisch wirkenden Tensidmischung

97 Teile der Grundrezeptur aus Herstellungsbeispiel A3 wurden in einem Vakuummischer mit 0,75 Teilen eines Tensids II und 2,25 Teilen des Tensids VIII für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Hetstellungs-Vergleichsbeispiel A13: Basismasse eines additionsvernetzenden Zweikomponenten-Silicon-Dentalabformmaterials mit einer nicht synergistisch wirkenden Tensid-Polyether-Mischung

93 Teile der Grundrezeptur aus Herstellungsbeispiel A3 wurden in einem Vakuummischer mit 0,75 Teilen eines Tensids X, 2,25 Teilen eines Tensids III und 4 Teilen eines Polyethers III für 15 Minuten homogen gemischt und entgast. Es wurde eine dünnfließende Paste (ISO 4823) erhalten. Die Paste stellt eine mögliche Basiskomponente einer nicht erfindungsgemäßen Zweikomponenten-Dentalabformmasse dar.

### Vergleichsbeispiel A1: Additionsvernetzende Zweikomponenten-Silicon-Dentalabformmasse mit einer Tensidmischung, die nicht synergistisch wirkt.

50 Teile der in Herstellungsbeispiel A2 beschriebenen Katalysatorkomponente und 50 Teile der in Herstellungs-Vergleichsbeispiel A1 beschriebenen Basiskomponente wurden aus einer Kartusche (Fa. Mixpac) ausgedrückt und über einen statischen Mischer (Fa. Mixpac) homogen gemischt.

Ergebnis 8A: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 1A und 1 B). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wurde. Die aufgebrachten Wassertropfen spreiten nicht auf der Oberfläche der Abformmasse und benetzen diese nicht ausreichend. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 38 und 44°. Dieses Vergleichsbeispiel zeigt, dass nicht jede beliebige Kombination von Tensiden zu einem synergistischen Effekt führt.

### Vergleichsbeispiel A2: Additionsvernetzende Zwei komponenten-Silicon-Dentalabformmasse mit einer Tensidmischung, die nicht synergistisch wirkt

50 Teile der in Herstellungsbeispiel A2 beschriebenen Katalysatorkomponente und 50 Teile der in Herstellungs-Vergleichsbeispiel A2 beschriebenen Basiskomponente wurden aus einer Kartusche (Fa. Mixpac) ausgedrückt und über einen statischen Mischer (Fa. Mixpac) homogen gemischt.

Ergebnis 9A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 65 und 75°. Dieses Vergleichsbeispiel zeigt, dass nicht jede beliebige Kombination von Tensiden zu einem synergistischen Effekt führt.

### Vergleichsbeispiel A3: Additionsvernetzende Zweikomponenten-Silicon-Dentalabformmasse ausschließlich mit dem Fluortensid aus den Beispielen A1 bis A7, d.h. nicht in Abmischung mit Silicontensid

50 Teile der in Herstellungsbeispiel A2 beschriebenen Katalysatorkomponente und 50 Teile der in Herstellungs-Vergleichsbeispiel A3 beschriebenen Basiskomponente wurden aus einer Kartusche (Fa. Mixpac) ausgedrückt und über einen statischen Mischer (Fa. Mixpac) homogen gemischt.

Ergebnis 10A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 60 und 72°. Dieses Vergleichsbeispiel zeigt, dass dasselbe Fluortensid, das in den Beispielen A1 bis A7 in der erfindungsgemäßen synergistischen Tensidmischung eingesetzt wurde, alleine nicht zu dem erfindungsgemäßen Effekt eines spontanen Spreitens eines aufgebrachten Wassertropfens auf der Oberfläche des Dentalabformmaterials führt.

### Vergleichsbeispiel A4: Additionsvernetzende Zweikomponenten-Silicon-Dentalabformmasse ausschließlich mit dem Fluortensid aus den Beispielen A1 bis A7, d.h. nicht in Abmischung mit Silicontensid bzw. Silicontensid und Polyetherpolymer

50 Teile der in Herstellungsbeispiel A2 beschriebenen Katalysatorkomponente und 50 Teile der in Herstellungs-Vergleichsbeispiel A4 beschriebenen Basiskomponente wurden aus einer Kartusche (Fa. Mixpac) ausgedrückt und über einen statischen Mischer (Fa. Mixpac) homogen gemischt.

Ergebnis 11A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 44 und 66°. Dieses Vergleichsbeispiel zeigt, dass dasselbe Fluortensid, das in den Beispielen A1 bis A7 in der erfindungsgemäßen synergistischen Tensid-(Polyether)-Mischung eingesetzt wurde, alleine nicht zu dem erfindungsgemäßen Effekt eines spontanen Spreitens eines aufgebrachten Wassertropfens auf der Oberfläche des Dentalabformmaterials führt.

### Vergleichsbeispiel A5: Additionsvernetzende Zweikomponenten-Silicon-. Dentalabformmasse ausschließlich mit dem Silicontensid aus den Beispielen A1 bis A5, d.h. nicht in der synergistischen Mischung mit Fluortensid bzw. Fluortensid und Polyetherpolymer

50 Teile der in Herstellungsbeispiel A2 beschriebenen Katalysatorkomponente und 50 Teile der in Herstellungs-Vergleichsbeispiel A5 beschriebenen Basiskomponente wurden aus einer Kartusche (Fa. Mixpac) ausgedrückt und über einen statischen Mischer (Fa. Mixpac) homogen gemischt.

Ergebnis 12A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 42 und 45°. Dieses Vergleichsbeispiel zeigt, dass dasselbe Silicontensid, das in den Beispielen A1 bis A5 in der erfindungsgemäßen synergistischen Tensid-(Polyether)-Mischung eingesetzt wurde, alleine nicht zu dem erfindungsgemäßen Effekt eines spontanen Spreitens eines aufgebrachten Wassertropfens auf der Oberfläche des Dentalabformmaterials führt.

### Vergleichsbeispiel A6: Additionsvernetzende Zweikomponenten-Silicon-Dentalabformmasse ausschließlich mit dem Silicontensid aus Beispiel A6, d.h. nicht in der synergistischen Mischung mit Fluortensid und Polyetherpolymer

50 Teile der in Herstellungsbeispiel A2 beschriebenen Katalysatorkomponente und 50 Teile der in Herstellungs-Vergleichsbeispiel A6 beschriebenen Basiskomponente wurden aus einer Kartusche (Fa. Mixpac) ausgedrückt und über einen statischen Mischer (Fa. Mixpac) homogen gemischt.

Ergebnis 13A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 33 und 42°. Dieses Vergleichsbeispiel zeigt, dass dasselbe Silicontensid, das in Beispiel A6 in der erfindungsgemäßen synergistischen Tensid-Polyether-Mischung eingesetzt wurde, alleine nicht zu dem erfindungsgemäßen Effekt eines spontanen Spreitens eines aufgebrachten Wassertropfens auf der Oberfläche des Dentalabformmaterials führt.

### Vergleichsbeispiel A7: Additionsvernetzende Zweikomponenten-Silicon-Dentalabformmasse mit einem Tensid V

50 Teile der in Herstellungsbeispiel A2 beschriebenen Katalysatorkomponente und 50 Teile der in Herstellungs-Vergleichsbeispiel A7 beschriebenen Basiskomponente wurden aus einer Kartusche (Fa. Mixpac) ausgedrückt und über einen statischen Mischer (Fa. Mixpac) homogen gemischt.

Ergebnis 14A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 46 und 58°. Dieses Vergleichsbeispiel zeigt, dass der Einsatz eines Fettalkoholethoxylat-Tensids nach dem Stand der Technik nicht zu dem erfindungsgemäßen Effekt eines spontanen Spreitens eines aufgebrachten Wassertropfens auf der Oberfläche des Dentalabformmaterials führt.

### Vergleichsbeispiel A8: Additionsvernetzende Zweikomponenten-Silicon-Dentalabformmasse mit einem Tensid VI

50 Teile der in Herstellungsbeispiel A2 beschriebenen Katalysatorkomponente und 50 Teile der in Herstellungs-Vergleichsbeispiel A8 beschriebenen Basiskomponente wurden aus einer Kartusche (Fa. Mixpac) ausgedrückt und über einen statischen Mischer (Fa. Mixpac) homogen gemischt.

Ergebnis 15A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 68 und 78°. Dieses Vergleichsbeispiel zeigt, dass der Einsatz eines Nonylphenylethoxylat-Tensids nach dem Stand der Technik nicht zu dem erfindungsgemäßen Effekt eines spontanen Spreitens eines aufgebrachten Wassertropfens auf der Oberfläche des Dentalabformmaterials führt.

### Vergleichsbeispiel A9: Additionsvernetzende Zweikomponenten-Silicon-Dentalabformmasse mit einem Silicontensid nach dem Stand der Technik

50 Teile der Katalysatorpaste und 50 Teile der Basispaste eines additionsvernetzenden Silicon-Dentalabformmaterials gemäß WO-A-2004/58196 (Handelsprodukt Aquasil Ultra LV, Detrey Dentsply, Lot 030 923) wurden aus einer Kartusche (Fa. Mixpac) ausgedrückt und über einen statischen Mischer (Fa. Mixpac) homogen gemischt.

Ergebnis 16A: Man erhielt eine dünnfließende Dentalabformmasse (ISO 4823), die sich wie in Ergebnis 8A beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 48 und 69°. Dieses Vergleichsbeispiel zeigt, dass Dentalabformmaterialien nach dem Stand der Technik keinen synergistischen Kontaktwinkeleffekt aufweisen.

### Vergleichsbeispiel A10: Additionsvernetzende Zweikomponenten-Silicon-Dentalabformmasse nach dem Stand der Technik

Ein additionsvernetzendes Silicon-Dentalabformmaterial (Handelsprodukt Panasil Contact Plus, Kettenbach GmbH + Co. KG, Lot 05041) wurde gemäß Herstellerangaben aus einer Kartusche (Fa. Mixpac) ausgedrückt und über einen statischen Mischer (Fa. Mixpac) homogen gemischt.

### Ergebnis 17A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 46 und 58°. Dieses Vergleichsbeispiel zeigt, dass additionsvernetzende Silicon-Dentalabformmaterialen nach dem Stand der Technik nicht zu dem erfindungsgemäßen Effekt eines spontanen Spreitens eines aufgebrachten Wassertropfens auf der Oberfläche des Dentalabformmaterials führen.

### Vergleichsbeispiel A11: Additionsvernetzende Zweikomponenten-Silicon-Dentalabformmasse mit 1,0 % Silicontensid gemäß US 4657959 Tabelle I, run 2

Ein additionsvernetzendes Silicon-Dentalabformmaterial gemäß Beispiel Tabelle I, run no 2, der US-A-4,657,959 wurde aus einer Kartusche (Fa. Mixpac) ausgedrückt und über einen statischen Mischer (Fa. Mixpac) homogen gemischt.

### Ergebnis 18A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 33 und 42°. Dieses Vergleichsbeispiel zeigt, dass bei Verwendung der Messmethode der vorliegenden Erfindung die Dentalabformmaterialien nach US 4657959 keine Kontaktwinkel < 10° und kein Spreiten von Wasser auf der Oberfläche erreicht wird.

### Vergleichsbeispiel A12: Additionsvernetzende Zweikomponenten-Silicon-Dentalabformmasse mit 0,75% Silicontensid gemäß US-A-4,657,959 Tabelle IV, run no. 4

Ein additionsvernetzendes Silicon-Dentalabformmaterial gemäß Beispiel Tabelle IV, run no 4, der US-A-4,657,959 wurde aus einer Kartusche (Fa. Mixpac) ausgedrückt und über einen statischen Mischer (Fa. Mixpac) homogen gemischt.

### Ergebnis 19A: wie Ergebnis 18A

### Vergleichsbeispiel A13: Additionsvernetzende Zweikomponenten-Silicon-Dentalabformmasse mit 1,0 % Silicontensid gemäß US-A-4,657,959 Tabelle IV, run 5

Ein additionsvernetzendes Silicon-Dentalabformmaterial gemäß Beispiel Tabelle IV, run 5 der US 4657959 wurde aus einer Kartusche (Fa. Mixpac) ausgedrückt und über einen statischen Mischer (Fa. Mixpac) homogen gemischt.

### Ergebnis 20A: wie Ergebnis 18A

### Vergleichsbeispiel A14: Additionsvernetzende Zweikomponenten-Silicon-Dentalabformmasse mit 1,0 % Fluortensid gemäß US-A-4,657,959 Tabelle IV, run 11 1

Ein additionsvernetzendes Silicon-Dentalabformmaterial gemäß Beispiel Tabelle IV, run 11, der US-A-4,657,959 wurde aus einer Kartusche (Fa. Mixpac) ausgedrückt und über einen statischen Mischer (Fa. Mixpac) homogen gemischt.

### Ergebnis 21A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 79 und 63°. Dieses Vergleichsbeispiel zeigt, dass bei Verwendung der Messmethode der vorliegenden Erfindung die Dentalabformmaterialien nach US-A-4,657,959 keine Kontaktwinkel < 10° und kein Spreiten von Wasser auf der Oberfläche erreicht wird.

### Vergleichsbeispiel A15: Additionsvernetzende Zweikomponenten-Silicon-Dentalabformmasse mit einer nicht synergistisch wirkenden Tensidmischung aus dem besten Silicontensid (run 2) und Fluortensid (run 11) aus Beispiel 1 der US-A-4,657,959

50 Teile der in US-A-4,657,959 beschriebenen Katalysatorkomponente und 50 Teile der in Herstellungs-Vergleichsbeispiel A9 beschriebenen Basiskomponente wurden aus einer Kartusche (Fa. Mixpac) ausgedrückt und über einen statischen Mischer (Fa. Mixpac) homogen gemischt.

### Ergebnis 22A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 39 bis 50°. Dieses Vergleichsbeispiel zeigt, dass nicht jede beliebige Kombination von Fluortensiden und Silicontensiden zu einem synergistischen Effekt führt. Überraschenderweise wurde ein synergistischer Effekt nur in Tensidmischungen mit bestimmten Fluortensiden und Silicontensiden erzielt. Die US-A-4,657,959 beschreibt keine Mischungen von verschiedenen Tensiden. Werden, wie in diesem Vergleichsbeispiel, die jeweils besten Tenside aus der experimentellen Reihen der Silicontenside und der Fluortenside der US-A-4,657,959 kombiniert, ist kein Spreiten von Wassertropfen auf der Oberfläche der Siliconabformmaterials zu erreichen.

### Vergleichsbeispiel A16: Addititionsvernetzende Zweikomponenten-Silicon-Dentalabformmasse mit einer nicht synergistisch wirkenden Tensidmischung aus dem besten Silicontensid (run 2) und Fluortensid (run 11) aus Beispiel 1 der US-A-4,657,959

50 Teile der in US 4657959 beschriebenen Katalysatorkomponente und 50 Teile der in Herstellungs-Vergleichsbeispiel A10 beschriebenen Basiskomponente wurden aus einer Kartusche (Fa. Mixpac) ausgedrückt und über einen statischen Mischer (Fa. Mixpac) homogen gemischt.

### Ergebnis 23A: wie Ergebnis 22A

### Vergleichsbeispiel A17: Additionsvernetzende Zweikomponenten-Silicon-Dentalabformmasse mit einer Tensidmischung, die nicht synergistisch wirkt

50 Teile der in Herstellungsbeispiel A2 beschriebenen Katalysatorkomponente und 50 Teile der in Herstellungs-Vergleichsbeispiel A11 beschriebenen Basiskomponente wurden aus einer Kartusche (Fa. Mixpac) ausgedrückt und über einen statischen Mischer (Fa. Mixpac) homogen gemischt.

### Ergebnis 24A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 70 bis 86°. Dieses Vergleichsbeispiel zeigt, dass nicht jede beliebige Kombination von Fluortensiden mit Silicontensiden zu einem synergistischen Effekt führt. Überraschenderweise wird ein synergistischer Effekt nur in Tensidmischungen mit bestimmten Fluortensiden und Silicontensiden erzielt. Wird, wie in diesem Beispiel, ein Fluortensid, das in den Beispielen A1 bis A7 in Kombination mit geeigneten Silicontensiden synergistische Effekte aufweist, mit anderen ungeeigneten Silicontensiden kombiniert, ist kein Spreiten von Wassertropfen auf der Oberfläche der Siliconabformmaterials zu erreichen.

### Vergleichsbeispiel A18: Additionsvernetzende Zweikomponenten-Silicon-Dentalabformmasse mit einer Tensidmischung, die nicht synergistisch wirkt

50 Teile der in Herstellungsbeispiel A2 beschriebenen Katalysatorkomponente und 50 Teile der in Herstellungs-Vergleichsbeispiel A12 beschriebenen Basiskomponente wurden aus einer Kartusche (Fa. Mixpac) ausgedrückt und über einen statischen Mischer (Fa. Mixpac) homogen gemischt.

### Ergebnis 25A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 46 bis 62°. Dieses Vergleichsbeispiel zeigt, dass nicht jede beliebige Kombination von Fluortensiden mit Silicontensiden zu einem synergistischen Effekt führt. Überraschenderweise wird ein synergistischer Effekt nur in Tensidmischungen mit bestimmten Fluortensiden und Silicontensiden erzielt. Wird, wie in diesem Beispiel, ein Fluortensid, das in den Beispielen A1 bis A7 in Kombination mit geeigneten Silicontensiden synergistische Effekte aufweist, mit einem anderen ungeeigneten Silicontensid kombiniert, ist kein Spreiten von Wassertropfen auf der Oberfläche des Siliconabformmaterials zu erreichen.

### Vergleichsbeispiel A19: Additionsvernetzende Zweikomponenten-Silicon-Dentalabformmasse mit einer Tensidmischung, die nicht synergistisch wirkt

50 Teile der in Herstellungsbeispiel A2 beschriebenen Katalysatorkomponente und 50 Teile der in Herstellungs-Vergleichsbeispiel A13 beschriebenen Basiskomponente wurden aus einer Kartusche (Fa. Mixpac) ausgedrückt und über einen statischen Mischer (Fa. Mixpac) homogen gemischt.

### Ergebnis 26A: wie Ergebnis 8A

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 35 bis 50°. Dieses Vergleichsbeispiel zeigt, dass nicht jede beliebige Kombination von Fluortensiden mit Silicontensiden zu einem synergistischen Effekt führt. Überraschenderweise wird ein synergistischer Effekt nur in Tensidmischungen mit bestimmten Fluortensiden und Silicontensiden erzielt. Wird, wie in diesem Beispiel, ein nicht geeignetes Fluortensid, wie z.B. Fluorad FC430, in Kombination mit geeigneten Silicontensiden, wie z.B. Tensid III, kombiniert, ist kein Spreiten von Wassertropfen auf der Oberfläche des Siliconabformmaterials zu erreichen.

### Herstellungsbeispiel B1: Basismasse eines kondensationsvernetzenden Silicon-Dentalabformmaterials mit einer erfindungsgemäßen synergistisch wirkenden Tensid-Mischung

97 Teile der Basismasse des Handelsprodukts Lastic 90 fine, Kettenbach GmbH + Co. KG Lot 20941 wurden in einem Vakuummischer mit 0,5 Teilen eines Tensids II, 2,50 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel B2: Basismasse eines kondensationsvernetzenden Silicon-Dentalabformmaterials mit einer erfindungsgemäßen synergistisch wirkenden Tensid-Mischung

97 Teile der Basismasse des Handelsprodukts Lastic 90 fine, Kettenbach GmbH + Co. KG Lot 20941 wurden in einem Vakuummischer mit 1,5 Teilen eines Tensids II, 1,5 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Beispiel B1: Kondensationsvernetzendes Silicon-Dentalabformmaterial mit einer erfindungsgemäßen, synergistisch wirkenden Tensid-Mischung

7,7 Teile einer Katalysatorpaste eines kondensationsvernetzenden Silicon-Dentalabformmaterials (Handelsprodukt Lastic Xtra Pastenhärter, Kettenbach GmbH + Co. KG, Lot 50861) und 92,3 Teile der in Herstellungsbeispiel B1 beschriebenen Basiskomponente wurden auf einem Mischblock mit einem Mischspatel während 30 Sekunden homogen gemischt.

Ergebnis 1 B: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 2A und 2B). Es zeigte sich, dass in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt. Ein solches Material ist in hervorragender Weise geeignet im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen kondensationsvernetzenden Silicon-Dentalabformmaterial (Vergleichsbeispiel B3) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb einer bis 10 Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet im Patientenmund trotz Einwirkung von feuchter Zahnsubstanz, Speichel und Blut an die abzuformenden Bereiche anzufließen und diese präzise in der Abformung abzubilden.

### Beispiel B2: Kondensationsvernetzendes Silicon-Dentalabformmaterial mit einer erfindungsgemäßen, synergistisch wirkenden Tensid-Mischung

7,7 Teile einer Katalysatorpaste eines kondensationsvernetzenden Silicon-Dentalabformmaterials (Handelsprodukt Lastic Xtra Pastenhärter, Kettenbach GmbH + Co. KG, Lot 50861) und 92,3 Teile der in Herstellungsbeispiel B2 beschriebenen Basiskomponente wurden auf einem Mischblock mit einem Mischspatel während 30 Sekunden homogen gemischt.

### Ergebnis 2B: wie Ergebnis 1 B

### Herstellungs-Vergleichsbeispiel B1: Basismasse eines kondensationsvernetzendes Silicon-Dentalabformmaterials ausschließlich mit dem Silicontensid aus dem Beispiel B1, d.h. nicht in synergistischer Mischung mit Fluortensid

98,4 Teile der Basismasse des Handelsprodukts Lastic 90 fine, Kettenbach GmbH + Co. KG, Lot 20941 wurden in einem Vakuummischer mit 1,6 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungs-Vergleichsbeispiel B2: Basismasse eines kondensationsvernetzenden Silicon-Dentalabformmaterials ausschließlich mit dem Fluortensid aus dem Beispiel B1, d.h. nicht in synergistischer Mischung mit Silicontensid

98,4 Teile der Basismasse des Handelsprodukts Lastic 90 fine, Kettenbach GmbH + Co. KG Lot 20941 wurden in einem Vakuummischer mit 1,6 Teilen eines Tensids II für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Vergleichsbeispiel B1: Kondensationsvernetzendes Silicon-Dentalabformmaterial ausschließlich mit dem Silicontensid aus dem Beispiel B1, d.h. nicht in synergistischer Mischung mit Fluortensid

7,7 Teile einer Katalysatorpaste eines kondensationsvernetzenden Silicon-Dentalabformmaterials (Handelsprodukt Lastic Xtra Pastenhärter, Kettenbach GmbH + Co. KG, Lot 50861) und 92,3 Teile der in Herstellungs-Vergleichsbeispiel B1 beschriebenen Basiskomponente wurden auf einem Mischblock mit einem Mischspatel während 30 Sekunden homogen gemischt.

Ergebnis 3B: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 1A, 1B, 2A und 2B). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wird. Die aufgebrachten Wassertropfen spreiten nicht auf der Oberfläche der Abformmasse und benetzen diese nicht ausreichend. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 38 und 44°. Dieses Vergleichsbeispiel zeigt, dass nicht jede beliebige Kombination von Tensiden zu einem synergistischen Effekt führt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 33 und 38°. Dieses Vergleichsbeispiel zeigt, dass dasselbe Silicontensid, das in Beispiel B1 in der erfindungsgemäßen synergistischen Tensid-Mischung eingesetzt wurde, allein nicht zu dem erfindungsgemäßen Effekt eines spontanen Spreitens eines aufgebrachten Wassertropfens auf der Oberfläche des Dentalabformmaterials führt.

### Vergleichsbeispiel B2: Kondensationsvernetzendes Silicon-Dentalabformmaterial ausschließlich mit dem Fluortensid aus Beispiel B1, d.h. nicht in synergistischer Mischung mit Silicontensid

7,7 Teile einer Katalysatorpaste eines kondensationsvernetzenden Silicon-Dentalabformmaterials (Handelsprodukt Lastic Xtra Pastenhärter, Kettenbach GmbH + Co. KG, Lot 50861) und 92,3 Teile der in Herstellungs-Vergleichsbeispiel B2 beschriebenen Basiskomponente wurden auf einem Mischblock mit einem Mischspatel während 30 Sekunden homogen gemischt.

### Ergebnis 4B: wie Ergebnis 3B

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 70 und 85°. Dieses Vergleichsbeispiel zeigt, dass dasselbe Silicontensid, das in Beispiel B1 in er erfindungsgemäßen synergistischen Tensid-Mischung eingesetzt wurde, allein nicht zu dem erfindungsgemäßen Effekt eines spontanen Spreitens eines aufgebrachten Wassertropfens auf der Oberfläche des Dentalabformmaterials führt.

### Vergleichsbeispiel B3: Kondensationsvernetzendes Silicon-Dentalabformmaterial nach dem Stand der Technik (Handelsprodukt)

Ein kondensationsvernetzendes Silicon-Dentalmaterial (Handelsprodukt Lastic 90 fine, Kettenbach GmbH + Co. KG, Lot 20941) wird gemäß Herstellerangaben auf einem Mischblock mit einem Mischspatel homogen gemischt.

### Ergebnis 5B: wie Ergebnis 3B

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 69 und 98°. Dieses Vergleichsbeispiel zeigt, dass kondensationsvernetzende Silicon-Dentalabfrommaterialien nach dem Stand der Technik nicht zu dem erfindungsgemäßen Effekt eines spontanen Spreitens eines aufgebrachten Wassertropfens auf der Oberfläche des Dentalabformmaterials führen.

### Herstellungsbeispiel C1: Basismasse eines kondensationsvernetzenden Alkoxysilylpolyether-Dentalabformmaterials mit einer erfindungsgemäßen synergistisch wirkenden Tensid-Mischung

97,8 Teile einer Basismasse eines kondensationsvernetzenden Alkoxysilylpolyether-Dentalabformmaterials gemäß PCT/EP 2005/001470 wurde in einem Vakuummischer mit 0,2 Teilen eines Tensids II, 2,0 Teilen eines Tensids III für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Herstellungsbeispiel C2: Basismasse eines kondensationsvernetzenden Alkoxysilylpolyether-Dentalabformmaterials mit einer erfindungsgemäß synergistisch wirkenden Tensid-Mischung

94 Teile einer Basismasse eines kondensationsvernetzenden Alkoxysilylpolyether-Dentalabformmaterials gemäß EP-A-1,226,808 (Handeslprodukt P2-Polyether mono, Heraeus-Kulzer, Lot 190 188) wurden mit 5,94 Teilen eines Tensids II, 0,06 Teilen eines Tensids III für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel C3: Basismasse eines kondensationsvernetzenden Alkoxysilylpolyether-Dentalabformmaterials mit einer erfindungsgemäß synergistisch wirkenden Tensid-Mischung

94 Teile einer Basismasse eines kondensationsvernetzenden Alkoxysilylpolyether-Dentalabformmaterials gemäß EP-A-1,226,808 (Handelsprodukt P2-Polyether mono, Heraeus-Kulzer, Lot 190 188) wurden mit 3,0 Teilen eines Tensids II, 3,0 Teilen eines Tensids III für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel CC3: Basismasse eines kondensationsvernetzenden Alkoxysilylpolyether-Dentalabformmaterials mit einer erfindungsgemäß synergistisch wirkenden Tensid-Polyol-Mischung

94 Teile einer Basismasse eines kondensationsvernetzenden Alkoxysilylpolyether-Dentalabformmaterials gemäß EP-A-1,226,808 (Handelsprodukt P2-Polyether mono, Heraeus-Kulzer, Lot 190 188) wurden mit 6,0 Teilen eines Tensids II und 1,8 Teilen Glycerin für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel CCC3: Basismasse eines kondensationsvernetzenden Alkoxysilylpolyether-Dentalabformmaterials mit einer erfindungsgemäß synergistisch wirkenden Tensid-Polyol-Mischung

92,2 Teile einer Basismasse eines kondensationsvernetzenden Alkoxysilylpolyether-Dentalabformmaterials gemäß EP-A-1,226,808 (Handelsprodukt P2-Polyether mono, Heraeus-Kulzer, Lot 190 188) wurden mit 3,0 Teilen eines Tensids II, 3,0 Teilen eines Tensids III und 1,8 Teilen Glycerin für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Beispiel C1: Kondensationsvernetzendes Alkoxysilylpolyether Dentalabformmaterial mit einer erfindungsgemäßen synergistisch wirkenden Tensid-Mischung

33,3 Teile einer Katalysatormasse eines kondensationsvernetzenden Alkoxysilylpolyether-Dentalabformmaterials gemäß PCT/EP 2005/001470 und 66,6 Teile der in Herstellungsbeispiel C1 beschriebenen Basiskomponente wurden aus einer Kartusche (Fa. Mixpac) ausgedrückt und über einen statischen Mischer (Fa. Mixpac) homogen gemischt.

Ergebnis 1C: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 3A und 3B). Es zeigte sich, dass in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt. Ein solches Material ist in hervorragender Weise geeignet im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen kondensationsvernetzenden Alkoxysilylpolyether-Dentalabformmaterial (Vergleichsbeispiele C3 und C4) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet im Patientenmund trotz Einwirkung von feuchter Zahnsubstanz, Speichel und Blut an die abzuformenden Bereiche anzufließen und diese präzise in der Abformung abzubilden.

### Beispiel C2: Kondensationsvernetzendes Alkoxysilylpolyether-Dentalabformmaterial mit einer erfindungsgemäß synergistisch wirkenden Tensid-Mischung

16,7 Teile einer Katalysatorpaste eines kondensationsvernetzenden Alkoxysilyl-Polyether Dentalabformmaterials gemäß EP-A-1,226,808 (Handelsprodukt P2-Polyether mono, Heraeus-Kulzer, Lot 190 188) und 83,3 Teile der in Herstellungsbeispiel C2 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (Heraeus-Kulzer) homogen gemischt.

Ergebnis 2C: Man erhielt eine mittelfließende Dentalabformmasse (ISO 4823), die sich wie in Ergebnis 1C beschrieben verhielt.

### Beispiel C3: Kondensationsvernetzendes Alkoxysilylpolyether-Dentalabformmaterial mit einer erfindungsgemäß synergistisch wirkenden Tensid-Mischung

16,7 Teile einer Katalysatorpaste eines kondensationsvernetzenden Alkoxysilyl-Polyether Dentalabformmaterials gemäß EP-A-1,226,808 (Handelsprodukt P2-Polyether mono, Heraeus-Kulzer, Lot 190 188) und 83,3 Teile der in Herstellungsbeispiel C3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (Heraeus-Kulzer) homogen gemischt.

Ergebnis 3C: Man erhielt eine mittelfließende Dentalabformmasse (ISO 4823), die sich wie in Ergebnis 1C beschrieben verhielt.

### Beispiel CC3: Kondensationsvernetzendes Alkoxysilylpolyether-Dentalabformmaterial mit einer erfindungsgemäß synergistisch wirkenden Tensid-Polyol-Mischung

16,7 Teile einer Katalysatorpaste eines kondensationsvernetzenden Alkoxysilyl-Polyether Dentalabformmaterials gemäß EP-A-1,226,808 (Handelsprodukt P2-Polyether mono, Heraeus-Kulzer, Lot 190 188) und 83,3 Teile der in Herstellungsbeispiel CC3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (Heraeus-Kulzer) homogen gemischt.

Ergebnis 3CC: Man erhielt eine mittelfließende Dentalabformmasse (ISO 4823), die sich wie in Ergebnis 1C beschrieben verhielt.

### Beispiel CCC3: Kondensationsvernetzendes Alkoxysilylpolyether-Dentalabformmaterial mit einer erfindungsgemäß synergistisch wirkenden Tensid-Polyol-Mischung

16,7 Teile einer Katalysatorpaste eines kondensationsvernetzenden Alkoxysilyl-Polyether Dentalabformmaterials gemäß EP-A-1,226,808 (Handelsprodukt P2-Polyether mono, Heraeus-Kulzer, Lot 190 188) und 83,3 Teile der in Herstellungsbeispiel CCC3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (Heraeus-Kulzer) homogen gemischt.

Ergebnis 3CCC: Man erhielt eine mittelfließende Dentalabformmasse (ISO 4823), die sich wie in Ergebnis 1C beschrieben verhielt.

### Herstellungs-Vergleichsbeispiel C1: Basismasse eines kondensationsvernetzenden Alkoxysilyl-Polyether-Dentalabformmaterials ausschließlich mit dem Silicontensid aus Beispiel C1, d.h. nicht in synergistischer Mischung mit Fluortensid

97,8 Teile einer Basismasse eines kondensationsvernetzenden Alkoxysilylpolyether-Dentalabformmaterials gemäß PCT/EP 2005/001470 wurden in einem Vakuummischer mit 2,2 Teilen eines Tensids III für 15 Minuten homogen gemischt und entgast. Es wurde eine dünnfließende Paste (ISO 4823) erhalten. Die Paste stellt eine mögliche Basiskomponente einer nicht erfindungsgemäßen Zweikomponenten-Dentalabformmasse dar.

### Herstellungs-Vergleichsbeispiel C2: Basismasse eines kondensationsvernetzenden Alkoxysilylpolyether-Dentalabformmaterials ausschließlich mit dem Fluortensid aus dem Beispiel C2, d.h. nicht in Abmischung mit Silicontensid

97,8 Teile einer Basismasse eines kondensationsvernetzenden Alkoxysilylpolyether-Dentalabformmaterials gemäß EP-A-1,226,808 (Handelsprodukt P2-Polyether mono, Heraeus-Kulzer, Lot 190 188) wurden mit 2,2 Teilen eines Tensids II für 15 Minuten homogen gemischt und entgast. Es wurde eine dünnfließende Paste (ISO 4823) erhalten. Die Paste stellt eine nicht erfindungsgemäße Basiskomponente für eine Zweikomponenten-Dentalabformmasse dar.

### Vergleichsbeispiel C1: Kondensationsvernetzendes Alkoxysilyl-Polyether-Dentalabformmaterial ausschließlich mit dem Silkontensid aus Beispiel C1, d.h. nicht in synergistischer Mischung mit Fluortensid

33,3 Teile einer Katalysatormasse eines kondensationsvernetzenden Alkoxysilylpolyether-Dentalabformmaterials gemäß PCT/EP 2005/001470 und 66,6 Teile der in Herstellungs-Vergleichsbeispiel C1 beschriebenen Basiskomponente wurden aus einer Kartusche (Fa. Mixpac) ausgedrückt und über einen statischen Mischer (Fa. Mixpac) homogen gemischt.

Ergebnis 4C: Man erhielt eine dünnfließende Dentalabformmasse (ISO 4823), deren Kontaktwinkel zu verschiedenen Zeitpunkten innerhalb der Verarbeitungszeit untersucht wurde (siehe Tabellen 3A und 3B). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wurde. Die aufgebrachten Wassertropfen spreiten nicht auf der Oberfläche der Abformmasse und benetzen diese nicht ausreichend. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 33 und 69°. Dieses Vergleichsbeispiel zeigt, dass dasselbe Silicontensid, das in Beispiel C1 in der erfindungsgemäßen synergistischen Tensid-Mischung eingesetzt wurde, allein nicht zu dem erfindungsgemäßen Effekt eines spontanen Spreitens eines aufgebrachten Wassertropfens auf der Oberfläche des Dentalabformmaterials führt.

### Vergleichsbeispiel C2: Basiskomponente einer kondensationsvernetzenden Zweikomponenten-Alkoxysilyl-Polyether-Dentalabformmasse ausschließlich mit dem Fluortensid aus dem Bespiel 9, d.h. nicht in Abmischung mit Silicontensid

16,7 Teile einer Katalysatorpaste eines kondensationsvernetzenden Alkoxysilyl-Polyether-Dentalabformmaterials gemäß EP-A-1,226,808 (Handelsprodukt P2-Polyether mono, Heraeus-Kulzer, Lot 190 188) und 83,3 Teile der in Herstellungs-Vergleichsbeispiel C2 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 5C: Man erhielt eine mittelfließende Dentalabformmasse, die sich wie in Ergebnis 4C beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 38 und 72°. Dieses Vergleichsbeispiel zeigt, dass dasselbe Silicontensid, das in Beispiel C2 in er erfindungsgemäßen synergistischen Tensid-Mischung eingesetzt wurde, allein nicht zu dem erfindungsgemäßen Effekt eines spontanen Spreitens eines aufgebrachten Wassertropfens auf der Oberfläche des Dentalabformmaterials führt.

### Vergleichsbeispiel C3: Kondensationsvernetzendes Alkoxysilyl-Polyether-Dentalabformmaterial nach dem Stand der Technik

Ein kondensationsvernetzendes Alkoxysilyl-Polyether-Dentalabformmaterial gemäß PCT/EP 2005/001470 wird aus einer Kartusche (Fa. Mixpac) ausgetragen und über einen statischen Mischer (Fa. Mixpac) homogen gemischt.

### Ergebnis 6C: wie Ergebnis 4C

Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 34 und 55°. Dieses Vergleichsbeispiel zeigt, dass kondensationsvernetzende Alkoxysilyl-Polyether-Dentalabfrommaterialien nach dem Stand der Technik nicht zu dem erfindungsgemäßen Effekt eines spontanen Spreitens eines aufgebrachten Wassertropfens auf der Oberfläche des Dentalabformmaterials führen.

### Vergleichsbeispiel C4: Kondensationsvernetzendes Alkoxysilyl-Polyether-Dentalabformmaterial nach dem Stand der Technik (Handelsprodukt)

Ein kondensationsvernetzendes Alkoxysilyl-Polyether-Dentalabformmaterial gemäß EP-A-1,226,808 (Handelsprodukt P2 Polyether mono, Heraeus Kulzer, Lot 190180) wird gemäß Herstellerangaben mit Hilfe eines elektrischen Austragsgeräts (Plug + Press, Kettenbach GmbH + Co. KG) aus Schlauchbeuteln ausgetragen und über einen dynamischen Mischer (Heraeus-Kulzer) homogen gemischt.

Ergebnis 7C: Man erhielt eine mittelfließende Dentalabformmasse, die sich wie in Ergebnis 6C beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 47 und 53°.

### Herstellungsbeispiel D1: Basismasse eines additionsvernetzenden Aziridino-Polyether-Dentalabformmaterials mit einer erfindungsgemäßen synergistisch wirkenden Tensid-Mischung

98,2 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 1,64 Teilen eines Tensids II, 0,16 Teilen eines Tensids III für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel DD1: Basismasse eines additionsvernetzenden Aziridino-Polyether-Dentalabformmaterials mit einer erfindungsgemäßen synergistisch wirkenden Tensid-Polyol-Mischung

98,2 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 1,64 Teilen eines Tensids II, 0,16 Teilen eines Tensids III und 1,80 Teilen Glycerin für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel D2: Basismasse eines additionsvernetzenden Aziridino-Polyether-Dentalabformmaterials mit einer erfindungsgemäßen synergistisch wirkenden Tensid-Mischung

98,2 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 0,9 Teilen eines Tensids II, 0,9 Teilen eines Tensids III für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel D3: Basismasse eines additionsvernetzenden Aziridino-Polyether-Dentalabformmaterials mit Tensid II

98,2 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 1,80 Teilen eines Tensids II, für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Herstellungsbeispiel DD3: Basismasse eines additionsvernetzenden Aziridino-Polyether-Dentalabformmaterials mit Tensid II und Glycerin

96,4 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 1,80 Teilen eines Tensids II und 1,80 Teilen Glycerin für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Beispiel D1: Additionsvernetzendes Aziridino-Polyether-Dentalabformmaterial mit einer erfindungsgemäßen, synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungsbeispiel D1 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 1D: Man erhielt eine mittelfließende Dentalabformmasse (ISO 4823), deren Kontaktwinkel zu verschiedenen Zeitpunkten innerhalb der Verarbeitungszeit untersucht wurde (siehe Tabellen 4A und 4B). Es zeigte sich, dass in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt. Ein solches Material ist in hervorragender Weise geeignet im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen additionsvernetzenden Aziridinopolyether-Dentalabformmaterialien (Vergleichsbeispiel D2) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb einer bis zehn Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet im Patientenmund trotz Einwirkung von feuchter Zahnsubstanz, Speichel und Blut an die abzuformenden Bereiche anzufließen und diese präzise in der Abformung abzubilden.

### Beispiel DD1: Additionsvernetzendes Aziridino-Polyether-Dentalabformmaterial mit einer erfindungsgemäßen, synergistisch wirkenden Tensid-Polyol-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungsbeispiel DD1 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

### Ergebnis 1 DD: Wie Ergebnis 1 D

### Beispiel D2: Additionsvernetzendes Aziridino-Polyether-Dentalabformmaterial mit einer erfindungsgemäßen, synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungsbeispiel D2 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

### Ergebnis 2D: wie Ergebnis 1 D

### Beispiel D3: Additionsvernetzendes Aziridino-Polyether-Dentalabformmaterial mit erfindungsgemäß eingesetztem Tensid II

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungsbeispiel D3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

### Ergebnis 3D: wie Ergebnis 1D

### Beispiel DD3: Additionsvernetzendes Aziridino-Polyether-Dentalabformmaterial mit erfindungsgemäß eingesetztem Tensid II und Glycerin

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungsbeispiel DD3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

### Ergebnis 3DD: wie Ergebnis 1 D

### Herstellungs-Vergleichsbeispiel D1: Basismasse eines additionsvernetzenden Aziridino-Polyether-Dentalabformmaterial ausschließlich mit dem Silkontensid aus Beispiel D1, d.h. nicht in synergistischer Mischung mit Fluortensid

98,2 Teile einer Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 1,8 Teilen eines Tensids III für 15 Minuten homogen gemischt und entgast. Es wurde eine mittelfließende Paste (ISO 4823) erhalten. Die Paste stellt eine mögliche Basiskomponente für eine Zweikomponenten-Dentalabformmasse dar.

### Herstellungs-Vergleichsbeispiel D2: Basismasse eines additionsvernetzenden Aziridino-Polyether-Dentalabformmaterials mit einer nicht synergistisch wirkenden Tensid-Mischung

97 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 0,75 Teilen des Tensids VII und 2,25 Teilen eines Tensid IX für 15 Minuten homogen gemischt und entgast. Es wurde eine mittelfließende Paste (ISO 4823) erhalten. Die Paste stellt eine mögliche Basiskomponente für eine Zweikomponenten-Dentalabformmasse dar.

### Herstellungs-Vergleichsbeispiel D3: Basismasse eines additionsvernetzenden Aziridino-Polyether-Dentalabformmaterials mit einer nicht synergistisch wirkenden Tensid-Mischung

97 Teile der Basismasse eines additionsvernetzenden Aziridino-Polyether-Dentalabformmaterials gemäß US- A-4,353,242 (Handelsprodukt Impregum Penta, 3M-Espe, Lot 206352) wurden in einem Vakuummischer mit 0,75 Teilen des Tensids VII und 2,25 Teilen eines Tensids IV für 15 Minuten homogen gemischt und entgast. Es wurde eine mittelfließende Paste (ISO 4823) erhalten. Die Paste stellt eine mögliche Basiskomponente für eine Zweikomponenten-Dentalabformmasse dar.

### Herstellungs-Vergleichsbeispiel D4: Basismasse eines additionsvernetzenden Aziridino-Polyether-Dentalabformmaterials mit einer nicht synergistisch wirkenden Tensid-Mischung

98,2 Teile der Basismasse eines additionsvernetzenden Aziridino-Polyether-Dentalabformmaterials gemäß US-A-4,353,242 (Handelsprodukt Impregum Penta, 3M-Espe, Lot 206352) wurden in einem Vakuummischer mit 0,45 Teilen des Tensids X und 1,35 Teilen eines Tensids III für 15 Minuten homogen gemischt und entgast. Es wurde eine mittelfließende Paste (ISO 4823) erhalten. Die Paste stellt eine mögliche Basiskomponente für eine Zweikomponenten-Dentalabformmasse dar.

### Vergleichsbeispiel D1: Additionsvernetzendes Aziridino-Polyether-Dentalabformmaterial ausschließlich mit dem Silicontensid aus Beispiel D1, d.h. nicht in synergistischer Mischung mit Fluortensid

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungs-Vergleichsbeispiel D1 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 4D: Man erhielt eine mittelfließende Dentalabformmasse (ISO 4823), deren Kontaktwinkel zu verschiedenen Zeitpunkten innerhalb der Verarbeitungszeit untersucht wurde (siehe Tabellen 4A und 4B). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wurde Die aufgebrachten Wassertropfen spreiten nicht auf der Oberfläche der Abformmasse und benetzen diese nicht ausreichend. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 19 und 29°. Dieses Vergleichsbeispiel zeigt, dass dasselbe Silicontensid, das in Beispiel D1 in er erfindungsgemäßen synergistischen Tensid-Mischung eingesetzt wurde, allein nicht zu dem erfindungsgemäßen Effekt eines spontanen Spreitens eines aufgebrachten Wassertropfens auf der Oberfläche des Dentalabformmaterials führt.

### Vergleichsbeispiel D2: Additionsvernetzendes Aziridino-Polyether-Dentalabformmaterial nach dem Stand der Technik (Handelsprodukt)

Ein additionsvernetzendes Aziridino-Polyether-Dentalabformmaterial gemäß US-A-4,353,242 (Handelsprodukt Impregum penta, 3M-Espe, Lot 206352) wird gemäß Herstellerangaben mit Hilfe eines elektrischen Austragsgeräts (Plug + Press-Dispenser, Kettenbach GmbH + Co. KG) homogen gemischt.

Ergebnis 5D: Man erhielt eine mittelfließende Dentalabformmasse, die sich wie in Ergebnis 4D beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 35 und 39°. Dieses Vergleichsbeispiel zeigt, dass additionsvernetzende Aziridino-Polyether-Dentalabformmaterialien nach dem Stand der Technik nicht zu dem erfindungsgemäßen Effekt eines spontanen Spreitens eines aufgebrachten Wassertropfens auf der Oberfläche des Dentalabformmaterials führen.

### Vergleichsbeispiel D3: Additionsvernetzendes Aziridino-Polyether-Dentalabformmaterial mit einer nicht synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungs-Vergleichsbeispiel D2 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 6D: Man erhielt eine mittelfließende Dentalabformmasse, die sich wie in Ergebnis 4D beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 49 bis 56°. Dieses Vergleichsbeispiel zeigt, dass nicht jede beliebige Kombination von Fluortensiden mit Silicontensiden zu einem synergistischen Effekt führt. Überraschenderweise wird ein synergistischer Effekt nur in Tensidmischungen mit bestimmten Fluortensiden und Silicontensiden erzielt. Mit dem in diesem Beispiel eingesetzten Fluortensid und Silicontensid ist kein Spreiten von Wassertropfen auf der Oberfläche des Aziridino-Polyether-Abformmaterials zu erreichen.

### Vergleichsbeispiel D4: Additionsvernetzendes Aziridino-Polyether-Dentalabformmaterial mit einer nicht synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungs-Vergleichsbeispiel D3 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 7D: Man erhielt eine mittelfließende Dentalabformmasse, die sich wie in Ergebnis 4D beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 40 bis 54°. Dieses Vergleichsbeispiel zeigt, dass nicht jede beliebige Kombination von Fluortensiden mit Silicontensiden zu einem synergistischen Effekt führt. Überraschenderweise wird ein synergistischer Effekt nur in Tensidmischungen mit bestimmten Fluortensiden und Silicontensiden erzielt. Mit dem in diesem Beispiel eingesetzten Fluortensid und Silicontensid ist kein Spreiten von Wassertropfen auf der Oberfläche des Aziridino-Polyether-Abformmaterials zu erreichen.

### Vergleichsbeispiel D5: Additionsvernetzendes Aziridino-Polyether-Dentalabformmaterial mit einer nicht synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 206352) und 83,3 Teile der in Herstellungs-Vergleichsbeispiel D4 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis 8D: Man erhielt eine mittelfließende Dentalabformmasse, die sich wie in Ergebnis 4D beschrieben verhielt. Die Gleichgewichtskontaktwinkel zwischen 40 und 120 s Materialalter (nach Mischbeginn der Katalysator- und der Basiskomponente) lagen 10 Sekunden nach Aufbringen des Wassertropfens zwischen 45 bis 66°. Dieses Vergleichsbeispiel zeigt, dass nicht jede beliebige Kombination von Fluortensiden, z.B. Fluorad FC430, mit Silicontensiden, z.B. Tensid III, zu einem synergistischen Effekt führt. Überraschenderweise wird ein synergistischer Effekt nur in Tensidmischungen mit bestimmten Fluortensiden und Silicontensiden erzielt. Mit dem in diesem Beispiel eingesetzten Fluortensid und Silicontensid ist kein Spreiten von Wassertropfen auf der Oberfläche des Aziridino-Polyether-Abformmaterials zu erreichen. Dieses Beispiel zeigt auch, dass aus der Lehre der DE-A-43 06 997 keine Rückschlüsse auf den erfindungsgemäßen synergistischen Effekt gezogen werden können.

Die Ergebnisse der Prüfungen sind in den folgenden Tabellen dargestellt.

**Tabelle 1A: Zeitabhängige Kontaktwinkelmessungen an additionsvernetzenden Siliconen mit erfindungsgemäßen synergistisch wirkenden Tensid- (Polyether-) kombinationen im Vergleich zum Stand der Technik⁴⁾**

| **Beispiele/Vergleichsbeispiele** | **Materialalter nach Mischbeginn 40 s ¹⁾ Zeitspanne bis ein Kontaktwinkel von 10° erreicht wird ²⁾ [s]** | **Materialalter nach Mischbeginn 60 s ¹⁾ Zeitspanne bis ein Kontaktwinkel von 10° erreicht wird ²⁾ [s]** | **Materialalter nach Mischbeginn 90 s ¹⁾ Zeitspanne bis ein Kontaktwinkel von 10° erreicht wird ²⁾ [s]** | **Materialalter nach Mischbeginn 120 s ¹⁾ Zeitspanne bis ein Kontaktwinkel von 10° erreicht wird ²⁾ [s]** |
|---|---|---|---|---|
| Beispiel A1 erfindungsgemäß | 3 | 3 | 3 | 3 |
| Beispiel A2 erfindungsgemäß | 3 | 3 | 3 | 3 |
| Beispiel A3 erfindungsgemäß | 4 | 4 | 4 | 4 |
| Beispiel A4 erfindungsgemäß | 9 | 6 | 6 | 6 |
| Beispiel AA4 erfindungsgemäß | 2 | 2 | 1 | 1 |
| Beispiel AAA4 erfindungsgemäß | 2 | 2 | 1 | 1 |
| Beispiel A5 erfindungsgemäß | 9 | 6 | 4 | 3 |
| Beispiel A6 erfindungsgemäß | 10 | 9 | 6 | 6 |
| Beispiel A7 erfindungsgemäß | 1 | 1 | 1 | 1 |
| Vergleichsbeispiel A1 | - ³⁾ | - ³⁾ | - ³⁾ | - ³⁾ |
| Vergleichsbeispiel A2 | - ³⁾ | - ³⁾ | - ³⁾ | - ³⁾ |
| Vergleichsbeispiel A3 | - ³⁾ | - ³⁾ | - ³⁾ | - ³⁾ |
| Vergleichsbeispiel A4 | - ³⁾ | - ³⁾ | - ³⁾ | - ³⁾ |
| Vergleichsbeispiel A5 | - ³⁾ | - ³⁾ | - ³⁾ | - ³⁾ |
| Vergleichsbeispiel A6 | - ³⁾ | - ³⁾ | - ³⁾ | - ³⁾ |
| Vergleichsbeispiel A7 | - ³⁾ | - ³⁾ | - ³⁾ | - ³⁾ |
| Vergleichsbeispiel A8 | - ³⁾ | - ³⁾ | - ³⁾ | - ³⁾ |
| Vergleichsbeispiel A9 | - ³⁾ | - ³⁾ | - ³⁾ | - ³⁾ |
| Vergleichsbeispiel A10 | - ³⁾ | - ³⁾ | - ³⁾ | - ³⁾ |
| Vergleichsbeispiel A11 | - ³⁾ | - ³⁾ | - ³⁾ | - ³⁾ |
| Vergleichsbeispiel A12 | - ³⁾ | - ³⁾ | - ³⁾ | - ³⁾ |
| Vergleichsbeispiel A13 | - ³⁾ | - ³⁾ | - ³⁾ | - ³⁾ |
| Vergleichsbeispiel A14 | - ³⁾ | - ³⁾ | - ³⁾ | - ³⁾ |
| Vergleichsbeispiel A15 | - ³⁾ | - ³⁾ | - ³⁾ | - ³⁾ |
| Vergleichsbeispiel A16 | - ³⁾ | - ³⁾ | - ³⁾ | - ³⁾ |
| Vergleichsbeispiel A17 | - ³⁾ | - ³⁾ | - ³⁾ | - ³⁾ |
| Vergleichsbeispiel A18 | - ³⁾ | - ³⁾ | - ³⁾ | - ³⁾ |
| Vergleichsbeispiel A19 | - ³⁾ | - ³⁾ | - ³⁾ | - ³⁾ |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

**Tabelle 1 B: Zeitabhängige Kontaktwinkelmessungen an additionsvernetzenden Siliconen mit erfindungsgemäßen synergistisch wirkenden Tensid- (Polyether-) kombinationen im Vergleich zum Stand der Technik ⁴⁾**

| **Beispiele/Vergleichsbeispiele** | **Materialalter nach Mischbeginn 40 s ¹⁾ Tropfenalter 10 s Kontaktwinkel ²⁾ [°]** | **Materialalter nach Mischbeginn 60 s ¹⁾ Tropfenalter 10 s Kontaktwinkel ²⁾ [°]** | **Materialalter nach Mischbeginn 90 s ¹⁾ Tropfenalter 10 s Kontaktwinkel ²⁾ [°]** | **Materialalter nach Mischbeginn 120 s ¹⁾ Tropfenalter 10 s Kontaktwinkel ²⁾ [°]** |
|---|---|---|---|---|
| Beispiel A1 erfindungsgemäß | < 10 | < 10 | < 10 | < 10 |
| Beispiel A2 erfindungsgemäß | <10 | <10 | < 10 | < 10 |
| Beispiel A3 erfindungsgemäß | <10 | <10 | <10 | <10 |
| Beispiel A4 erfindungsgemäß | <10 | <10 | < 10 | < 10 |
| Beispiel AA4 erfindungsgemäß | <10 | <10 | < 10 | <10 |
| Beispiel AAA4 erfindungsgemäß | < 10 | <10 | <10 | < 10 |
| Beispiel A5 erfindungsgemäß | < 10 | < 10 | < 10 | < 10 |
| Beispiel A6 erfindungsgemäß | < 10 | < 10 | <10 | <10 |
| Beispiel A7 erfindungsgemäß | < 10 | < 10 | <10 | < 10 |
| Vergleichsbeispiel A1 | 38 | 38 | 40 | 44 |
| Vergleichsbeispiel A2 | 65 | 68 | 74 | 75 |
| Vergleichsbeispiel A3 | 60 | 63 | 68 | 72 |
| Vergleichsbeispiel A4 | 44 | 50 | 58 | 66 |
| Vergleichsbeispiel A5 | 43 | 42 | 44 | 45 |
| Vergleichsbeispiel A6 | 36 | 33 | 42 | 39 |
| Vergleichsbeispiel A7 | 47 | 52 | 58 | 46 |
| Vergleichsbeispiel A8 | 68 | 70 | 75 | 78 |
| Vergleichsbeispiel A9 | 48 | 54 | 62 | 69 |
| Vergleichsbeispiel A10 | 46 | 50 | 55 | 58 |
| Vergleichsbeispiel A11 | 33 | 37 | 40 | 42 |
| Vergleichsbeispiel A12 | 33 | 37 | 40 | 42 |
| Vergleichsbeispiel A13 | 33 | 37 | 40 | 42 |
| Vergleichsbeispiel A14 | 79 | 77 | 75 | 63 |
| Vergleichsbeispiel A15 | 39 | 42 | 45 | 50 |
| Vergleichsbeispiel A16 | 39 | 42 | 45 | 50 |
| Vergleichsbeispiel A17 | 70 | 72 | 76 | 86 |
| Vergleichsbeispiel A18 | 46 | 54 | 58 | 62 |
| Vergleichsbeispiel A19 | 49 | 45 | 38 | 49 |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

**Tabelle 2A: Zeitabhängige Kontaktwinkelmessungen an kondensationsvernetzenden Siliconen mit einer erfindungsgemäßen synergistisch wirkenden Tensidkombination im Vergleich zum Stand der Technik⁴⁾**

| **Beispiele/Vergleichsbeispiele** | **Materialalter nach Mischbeginn 40 s¹⁾ Zeitspanne bis ein Kontaktwinkel von 10° erreicht wird ²⁾ [s]** | **Materialalter nach Mlechbeginn 60 s ¹⁾ Zeitspanne bis ein Kontaktwinkel von 10° erreicht wird ²⁾ [s]** | **Materialalter nach Mischbeginn 90 s ¹⁾ Zeitspanne bis ein Kontaktwinkel von 10° erreicht wird ²⁾ [s]** | **Materialalter nach Mischbeginn 120 s ¹⁾ Zeitspanne bis ein Kontaktwinkel von 10° erreicht wird ²⁾ [s]** |
|---|---|---|---|---|
| Beispiel B1 erfindungsgemäß | < 1 | < 1 | < 1 | < 1 |
| Beispiel B2 erfindungsgemäß | < 1 | < 1 | < 1 | < 1 |
| Vergleichsbeispiel B1 | - ³⁾ | - ³⁾ | - ³⁾ | - ³⁾ |
| Vergleichsbeispiel B2 | - ³⁾ | - ³⁾ | - ³⁾ | - ³⁾ |
| Vergleichsbeispiel B3 | - ³⁾ | - ³⁾ | - ³⁾ | - ³⁾ |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

**Tabelle 2B: Zeitabhängige Kontaktwinkelmessungen an kondensationsvernetzenden Siliconen mit einer erfindungsgemäßen synergistisch wirkenden Tensidkombination im Vergleich zum Stand der Technik⁴⁾**

| **Beispiele/Vergleichsbeispiele** | **Materialalter nach Mischbeginn 40 s ¹⁾ Tropfenalter 10 s Kontaktwinkel ²⁾ [°]** | **Materialalter nach Mischbeginn 60 s ¹⁾ Tropfenalter 10 s Kontaktwinkel ²⁾ [°]** | **Materialalter nach Mischbeginn 90 s ¹⁾ Tropfenalter 10 s Kontaktwinkel ²⁾ [°]** | **Materialalter nach Mischbeginn 120 s¹⁾ Tropfenalter 10 s Kontaktwinkel ²⁾ n** |
|---|---|---|---|---|
| Beispiel B1 erfindungsgemäß | <10 | <10 | <10 | < 10 |
| Beispiel B2 erfindungsgemäß | <10 | < 10 | < 10 | < 10 |
| Vergleichsbeispiel B1 | 33 | 35 | 40 | 38 |
| Vergleichsbeispiel B2 | 70 | 72 | 85 | 85 |
| Vergleichsbeispiel B3 | 69 | 89 | 90 | 98 |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

**Tabelle 3A: Zeitabhängige Kontaktwinkelmessungen an kondensationsvernetzenden Alkoxysilylpolyethern mit einer erfindungsgemäßen synergistisch wirkenden Tensidkombination im Vergleich zum Stand der Technik⁴⁾**

| **Beispiele/Vergleichsbeispiele** | **Materialalter nach Mischbeginn 40 s ¹⁾ Zeitspanne bis ein Kontaktwinkel von 10° erreicht wird ²⁾ [s]** | **Materialalter nach Mischbeginn 60 s ¹⁾ Zeitspanne bis ein Kontaktwinkel von 10° erreicht wird ²⁾ [s]** | **Materialalter nach Mischbeginn 90 s ¹⁾ Zeitspanne bis ein Kontaktwinkel von 10° erreicht wird ²⁾ [s]** | **Materialalter nach Mischbeginn 120 s ¹⁾ Zeitspanne bis ein Kontaktwinkel von 10° erreicht wird ²⁾ [s]** |
|---|---|---|---|---|
| Beispiel C1 erfindungsgemäß | <1 | <1 | <1 | <1 |
| Beispiel C2 erfindungsgemäß | 5 | 3 | 3 | 5 |
| Beispiel C3 erfindungsgemäß | <1 | <1 | <1 | <1 |
| Beispiel CC3 erfindungsgemäß | 3 | 3 | 3 | 3 |
| Beispiel CCC3 erfindungsgemäß | 1 | 1 | 1 | 1 |
| Vergleichsbeispiel C1 | - ³⁾ | - ³⁾ | - ³⁾ | - ³⁾ |
| Vergleichsbeispiel C2 | - ³⁾ | - ³⁾ | - ³⁾ | - ³⁾ |
| Vergleichsbeispiel C3 | - ³⁾ | - ³⁾ | - ³⁾ | - ³⁾ |
| Vergleichsbeispiel C4 | - ³⁾ | - ³⁾ | - ³⁾ | - ³⁾ |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

**Tabelle 3B: Zeitabhängige Kontaktwinkelmessungen an kondensationsvernetzenden Alkoxysilylpolyethern mit einer erfindungsgemäßen synergistisch wirkenden Tensidkombination im Vergleich zum Stand der Technik⁴⁾**

| **Beispiele/Verglelchsbeispiele** | **Materialalter nach Mischbeginn 40 s ¹⁾ Tropfenalter 10 s Kontaktwinkel ²⁾ [s]** | **Materialalter nach Mischbeginn 60 s ¹⁾ Tropfenalter 10 s Kontaktwinkel ²⁾ [°]** | **Materialalter nach Mischbeginn 90 s ¹⁾ Tropfenalter 10 s Kontaktwinkel ²⁾ n** | **Materialalter nach Mischbeginn 120 s ¹⁾ Tropfenalter 10 s Kontaktwinkel ²⁾ [°]** |
|---|---|---|---|---|
| Beispiel C1 erfindungsgemäß | <10 | < 10 | < 10 | < 10 |
| Beispiel C2 erfindungsgemäß | < 10 | <10 | <10 | <10 |
| Beispiel C3 erfindungsgemäß | <10 | <10 | <10 | <10 |
| Beispiel CC3 erfindungsgemäß | <10 | <10 | <10 | < 10 |
| Beispiel CCC3 erfindungsgemäß | <10 | <10 | < 10 | < 10 |
| Vergleichsbeispiel C1 | 33 | 48 | 60 | 69 |
| Vergleichsbeispiel C2 | 38 | 52 | 62 | 72 |
| Vergleichsbeispiel C3 | 34 | 39 | 50 | 55 |
| Vergleichsbeispiel C4 | 47 | 49 | 53 | 49 |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1 °C mit der Messmethode "liegender Tropfen" unter Verwendung eines Edelstahlprobeträgers mit einer Vertiefung von 50 µm | | | | |

**Tabelle 4A: Zeitabhängige Kontaktwinkelmessungen an additionsvernetzenden Aziridino-Polyethern mit einer erfindungsgemäßen synergistisch wirkenden Tensidkombination im Vergleich zum Stand der Technik ⁴⁾**

| **Beispiele/Vergleichsbeispiele** | **Materialalter nach Mischbeginn 40 s ¹⁾ Zeitspanne bis ein Kontaktwinkel von 10° erreicht wird ²⁾ [8]** | **Materialalter nach Mischbeginn 60 s¹⁾ Zeitspanne bis ein Kontaktwinkel von 10° erreicht wird ²⁾ [s]** | **Materialalter nach Mischbeginn 90 s ¹⁾ Zeitspanne bis ein Kontaktwinkel von 10° erreicht wird ²⁾ [s]** | **Materialalter nach Mischbeginn 120 s ¹⁾ Zeitspanne bis ein Kontaktwinkel von 10° erreicht wird ²⁾ [s]** |
|---|---|---|---|---|
| Beispiel D1 erfindungsgemäß | <1 | <1 | <1 | <1 |
| Beispiel DD1 erfindungsgemäß | <1 | <1 | <1 | <1 |
| Beispiel D2 erfindungsgemäß | <1 | <1 | <1 | <1 |
| Beispiel D3 erfindungsgemäß | <1 | <1 | <1 | <1 |
| Beispiel DD3 erfindungsgemäß | <1 | <1 | <1 | <1 |
| Vergleichsbeispiel D1 | - ³⁾ | - ³⁾ | - ³⁾ | - ³⁾ |
| Vergleichsbeispiel D2 | - ³⁾ | - ³⁾ | - ³⁾ | - ³⁾ |
| Vergleichsbeispiel D3 | - ³⁾ | - ³⁾ | - ³⁾ | - ³⁾ |
| Vergleichsbeispiel D4 | - ³⁾ | - ³⁾ | - ³⁾ | - ³⁾ |
| Vergleichsbeispiel D5 | - ³⁾ | - ³⁾ | - ³⁾ | - ³⁾ |
| **¹⁾** Zeitpunkt des Aufsetzens des Wassertropfens nach Mischbeginn des Abformmaterials | | | | |
| **²⁾** Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1°C mit der Messmethode liegender Tropfen" unter Verwendung eines Edelstahlprobeträgers mit einer Vertiefung von 50 µm | | | | |

**Tabelle 4B: Zeitabhängige Kontaktwinkelmessungen an additionsvernetzenden Aziridino-Polyethern mit einer erfindungsgemäßen synergistisch wirkenden Tensidkombination im Vergleich zum Stand der Technik ⁴⁾**

| **Beispiele/Vergleichsbeispiele** | **Materialalter nach Mischbeginn 40 s¹⁾ Tropfenalter 10 s Kontaktwinkel ²⁾ [°]** | **Materialalter nach Mischbeginn 60 s¹⁾ Tropfenalter 10 s Kontaktwinkel.²⁾ [°]** | **Materialalter nach Mischbeginn 90 s¹⁾ Tropfenalter 10 s Kontaktwinkel ²⁾ [°]** | **Materialalter nach Mischbeginn 120 s ¹⁾ Tropfenalter 10 s Kontaktwinkel ²⁾ [°]** |
|---|---|---|---|---|
| Beispiel D1 erfindungsgemäß | <10 | <10 | <10 | <10 |
| Beispiel DD1 erfindungsgemäß | < 10 | <10 | <10 | <10 |
| Beispiel D2 erfindungsgemäß | <10 | <10 | <10 | <10 |
| Beispiel D3 erfindungsgemäß | < 10 | < 10 | <10 | <10 |
| Beispiel DD3 erfindungsgemäß | < 10 | < 10 | <10 | < 10 |
| Vergleichsbeispiel D1 | 19 | 28 | 29 | 26 |
| Vergleichsbeispiel D2 | 35 | 37 | 37 | 39 |
| Vergleichsbeispiel D3 | 52 | 49 | 50 | 56 |
| Vergleichsbeispiel D4 | 40 | 44 | 50 | 54 |
| Vergleichsbeispiel D5 | 49 | 49 | 62 | 65 |
| ¹⁾ Zeitpunkt des Aufsetzens des Wassertropfens nach Mischbeginn des Abformmaterials | | | | |
| ²⁾ Tropfengröße ca. 2 µl, Messzeit Tropfen max. 30 s | | | | |
| ³⁾ Innerhalb der Messzeit von 30s nach Wassertropfenaufgabe, wird kein Kontaktwinkel <10° erreicht | | | | |
| ⁴⁾ gemessen mit einem Kontaktwinkelmessgerät G40/G23M der Firma Krüss bei 23°C ± 1°C mit der Messmethode "liegender Tropfen" unter Verwendung eines Edelstahlprobeträgers mit einer Vertiefung von 50 µm | | | | |

## Patentansprüche

1. Dentalabformmasse enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzende Esterreste einer ethylenisch ungesättigten Carbonsäure enthaltenden Polyether oder der durch ringöffnende Metathesereaktion vernetzenden Polyether, Silicone oder Kautschuke, und enthaltend ferner ein mindestens einen (Poly)alkylenoxidrest sowie eine Silizium enthaltende Gruppe aufweisendes nichtionisches Tensid mit einer Molmasse von weniger als 6000 g/mol, und ein nichtionisches Fluortensid, **dadurch gekennzeichnet, dass** als nichtionisches Fluortensid eine Verbindung der Formel
F-(CF₂-CF₂)₁₋₇-CH₂-CH₂-(O-CH₂-CH₂)₁₋₁₅-OH
eingesetzt wird.

2. Dentalabformmasse nach Anspruch 1, **dadurch gekennzeichnet, dass** diese 40 Sekunden nach Mischbeginn einen niedrigen initialen Wassertropfen-Kontaktwinkel von < 10° aufweist, gemessen bei einem Tropfenalter von 10 Sekunden.

3. Dentalabformmasse nach Anspruch 2, **dadurch gekennzeichnet, dass** der Wassertropfen-Kontaktwinkel 40 Sekunden nach Mischbeginn folgende Werte annimmt: nach einem Tropfenalter von 0,25 Sekunden einen Wassertropfen-Kontaktwinkel von <75°, vorzugsweise von <40°; nach einem Tropfenalter von 0,5 Sekunden einen Wassertropfen-Kontaktwinkel von <55°, vorzugsweise <30°; nach einem Tropfenalter von 1 Sekunde einen Wassertropfen-Kontaktwinkel von <35°, vorzugsweise < 25°; nach einem Tropfenalter von 2 Sekunden: einen Wassertropfen-Kontaktwinkel von <20°; und nach einem Tropfenalter von 3 Sekunden einen Wassertropfen-Kontaktwinkel von <10°.

4. Dentalabformmasse nach einem der Ansprüche 1 bis 3, **dadurch gekennzeichnet, dass** das Silizium aufweisende nichtionische Tensid enthaltend mindestens einen (Poly)alkylenoxidrest eine Molmasse von weniger als 4000 g/mol, insbesondere von 350 bis 2000 g/mol, besitzt.

5. Dentalabformmasse nach einem der Ansprüche 1 bis 3, **dadurch gekennzeichnet, dass** das Silicium aufweisende nichtionische Tensid ein Organosiloxantensid der Formel II und/oder der Formel III ist oder ein Organocarbonsilantensid der Formel IV, V und/oder der Formel VI ist 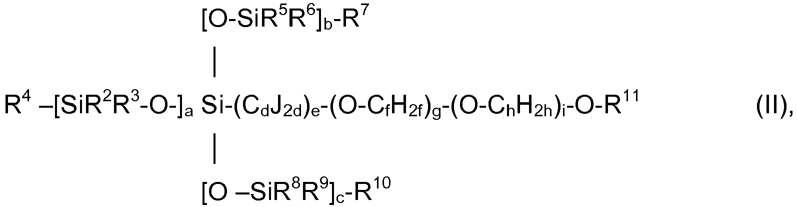 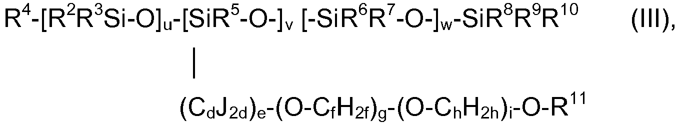 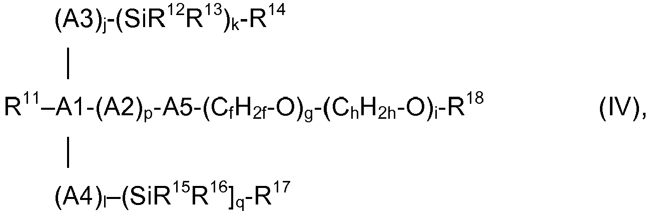 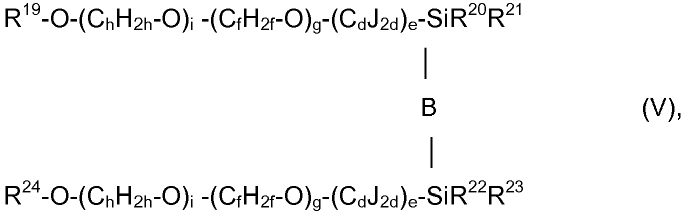
R¹⁹-O-(CₕH₂ₕ-O)ᵢ-(C_{f}H_{2f}-O)_{g}-(C_{d}J_{2d})ₑ-SiR²⁰R²¹ R²² (VI)
worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
a, b, c und w unabhängig voneinander ganze Zahlen von 0 bis 100 bedeuten, vorzugsweise 0 bis 75, insbesondere 0 bis 35 und ganz besonders bevorzugt 0 bis 15,
v eine ganze Zahl von 1 bis 100 bedeutet, vorzugsweise 1 bis 15 und ganz besonders bevorzugt 1 bis 6,
wobei die Summe von a, b und c zwischen 1 und 300 beträgt, vorzugsweise 1 bis 50, insbesondere 1 bis 10 und ganz besonders bevorzugt 1 bis 3, und die Summe von v und w zwischen 1 und 200 beträgt, vorzugsweise 2 bis 90,
u 0 oder 1 ist,
d eine ganze Zahl von 1 bis 10 ist, vorzugsweise 1 bis 6 und insbesondere 1 bis 3,
J Wasserstoff oder Fluor bedeutet, vorzugsweise Wasserstoff,
e 0 oder 1 ist,
f und h unabhängig voneinander ganze Zahlen von 2 bis 6 bedeuten,
g und i unabhängig voneinander ganze Zahlen von 0 bis 30 sind, vorzugsweise 0 bis 15, wobei die Summe von g und i 1 bis 60 bedeutet, vorzugsweise 2 bis 30, insbesondere 2 bis 15,
R¹¹ Wasserstoff, Alkyl, Alkenyl oder Aryl ist, das gegebenenfalls teilweise oder vollständig fluoriert ist, vorzugsweise Wasserstoff oder Methyl,
R¹², R¹³, R¹⁴, R¹⁵, R¹⁶ und R¹⁷ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
k und q unabhängig voneinander 0 oder 1 bedeuten,
A1 Kohlenstoff oder Silizium bedeutet,
A2, A3 und A4 unabhängig voneinander eine Gruppe C_{d}J_{2d} ist, worin J und d die oben definierten Bedeutungen aufweisen,
j, p und l unabhängig voneinander 0 oder 1 sind,
A5 eine zweiwertige Brückengruppe, insbesondere -O-, -CO-O- oder -CObedeutet,
R¹⁸ Wasserstoff, Alkyl, Alkenyl oder Aryl ist, das gegebenenfalls teilweise oder vollständig fluoriert ist, vorzugsweise Wasserstoff oder Methyl,
R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
BG eine zweiwertige Brückengruppe ist, und
R¹⁹ und R²⁴ unabhängig voneinander Wasserstoff, Alkyl, Alkenyl oder Aryl sind, die gegebenenfalls teilweise oder vollständig fluoriert sind, vorzugsweise Wasserstoff oder Methyl,
mit der Massgabe, dass von den Resten R², R³ und R⁴ und/oder den Resten R⁵, R⁶ und R⁷ und/oder den Resten R⁸, R⁹ und R¹⁰ und/oder den Resten R¹⁵, R¹⁶ und R¹⁷ und/oder den Resten R²⁰ und R²¹ und/oder den Resten R²² und R²³ und/oder den Resten R²⁰, R²¹ und R²² nur einer Wasserstoff sein kann,
wobei f und h innerhalb eines Moleküls im Rahmen der gegebenen Definition unterschiedliche Werte annehmen können.

6. Dentalabformmasse nach Anspruch 5, **dadurch gekennzeichnet, dass** das Silizium aufweisende nichtionische Tensid enthaltend mindestens einen (Poly)alkylenoxidrest eine Verbindung der Formel VII, VIII, IX oder X ist 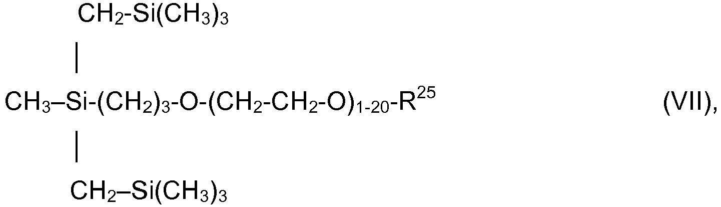 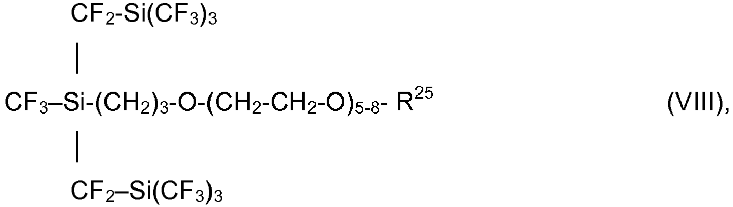 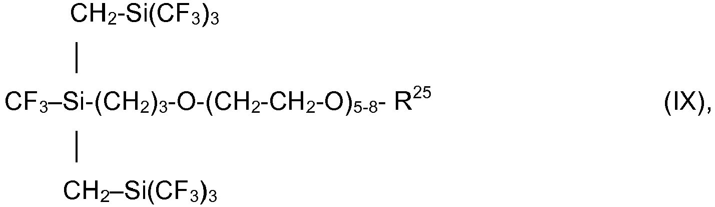 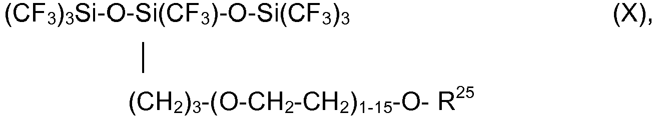 worin R²⁵ Wasserstoff, Methyl, Ethyl, Propyl oder Butyl ist, vorzugsweise Wasserstoff oder Methyl.

7. Dentalabformmasse nach einem der Ansprüche 1 bis 6, **dadurch gekennzeichnet, dass** diese nebem dem Silizium aufweisenden nichtionischen Tensid und nichtionischen Fluortensid als weitere Komponente einen Alkenylreste und/oder Alkinylreste enthaltenden Polyether und/oder einen hydroxyl- und/oder aryloxy- und/oder arylalkyloxy- und/oder alkoxy-terminierten Polyether enthält.

8. Dentalabformmasse nach einem der Ansprüche 1 bis 6, **dadurch gekennzeichnet, dass** diese nebem dem Silizium aufweisenden nichtionischen Tensid und nichtionischen Fluortensid als weitere Komponente ein Polyol enthält.

9. Dentalabformmasse nach Anspruch 8, **dadurch gekennzeichnet, dass** diese als weitere Komponente einen Alkenylreste und/oder Alkinylreste enthaltenden Polyether und/oder einen aryloxy- und/oder arylalkyloxy- und/oder hydroxyl- und/oder alkoxy-terminierten Polyether enthält.

10. Dentalabformmasse nach einem der Ansprüche 8 bis 9, **dadurch gekennzeichnet, dass** das Polyol ausgewählt wird aus der Gruppe der Kohlehydrate, der Polyvinylalkohole, der aliphatischen Di-, Tri-, Tetra-, Penta- und/oder Hexaole und Mischungen von zwei oder mehreren dieser Polyole.

11. Dentalabformmasse nach Anspruch 10, **dadurch gekennzeichnet, dass** das Polyol ausgewählt wird aus der Gruppe der Polyvinylalkohole, der Polysaccharide, Trimethylolpropan, Pentaerythritol, Dipentaerythritol, Glycerin, Allyloxy-1,2-propandiol, 2-Methyl-2,4-pentandiol, Trimethylolpropanallylether, Decandiol, Nonandiol, Octandiol, Heptandiol, Hexandiol, Pentandiol, Butandiol, Propandiol, Ethandiol, Fructose, Glucose und Mischungen von zwei oder mehreren dieser Polyole, insbesondere Glycerin.

12. Dentalabformmasse nach Anspruch 7, **dadurch gekennzeichnet, dass** der Alkenylreste enthaltende Polyether eine Verbindung der Formel XII und der hydroxyl- und/oder alkoxy-terminierte Polyether eine Verbindung der Formel XIII ist
CH₂=CH-CH₂-O-(Cₙ₂H₂ₙ₂-O)ₘ₉-CH₂-CH=CH₂ (XII),
R³¹-O-(Cₙ₂H₂ₙ₂-O)ₘ₉-R³² (XIII),
worin n2 eine ganze Zahl von 2 bis 8, vorzugsweise von 2 bis 4 bedeutet, m9 eine ganze Zahl von 3 bis 70000, vorzugsweise von 10 bis 2500 ist, R³¹ und R³² unabhängig voneinander Wasserstoff oder C₁-C₆-Alkyl, insbesondere Wasserstoff und/oder Methyl, Ethyl oder Propyl bedeuten, und wobei R³¹, R³², n2 und m9 innerhalb eines Moleküls im Rahmen der gegebenen Definitionen unterschiedlich sein können.

13. Dentalabformmasse nach einem der Ansprüche 1 bis 12, **dadurch gekennzeichnet, dass** das Gewichtsverhältnis von Silizium aufweisendem Tensid zu Fluortensid 100 : 1 bis 1 : 100, bevorzugt 50 : 1 bis 1 : 50, besonders bevorzugt 10 : 1 bis 1 : 10 und insbesondere bevorzugt 5 : 1 bis 1 : 5 beträgt.

14. Dentalabformmasse nach einem der Ansprüche 1 bis 13, **dadurch gekennzeichnet, dass** diese eine durch Additionsreaktion vernetzende Organopolysiloxan-Mehrkomponenten Dentalabformmasse enthaltend Komponenten A und B ist, worin
a) Komponente A ein Organopolysiloxan mit mindestens zwei ethylenisch ungesättigten Gruppen und einen Hydrosilylierungskatalysator,
b) Komponente B ein Organohydrogenpolysiloxan, und
c) mindestens eine der Komponenten A und/oder B ein Silizium aufweisendes nichtionisches Tensid und/oder ein nichtionisches Fluortensid enthält und Komponenten A und B weisen eine solche Zusammensetzung auf, so dass nach dem Vermischen der Komponenten A und B eine Kombination von Silizium aufweisendem nichtionischem Tensid und nichtionischem Fluortensid vorliegt.

15. Dentalabformmasse nach einem der Ansprüche 1 bis 14, **dadurch gekennzeichnet, dass** diese eine durch Kondensationsreaktion vernetzende Organopolysiloxan-Mehrkomponenten Dentalabformmasse enthaltend Komponenten C und D ist, worin
d) Komponente C ein Organopolysiloxan mit mindestens zwei Hydroxylgruppen,
e) Komponente D ein Kieselsäureester, Polykieselsäureester und/oder ein Organopolysiloxan mit mindestens zwei Alkoxygruppen sowie einen Kondensationskatalysator, und
f) mindestens eine der Komponenten C und/oder D ein Silizium aufweisendes nichtionisches Tensid und/oder ein nichtionisches Fluortensid enthält, und Komponenten C und D eine solche Zusammensetzung aufweisen, so dass nach dem Vermischen der Komponenten C und D eine Kombination von Silizium aufweisendem nichtionischem Tensid und nichtionischem Fluortensid vorliegt.

16. Dentalabformmasse nach einem der Ansprüche 1 bis 14, **dadurch gekennzeichnet, dass** diese eine durch Additionsreaktion vernetzende Alkenylreste enthaltende Polyether Mehrkomponenten Dentalabformmasse enthaltend Komponenten E und F ist, worin
g) Komponente E einen Vernetzungskatalysator,
h) Komponente F einen vernetzbaren und Alkenylreste aufweisenden Polyether sowie ein Organohydrogenpolysiloxan und/oder SiH-Polyether, und
i) mindestens eine der Komponenten E und/oder F ein Silicium aufweisendes nichtionisches Tensid und/oder ein nichtionisches Fluortensid enthält, und Komponenten E und F eine solche Zusammensetzung aufweisen, so dass nach dem Vermischen der Komponenten E und F eine Kombination von Silizium aufweisendem nichtionischem Tensid und nichtionischem Fluortensid vorliegt.

17. Dentalabformmasse nach einem der Ansprüche 1 bis 14, **dadurch gekennzeichnet, dass** diese eine Alkoxysilylreste enthaltende Polyether Mehrkomponenten Dentalabformmasse enthaltend Komponenten G und H ist, worin
j) Komponente G einen vernetzbaren und Alkoxysilylreste aufweisenden Polyether,
k) Komponente H Wasser, und
l) mindestens eine der Komponenten G und/oder H einen Katalysator sowie ein Silizium aufweisendes nichtionisches Tensid und/oder ein nichtionisches Fluortensid enthält, und Komponenten G und H eine solche Zusammensetzung aufweisen, so dass nach dem Vermischen der Komponenten G und H eine Kombination von Silizium aufweisendem nichtionischem Tensid und nichtionischem Fluortensid vorliegt.

18. Dentalabformmasse enthaltend vernetzbare Polyether, die Alkoxysilylreste, Aziridinoreste, von einer ethylenisch ungesättigten Carbonsäure abgeleitete Reste oder Alkenylreste als vernetzbare Gruppen oder über ringöffnende Metathesereaktion vernetzbare Gruppen aufweisen, und ein nichtionisches Fluortensid, das mindestens einen teil- oder perfluorierten Kohlenwasserstoffrest aufweist, der über ein Sauerstoffatom, eine Aminogruppe, eine Ketogruppe, eine Carbonsäureestergruppe, eine Phosphorsäureester-gruppe, eine Carbonsäureamidgruppe und/oder eine Phosphorsäureamid-gruppe mit einem (Poly)alkylenoxidrest, einem Kohlenhydratrest, einem aliphatischen Polyhydroxyrest oder einem Stickstoff enthaltenden heterocyclischen Rest verbunden ist oder das mindestens einen teil- oder perfluorierten Kohlenwasserstoffrest und mindestens einen Aminoxidrest aufweist.

19. Dentalabformmasse nach Anspruch 18, **dadurch gekennzeichnet, dass** das Fluortensid ein mindestens einen (Poly)alkylenoxidrest aufweisendes nichtionisches Fluortensid ist, das aus einem teil- oder perfluorierten Kohlenwasserstoffrest besteht, der über ein Sauerstoffatom oder eine Estergruppe als Brückengruppe mit einem (Poly)alkylenoxidrest verbunden ist.

20. Dentalabformmasse nach Anspruch 18, **dadurch gekennzeichnet, dass** diese vernetzbare Polyether mit Alkoxysilylresten oder mit Aziridinoresten als vernetzbaren Gruppen aufweist oder dass dieses vernetzbare Polyether mit über ringöffnende Metathesereaktion vernetzbaren Gruppen aufweist.

21. Dentalabformmasse nach Anspruch 18 bis 20, **dadurch gekennzeichnet, dass** diese zusätzlich mindestens ein eine Silizium enthaltende Gruppe aufweisendes nichtionisches Tensid mit einer Molmasse von weniger als 6000 g/mol enthält.

22. Dentalabformmasse nach einem der Ansprüche 18 bis 21, **dadurch gekennzeichnet, dass** diese als zusätzliche Komponenten Polyole und/oder Alkenylreste und/oder Alkinylreste enthaltende Polyether und/oder hydroxyl- und/oder alkoxy-terminierte Polyether enthält.

23. Dentalabformmasse nach einem der Ansprüche 18 bis 22, **dadurch gekennzeichnet, dass** diese eine Mehrkomponenten-Dentalabformmasse enthaltend Komponenten I und J ist, worin
m) Komponente I einen vernetzbaren und Alkoxysilylreste oder Aziridinoreste aufweisenden Polyether oder einen über ringöffnende Metathesereaktion vernetzbare Gruppen enthaltenden Polyether,
n) Komponente J einen Katalysator, und
o) mindestens eine der Komponenten I und/oder J ein nichtionisches Fluortensid und gegebenenfalls ein Silizium aufweisendes nichtionisches Tensid enthält, wobei Komponenten I und J vorzugsweise eine solche Zusammensetzung aufweisen, so dass nach dem Vermischen der Komponenten I und J eine Kombination von Silizium aufweisendem nichtionischem Tensid und nichtionischem Fluortensid vorliegt.

24. Dentalabformmasse nach einem der Ansprüche 1 bis 14, **dadurch gekennzeichnet, dass** diese einen vernetzbaren Polyalkylenether, der von Acrylsäure und/oder Methacrylsäure abgeleitete Reste aufweist, einen chemisch oder durch Strahlung aktivierbaren Initiator sowie ein Silizium aufweisendes nichtionisches Tensid und ein nichtionisches Fluortensid enthält.

25. Dentalabformmasse nach einem der Ansprüche 1 bis 14, **dadurch gekennzeichnet, dass** diese eine über ringöffnende Metathese Polymerisation (ROMP) vernetzbare Gruppen aufweisende Polyether, Polysiloxane und/oder Synthesekautschuke enthaltende Polyether Mehrkomponenten Dentalabformmasse enthaltend Komponenten K und L ist, worin
p) Komponente K Polyether, Polysiloxane und/oder Synthesekautschuke, die über ROMP vernetzbare Gruppen aufweisen,
q) Komponente L einen ROMP Vernetzungskatalysator, und
r) mindestens eine der Komponenten K und/oder L ein Silizium aufweisendes nichtionisches Tensid und/oder ein nichtionisches Fluortensid enthält, wobei Komponenten K und L eine solche Zusammensetzung aufweisen, so dass nach dem Vermischen der Komponenten K und L eine Kombination von Silizium aufweisendem nichtionischem Tensid und nichtionischem Fluortensid vorliegt.

26. Dentalabformmasse nach einem der Ansprüche 1 bis 25, **dadurch gekennzeichnet, dass** diese Füllstoffe enthält, vorzugsweise in einem Gesamtanteil von 0,01 bis 80 Gew.-%, besonders bevorzugt 0,05 bis 75 Gew.-%, und ganz besonders bevorzugt 0,1 bis 70 Gew.-%, bezogen auf die gesamte Dentalabformmasse.

27. Dentalabformmasse nach einem der Ansprüche 1 bis 26, **dadurch gekennzeichnet, dass** diese ein oder mehrere der folgenden Zusatzstoffe enthält: Puffersalze, Wasserfänger, Pastenbildner, weitere Tenside, Wirkstoffe, Weichmacher, optische Abtastung ermöglichende Substanzen, Geschmacks- und/oder Geruchsstoffe, Diagnostik ermöglichende Substanzen, Fluoridisierungsmittel, Bleichsubstanzen, Desensibilisierungsmittel, Haftverbundvermittler, Farbstoffe, Indikatoren, Stabilisatoren (Antioxidantien) sowie antibakterielle Substanzen.

28. Mischungen erhältlich durch Vermischen der Dentalabformmassen nach einem der Ansprüche 1 bis 27.

29. Ausgehärtetes Abformmaterial erhältlich durch Aushärten der Dentalabformmassen nach einem der Ansprüche 1 bis 28.

30. Verwendung einer Mischung aus Silizium aufweisendem nichtionischem Tensid, das mindestens einen (Poly)alkylenoxidrest und eine Molmasse von weniger als 6000 g/mol aufweist, und aus nichtionischem Fluortensid, das mindestens einen teil- oder perfluorierten Kohlenwasserstoffrest aufweist, der über ein Sauerstoffatom mit einem (Poly)alkylenoxidrest verbunden ist, zur Herstellung von elastomeren Dentalabformmassen, **dadurch gekennzeichnet, dass** als nichtionisches Fluortensid eine Verbindung der Formel
F-(CF₂-CF₂)₁₋₇-CH₂-CH₂-(O-CH₂-CH₂)₁₋₁₅-OH
eingesetzt wird.

31. Verwendung einer Mischung nach Anspruch 30, **dadurch gekennzeichnet, dass** diese zusätzlich Alkenyl-, Alkinyl-, Alkyloxy-, Aryloxy-, Arylalkyloxy- und/oder Hydroxy-endgestoppten Polyalkylenether enthält.

32. Verwendung einer Mischung nach Anspruch 30, **dadurch gekennzeichnet, dass** diese zusätzlich Polyol enthält.

## Claims

1. A dental impression substance containing curable polymers selected from the group of organopolysiloxanes which crosslink by an addition reaction, organopolysiloxanes which crosslink by a condensation reaction, polyethers which contain alkoxy silyl radicals and crosslink by a condensation reaction, polyethers which contain aziridino radicals and crosslink by an addition reaction, polyethers which contain alkenyl radicals and crosslink by an addition reaction, polyethers which contain ester radicals of an ethylenically unsaturated carboxylic acid and crosslink by a radical polymerisation reaction, or polyethers which crosslink by a ring-opening metathesis reaction, silicones, or rubbers, and further containing at least one (poly)alkylene oxide radical as well as a non-ionic surfactant comprising a silicon-containing group and having a molar mass of less than 6000 g/mol, and a non-ionic fluorosurfactant, **characterised in that** the used non-ionic fluorosurfactant is a compound of formula
F-(CF₂-CF₂)₁₋₇-CH₂-CH₂-(O-CH₂-CH₂)₁₋₁₅-OH.

2. The dental impression substance according to claim 1, **characterised in that** it has a low initial water droplet contact angle of < 10° 40 seconds after the onset of mixing, measured at a droplet age of 10 seconds.

3. The dental impression substance according to claim 2, **characterised in that** the water droplet contact angle 40 seconds after the onset of mixing assumes the following values: after a droplet age of 0.25 seconds, a water droplet contact angle of < 75°, preferably of < 40°; after a droplet age of 0.5 seconds, a water droplet contact angle of < 55°, preferably < 30°; after a droplet age of 1 second, a water droplet contact angle of < 35°, preferably < 25°; after a droplet age of 2 seconds, a water droplet contact angle of < 20°; and after a droplet age of 3 seconds, a water droplet contact angle of < 10°.

4. The dental impression substance according to any one of claims 1 to 3, **characterised in that** the silicon-comprising non-ionic surfactant containing at least one (poly)alkylene oxide radical has a molar mass of less than 4000 g/mol, in particular of 350 to 2000 g/mol.

5. The dental impression substance according to any one of claims 1 to 3, **characterised in that** the silicon-comprising non-ionic surfactant is an organosiloxane surfactant of formula II and/or of formula III, or an organocarbosilane surfactant of formula IV, V and/or of formula VI 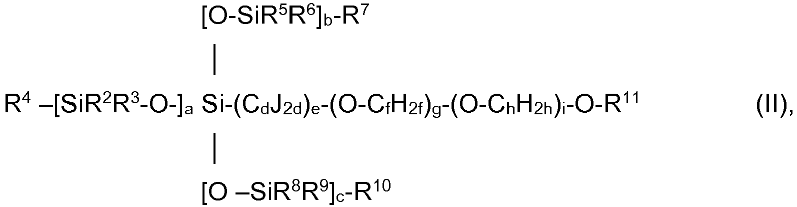 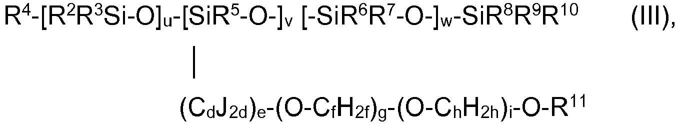 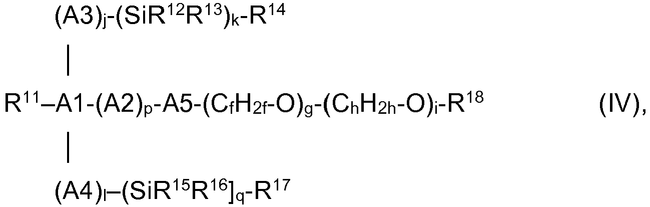 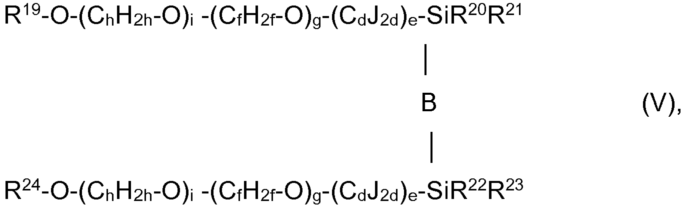
R¹⁹-O-(CₕH₂ₕ-O)ᵢ-(C_{f}H_{2f}-O)_{g}-(C_{d}J_{2d})ₑ₋SiR²⁰R²¹R²² (VI)
in which R², R³, R⁴, R⁵, R⁶, R⁷, R⁸, R⁹ and R¹⁰, independently of one another, stand for hydrogen, alkyl, alkyloxy, alkenyl, alkenyloxy, alkynyl, alkynyloxy, aryl, aryloxy, aralkyl, aralkyloxy, alkylaryl and/or alkylaryloxy, which are optionally fluorinated in part or completely, preferably alkyl or alkenyl and in particular C₁-C₆-alkyl,
a, b, c and w, independently of one another, stand for integers from 0 to 100, preferably 0 to 75, in particular 0 to 35, and most preferably 0 to 15,
v stands for an integer from 1 to 100, preferably 1 to 15, and most preferably 1 to 6,
in which the sum of a, b and c is between 1 and 300, preferably 1 to 50, in particular 1 to 10, and most preferably 1 to 3,
and the sum of v and w is between 1 and 200, preferably 2 to 90,
u is 0 or 1,
d is an integer from 1 to 10, preferably 1 to 6, and in particular 1 to 3,
J stands for hydrogen or fluorine, preferably hydrogen,
e is 0 or 1,
f and h, independently of one another, stand for integers from 2 to 6,
g and i, independently of one another, are integers from 0 to 30, preferably 0 to 15, in which the sum of g and i stands for 1 to 60, preferably 2 to 30, in particular 2 to 15,
R¹¹ is hydrogen, alkyl, alkenyl or aryl, which is optionally fluorinated in part or completely, preferably hydrogen or methyl,
R¹², R¹³, R¹⁴, R¹⁵, R¹⁶ and R¹⁷, independently of one another, stand for hydrogen, alkyl, alkyloxy, alkenyl, alkenyloxy, alkynyl, alkynyloxy, aryl, aryloxy, aralkyl, aralkyloxy, alkylaryl and/or alkylaryloxy, which are optionally fluorinated, in part or completely, preferably alkyl or alkenyl, and in particular C₁-C₆-alkyl,
k and q, independently of one another, stand for 0 or 1,
A1 stands for carbon or silicon,
A2, A3 and A4, independently of one another, are a C_{d}J_{2d} group, in which J and d have the meanings defined above,
j, p and l, independently of one another, are 0 or 1,
A5 stands for a bivalent bridge group, in particular -O-, -CO-O- or -CO-,
R¹⁸ is hydrogen, alkyl, alkenyl or aryl, which is optionally fluorinated in part or completely, preferably hydrogen or methyl,
R²⁰, R²¹, R²² and R²³, independently of one another, stand for hydrogen, alkyl, alkyloxy, alkenyl, alkenyloxy, alkynyl, alkynyloxy, aryl, aryloxy, aralkyl, aralkyloxy, alkylaryl and/or alkylaryloxy, which are optionally fluorinated, in part or completely, preferably alkyl or alkenyl, and in particular C₁-C₆-alkyl,
BG is a divalent bridge group, and
R¹⁹ and R²⁴, independently of one another, are hydrogen, alkyl, alkenyl or aryl, which are optionally fluorinated in part or completely, preferably hydrogen or methyl,
with the proviso that among the radicals R², R³ and R⁴ and/or the radicals R⁵, R⁶ and R⁷ and/or the radicals R⁸, R⁹ and R¹⁰ and/or the radicals R¹⁵, R¹⁶ and R¹⁷ and/or the radicals R²⁰ and R²¹ and/or the radicals R²² and R²³ and/or the radicals R²⁰, R²¹ and R²², only one can be hydrogen,
in which f and h, within a molecule, can assume different value within the scope of the given definition.

6. The dental impression substance according to claim 5, **characterised in that** the silicon-comprising non-ionic surfactant containing at least one (poly)alkylene oxide radical is a compound of formula VII, VIII, IX or X 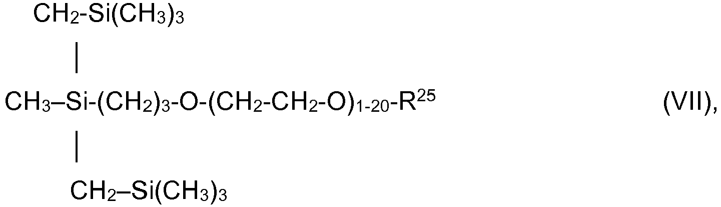 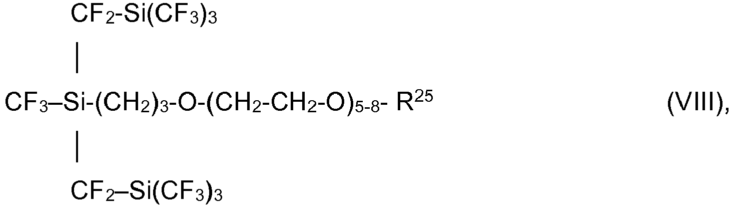 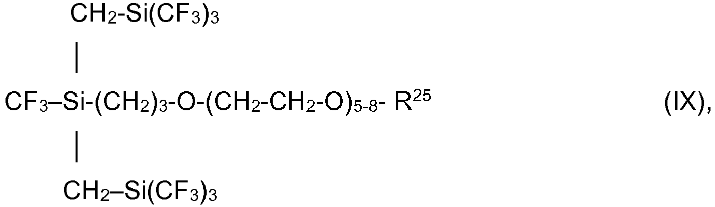 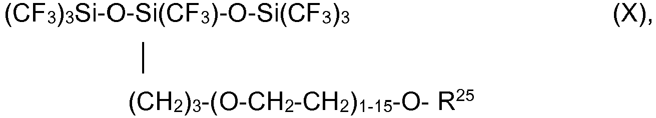 in which R²⁵ is hydrogen, methyl, ethyl, propyl or butyl, preferably hydrogen or methyl.

7. The dental impression substance according to any one of claims 1 to 6, **characterised in that**, in addition to the silicon-comprising non-ionic surfactant and non-ionic fluorosurfactant, it contains as a further component a polyether containing alkenyl radicals and/or alkynyl radicals, and/or a polyether terminated with hydroxyl and/or aryloxy and/or arylalkyloxy and/or alkoxy.

8. The dental impression substance according to any one of claims 1 to 6, **characterised in that**, in addition to the silicon-comprising non-ionic surfactant and non-ionic fluorosurfactant, it contains as a further component a polyol.

9. The dental impression substance according to claim 8, **characterised in that** it contains as a further component a polyether containing alkenyl radicals and/or alkynyl radicals, and/or a polyether terminated with aryloxy and/or arylalkyloxy and/or hydroxyl and/or alkoxy.

10. The dental impression substance according to either claim 8 or claim 9, **characterised in that** the polyol is selected from the group of carbohydrates, polyvinyl alcohols, aliphatic diols, triols, tetraols, pentaols and/or hexaols, and mixtures of two or more of these polyols.

11. The dental impression substance according to claim 10, **characterised in that** the polyol is selected from the group of polyvinyl alcohols, polysaccharides, trimethylol propane, pentaerythritol, dipentaerythritol, glycerol, allyloxy-1,2-propane diol, 2-methyl-2,4-pentane diol, trimethylol propane allyl ether, decane diol, nonane diol, octane diol, heptane diol, hexane diol, pentane diol, butane diol, propane diol, ethane diol, fructose, glucose, and mixtures of two or more of these polyols, in particular glycerol.

12. The dental impression substance according to claim 7, **characterised in that** the polyether containing alkenyl radicals is a compound of formula XII, and the polyether terminated with hydroxyl and/or alkoxy is a compound of formula XIII
CH₂=CH-CH₂-O-(Cₙ₂H₂ₙ₂-O)ₘ₉-CH₂-CH=CH₂ (XII),
R³¹-O-(Cₙ₂H₂ₙ₂-O)ₘ₉-R³² (XIII),
in which n2 stands for an integer from 2 to 8, preferably from 2 to 4,
m9 is an integer from 3 to 70000, preferably from 10 to 2500,
R³¹ and R³², independently of one another, stand for hydrogen or C₁-C₆-alkyl, in particular hydrogen and/or methyl, ethyl or propyl,
and in which R³¹, R³², n2 and m9, within a molecule, can be different within the scope of the given definitions.

13. The dental impression substance according to any one of claims 1 to 12, **characterised in that** the ratio by weight of silicon-comprising surfactant to fluorosurfactant is 100:1 to 1:100, preferably 50:1 to 1:50, more preferably 10:1 to 1:10, and in particular preferably 5:1 to 1:5.

14. The dental impression substance according to any one of claims 1 to 13, **characterised in that** it is an organopolysiloxane multi-component dental impression substance which crosslinks by an addition reaction and contains components A and B, in which
a) component A contains an organopolysiloxane with at least two ethylenically unsaturated groups and a hydrosilylation catalyst,
b) component B contains an organohydrogen polysiloxane, and
c) at least one of the components A and/or B contains a silicon-comprising non-ionic surfactant and/or a non-ionic fluorosurfactant, and components A and B have such a composition so that once the components A and B are mixed, a combination of silicon-comprising non-ionic surfactant and non-ionic fluorosurfactant is present.

15. The dental impression substance according to any one of claims 1 to 14, **characterised in that** it is an organopolysiloxane multi-component dental impression substance which crosslinks by a condensation reaction and contains components C and D, in which
d) component C contains an organopolysiloxane with at least two hydroxyl groups,
e) component D contains a silicic acid ester, polysilicic acid ester and/or an organopolysiloxane with at least two alkoxy groups, as well as a condensation catalyst, and
f) at least one of the components C and/or D contains a silicon-comprising non-ionic surfactant and/or a non-ionic fluorosurfactant, and components C and D have such a composition so that once the components C and D are mixed, a combination of silicon-comprising non-ionic surfactant and non-ionic fluorosurfactant is present.

16. The dental impression substance according to any one of claims 1 to 14, **characterised in that** it is a polyether multi-component dental impression substance which crosslinks by an addition reaction, contains alkenyl radicals and contains components E and F, in which
g) component E contains a crosslinking catalyst,
h) component F contains a crosslinkable polyether which comprises alkenyl radicals, as well as an organohydrogen polysiloxane and/or SiH-polyether, and
i) at least one of the components E and/or F contains a silicon-comprising non-ionic surfactant and/or a non-ionic fluorosurfactant, and components E and F have such a composition so that once the components E and F are mixed, a combination of silicon-comprising non-ionic surfactant and non-ionic fluorosurfactant is present.

17. The dental impression substance according to any one of claims 1 to 14, **characterised in that** it is a polyether multi-component dental impression substance which contains alkoxy silyl radicals and components G and H, in which
j) component G contains a crosslinkable polyether comprising alkoxy silyl radicals,
k) component H contains water, and
l) at least one of the components G and/or H contains a catalyst as well as a silicon-comprising non-ionic surfactant and/or a non-ionic fluorosurfactant, and components G and H have such a composition so that once the components G and H are mixed, a combination of silicon-comprising non-ionic surfactant and non-ionic fluorosurfactant is present.

18. A dental impression substance containing crosslinkable polyethers which comprise alkoxy silyl radicals, aziridino radicals, radicals derived from an ethylenically unsaturated carboxylic acid or alkenyl radicals as crosslinkable groups, or groups which can crosslink by means of a ring-opening metathesis reaction, and a non-ionic fluorosurfactant which has at least one partially fluorinated or perfluorinated hydrocarbon radical which is connected to a (poly)alkylene oxide radical, a carbohydrate radical, an aliphatic polyhydroxy radical, or a heterocyclic radical which contains nitrogen by means of an oxygen atom, an amino group, a keto group, a carboxylic acid ester group, a phosphoric acid ester group, a carboxylic acid amide group and/or a phosphoric acid amide group, or which has at least one partially fluorinated or perfluorinated hydrocarbon radical and at least one amine oxide radical.

19. The dental impression substance according to claim 18, **characterised in that** the fluorosurfactant is a non-ionic fluorosurfactant which has at least one (poly)alkylene oxide radical and consists of a partially fluorinated or perfluorinated hydrocarbon radical which is connected to a (poly)alkylene oxide radical by means of an oxygen atom or an ester group as a bridge group.

20. The dental impression substance according to claim 18, **characterised in that** it has crosslinkable polyethers with alkoxy silyl radicals or with aziridino radicals as crosslinkable groups, or **in that** this crosslinkable polyether has groups which can crosslink by means of a ring-opening metathesis reaction.

21. The dental impression substance according to any one of claims 18 to 20, **characterised in that** it additionally contains at least one non-ionic surfactant which comprises a silicon-containing group and has a molar mass of less than 6000 g/mol.

22. The dental impression substance according to any one of claims 18 to 21, **characterised in that** it contains as an additional component polyols and/or polyethers containing alkenyl radicals and/or alkynyl radicals, and/or polyethers terminated with hydroxyl and/or alkoxy.

23. The dental impression substance according to any one of claims 18 to 22, **characterised in that** it is a multi-component dental impression substance containing components I and J, in which
m) component I contains a crosslinkable polyether comprising alkoxy silyl radicals or aziridino radicals, or a polyether containing groups which can crosslink by means of a ring-opening metathesis reaction,
n) component J contains a catalyst, and
o) at least one of the components I and/or J contains a non-ionic fluorosurfactant and optionally a silicon-comprising non-ionic surfactant, in which components I and J preferably have such a composition so that once the components I and J are mixed, a combination of silicon-comprising non-ionic surfactant and non-ionic fluorosurfactant is present.

24. The dental impression substance according to any one of claims 1 to 14, **characterised in that** it contains a crosslinkable polyalkylene ether which comprises radicals derived from acrylic acid and/or methacrylic acid, an initiator which can be activated chemically or by radiation, as well as a silicon-comprising non-ionic surfactant and a non-ionic fluorosurfactant.

25. The dental impression substance according to any one of claims 1 to 14, **characterised in that** it is a multi-component dental impression substance which contains polyethers comprising groups which can crosslink by means of ring-opening metathesis polymerisation (ROMP), polyethers containing polysiloxanes and/or synthetic rubbers, and components K and L, in which
p) component K contains polyethers, polysiloxanes and/or synthetic rubbers comprising groups which can crosslink by means of ROMP,
q) component L contains a ROMP crosslinking catalyst, and
r) at least one of the components K and/or L contains a silicon-comprising non-ionic surfactant and/or a non-ionic fluorosurfactant, in which components K and L have such a composition so that once the components K and L are mixed, a combination of silicon-comprising non-ionic surfactant and non-ionic fluorosurfactant is present.

26. The dental impression substance according to any one of claims 1 to 25, **characterised in that** it contains fillers, preferably in a total proportion of 0.01 to 80 % by weight, more preferably 0.05 to 75 % by weight, and most preferably 0.1 to 70 % by weight, based the total dental impression substance.

27. The dental impression substance according to any one of claims 1 to 26, **characterised in that** it contains one or more of the following additives: buffer salts, water collectors, paste-forming agents, other surfactants, active substances, plasticisers, substances which make optical scanning possible, flavourings and/or aromas, substances which make diagnostics possible, fluoridation agents, bleach substances, desensitisation agents, adhesive bond mediators, dyes, indicators, stabilisers (antioxidants) as well as antibacterial substances.

28. Mixtures which can be obtained by mixing the dental impression substances according to claim any one of claims 1 to 27.

29. A cured impression material which can be obtained by curing the dental impression substances according to any one of claims 1 to 28.

30. A use of a mixture of silicon-comprising non-ionic surfactant which comprises at least one (poly)alkylene oxide radical and has a molar mass of less than 6000 g/mol, and of non-ionic fluorosurfactant which comprises at least one partially fluorinated or perfluorinated hydrocarbon radical which is connected to a (poly)alkylene oxide radical, for the production of elastomer dental impression substances **characterised in that** the used non-ionic fluorosurfactant is a compound of formula
F-(CF₂-CF₂)₁₋₇-CH₂-CH₂-(O-CH₂-CH₂)₁₋₁₅-OH.

31. The use of a mixture according to claim 30, **characterised in that** it additionally contains alkenyl, alkynyl, alkyloxy, aryloxy, arylalkyloxy and/or hydroxy endstopped polyalkylene ether.

32. The use of a mixture according to claim 30, **characterised in that** it additionally contains polyol.

## Revendications

1. Matériau d'empreinte dentaire contenant des polymères durcissables choisis dans le groupe des organopolysiloxanes subissant une réticulation par réaction d'addition, des organopolysiloxanes subissant une réticulation par réaction de condensation, des polyéthers contenant des radicaux alkoxysilyle subissant une réticulation par réaction de condensation, des polyéthers contenant des radicaux aziridino subissant une réticulation par réaction d'addition, des polyéthers contenant des radicaux alcényle subissant une réticulation par réaction d'addition, des polyéthers contenant des radicaux d'esters d'un acide carboxylique éthyléniquement insaturé, lesquels subissent une réticulation par réaction de polymérisation radicalaire, ou des polyéthers, silicones ou caoutchoucs subissant une réticulation par réaction de métathèse impliquant une ouverture de cycle, et contenant en outre un agent tensioactif non-ionique d'une masse moléculaire inférieure à 6 000 g/mol comportant au moins un radical de (poly)oxyde d'alkylène ainsi qu'un groupe contenant du silicium, et un agent tensioactif fluoré de type non-ionique, **caractérisé en ce que** l'on met en oeuvre, en tant qu'agent tensioactif fluoré de type non-ionique un composé de la formule suivante :
F-(CF₂-CF₂)₁₋₇-CH₂-CH₂-(O-CH₂-CH₂)₁₋₁₅-OH

2. Matériau d'empreinte dentaire selon la revendication 1, **caractérisé en ce qu'**il présente, 40 secondes après le début du processus de mélange, un faible angle de contact initial de < 10° avec une goutte d'eau, la mesure étant réalisée 10 secondes après la pose de ladite goutte.

3. Matériau d'empreinte dentaire selon la revendication 2, **caractérisé en ce que** 40 secondes après le début du processus de mélange, ledit angle de contact avec une goutte d'eau correspond aux valeurs suivantes : 0,25 secondes après la pose d'une goutte d'eau à un angle de contact avec ladite goutte d'eau de < 75°, préférentiellement < 40° ; 0,5 secondes après la pose d'une goutte d'eau à un angle de contact avec ladite goutte d'eau de < 55°, préférentiellement de < 30° ; 1 seconde après la pose d'une goutte d'eau à un angle de contact avec ladite goutte d'eau de < 35°, préférentiellement de < 25° ; 2 secondes après la pose d'une goutte d'eau : à un angle de contact avec ladite goutte d'eau de < 20° ; et 3 secondes après la pose d'une goutte d'eau à un angle de contact avec ladite goutte d'eau de < 10°.

4. Matériau d'empreinte dentaire selon l'une des revendications 1 à 3, caractérisé ce en que ledit agent tensioactif non-ionique comportant du silicium et contenant au moins un radical (poly)oxyde d'alkylène possède une masse moléculaire inférieure à 4 000 g/mol, comprise notamment entre 350 et 2 000 g/mol.

5. Matériau d'empreinte dentaire selon l'une des revendications 1 à 3, **caractérisé en ce que** ledit agent tensioactif non-ionique comportant du silicium est un agent tensioactif de type organosiloxane répondant à la formule II et/ou à la formule III ou un agent tensioactif de type organo-carbosilane répondant aux formules IV, V et/ou à la formule VI 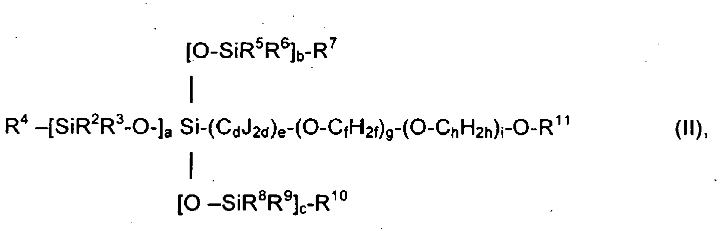 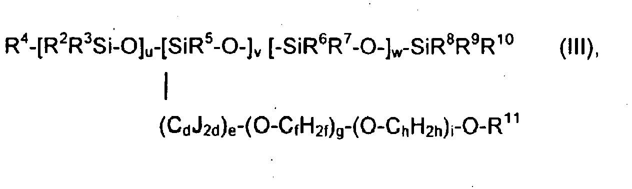 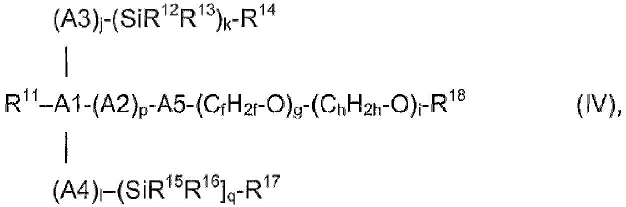 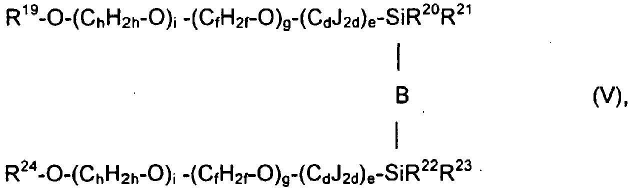
R¹⁹-O-(CₕH₂ₕ-O)ᵢ-(C_{f}H_{2f}-O)_{g}-(C_{d}J_{2d})ₑ-SiR²⁰R²¹R²² (VI)
dans lesquelles R², R³, R⁴, R⁵, R⁶, R⁷, R⁸, R⁹ et R¹⁰ signifient, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant complètement ou partiellement fluorés, s'agissant préférentiellement d'alkyle ou d'alcényle et notamment d'alkyle en C₁-C₆,
a, b, c et w signifiant, indépendamment l'un de l'autre, des nombres entiers compris entre 0 et 100, de préférence entre 0 et 75, notamment entre 0 et 35 et, avec une préférence particulière, entre 0 et 15,
V signifiant un nombre entier compris entre 1 et 100, de préférence entre 1 et 15 et, avec une préférence particulière, entre 1 et 6,
la somme de a, b et c étant comprise entre 1 et 300, de préférence entre 1 et 50, notamment entre 1 et 10 et, avec une préférence particulière, entre 1 et 3,
et la somme de v et w étant comprise entre 1 et 200, de préférence entre 2 et 90,
u étant 0 ou 1,
d étant un nombre entier compris entre 1 et 10, de préférence entre 1 et 6 et notamment entre 1 et 3,
J signifiant l'hydrogène ou le fluor, de préférence l'hydrogène,
e étant 0 ou 1,
f et h signifiant, indépendamment l'un de l'autre, des nombres entiers compris entre 2 et 6,
g et i étant, indépendamment l'un de l'autre, des nombres entiers compris entre 0 et 30, de préférence entre 0 et 15, la somme de g et i signifiant 1 à 60, de préférence 2 à 30, notamment 2 à 15,
R¹¹ étant l'hydrogène, alkyle, alcényle ou aryle, lesquels sont le cas échéant complètement ou partiellement fluorés, s'agissant préférentiellement d'hydrogène ou de méthyle,
R¹², R¹³, R¹⁴, R¹⁵, R¹⁶ et R¹⁷ signifiant, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant complètement ou partiellement fluorés, s'agissant préférentiellement d'alkyle ou d'alcényle et notamment d'alkyle en C₁-C₆,
k et q signifiant, indépendamment l'un de l'autre, 0 ou 1,
A1 signifiant carbone ou silicium,
A2, A3 et A4 étant, indépendamment l'un de l'autre, un groupe C_{d}J_{2d}, dans lequel J et d ont les significations définies ci-dessus,
j, p et l étant, indépendamment l'un de l'autre, 0 ou 1,
A5 signifiant un groupe de liaison divalent, s'agissant notamment de -O-, -CO-O- ou de -CO-,
R¹⁸ étant l'hydrogène, alkyle, alcényle ou aryle, lesquels sont le cas échéant complètement ou partiellement fluorés, s'agissant préférentiellement d'hydrogène ou de méthyle,
R²⁰, R²¹, R²² et R²³ signifiant, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant complètement ou partiellement fluorés, s'agissant préférentiellement d'alkyle ou d'alcényle et notamment d'alkyle en C₁-C₆, BG étant un groupe de liaison divalent, et
R¹⁹ et R²⁴ étant, indépendamment l'un de l'autre, l'hydrogène, alkyle, alcényle ou aryle, lesquels sont le cas échéant complètement ou partiellement fluorés, s'agissant préférentiellement d'hydrogène ou de méthyle,
à conditions que, parmi les radicaux R², R³ et R⁴ et/ou les radicaux R⁵, R⁶ et R⁷ et/ou les radicaux R⁸, R⁹ et R¹⁰ et/ou les radicaux R¹⁵, R¹⁶ et R¹⁷ et/ou les radicaux R²⁰ et R²¹ et/ou les radicaux R²² et R²³ et/ou les radicaux R²⁰, R²¹ et R²², un seul puisse être de l'hydrogène,
f et h pouvant, au sein d'une même de la molécule, adopter différentes valeurs rentrant dans le cadre de la définition donnée.

6. Matériau d'empreinte dentaire selon la revendication 5, **caractérisé en ce que** ledit agent tensioactif non-ionique comportant du silicium et contenant au moins un radical (poly)oxyde d'alkylène est un composé répondant aux formules VII, VIII, IX ou X 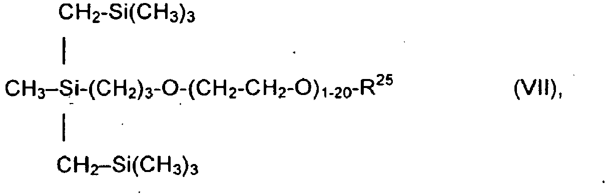 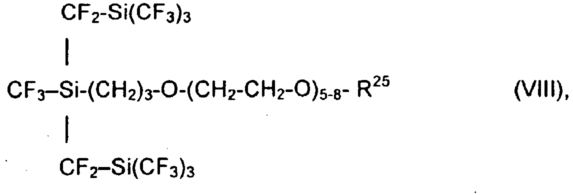 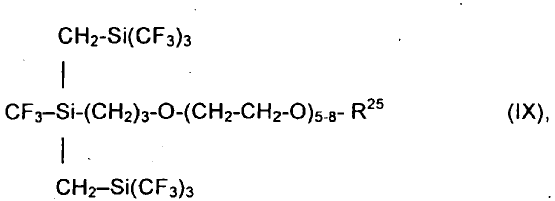 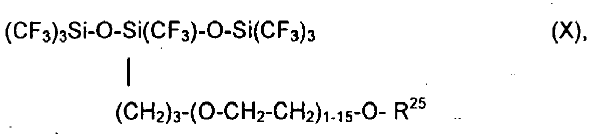 dans lesquelles R²⁵ est l'hydrogène, méthyle, éthyle, propyle ou butyle, s'agissant préférentiellement d'hydrogène ou de méthyle.

7. Matériau d'empreinte dentaire selon l'une des revendications 1 à 6, **caractérisé en ce qu'**il contient, en tant que constituant supplémentaire outre ledit agent tensioactif non-ionique comportant du silicium et ledit agent tensioactif fluoré de type non-ionique, un polyéther contenant des radicaux alcényle et/ou des radicaux alcynyle, et/ou un polyéther pourvu d'unités terminales de type hydroxyle et/ou aryloxy et/ou arylalkyloxy et/ou alkoxy.

8. Matériau d'empreinte dentaire selon l'une des revendications 1 à 6, **caractérisé en ce qu'**il contient, outre ledit agent tensioactif non-ionique comportant du silicium et ledit agent tensioactif fluoré de type non-ionique, un polyol en tant que constituant supplémentaire.

9. Matériau d'empreinte dentaire selon la revendication 8, **caractérisé en ce qu'**il contient, en tant que constituant supplémentaire, un polyéther contenant des radicaux alcényle et/ou des radicaux alcynyle, et/ou un polyéther pourvu d'unités terminales de type aryloxy et/ou arylalkyloxy et/ou hydroxyle et/ou alkoxy.

10. Matériau d'empreinte dentaire selon l'une des revendications 8 à 9, **caractérisé en ce que** ledit polyol est choisi dans le groupe des glucides, des alcools polyvinyliques, des di-, tri-, tétra-, penta- et/ou hexaols et des mélanges de deux ou de plusieurs de ces polyols.

11. Matériau d'empreinte dentaire selon la revendication 10, **caractérisé en ce que** ledit polyol est choisi dans le groupe constitué par les alcools polyvinyliques, les polysaccharides, le triméthylolpropane, le pentaérythritol, le dipentaérythritol, le glycérol, l'allyloxy-1,2-propanediol, le 2-méthyl-2,4-pentanediol, le triméthylolpropanallyléther, le décanediol, le nonanediol, l'octanediol, l'heptanediol, le pentanediol, le butanediol, l'éthanediol, le fructose, le glucose et les mélanges de deux ou de plusieurs de ces polyols, s'agissant notamment de glycérol.

12. Matériau d'empreinte dentaire selon la revendication 7, **caractérisé en ce que** ledit polyéther contenant des radicaux alcényle est un composé de formule XII et ledit polyéther pourvu d'unités terminales de type hydroxyle et/ou alkoxy est un composé de formule XIII
CH₂=CH-CH₂-O-(Cₙ₂H₂ₙ₂-O)ₘ₉-CH₂-CH=CH₂ (XII),
R³¹-O-(Cₙ₂H₂ₙ₂-O)ₘ₉-R³² (XIII).
dans lesquelles n2 signifie un nombre entier compris entre 2 et 8, de préférence entre 2 et 4, m9 est un nombre entier compris entre 3 et 70 000, de préférence entre 10 et 2 500,
R³¹ et R³² signifient, indépendamment l'un de l'autre, l'hydrogène ou alkyle en C₁-C₆, notamment l'hydrogène et/ou méthyle, éthyle ou propyle, et R³¹, R³², n2 et m9 pouvant, au sein d'une même molécule, être différents dans le cadre des définitions données.

13. Matériau d'empreinte dentaire selon l'une des revendications 1 à 12, **caractérisé en ce que** le rapport de poids entre ledit agent tensioactif comportant du silicium et ledit agent tensioactif fluoré est compris entre 100 : 1 et 1 : 100, de préférence entre 50 : 1 et 1 : 50, avec une préférence particulière entre 10 : 1 et 1 : 10 et, avec une préférence toute particulière, entre 5 : 1 et 1 : 5.

14. Matériau d'empreinte dentaire selon l'une des revendications 1 à 13, **caractérisé en ce qu'**il s'agit d'un matériau d'empreinte dentaire à plusieurs constituants de type organopolysiloxane subissant une réticulation par réaction d'addition, lequel contient les constituants A et B,
a) le constituant A étant un organopolysiloxane pourvu d'au moins deux groupes éthyléniquement insaturés et un catalyseur d'hydrosilylation,
b) le constituant B étant un polysiloxane organohydrogéné, et
c) au moins un des constituants A et/ou B contenant un agent tensioactif non-ionique comportant du silicium et/ou un agent tensioactif fluoré de type non-ionique, et les constituants A et B présentant une composition telle que, suite au processus de mélange des constituants A et B, on obtient une combinaison de l'agent tensioactif non-ionique comportant du silicium et de l'agent tensioactif fluoré de type non-ionique.

15. Matériau d'empreinte dentaire selon l'une des revendications 1 à 14, **caractérisé en ce qu'**il s'agit d'un matériau d'empreinte dentaire à plusieurs constituants de type organopolysiloxane subissant une réticulation par réaction de condensation, lequel contient les constituants C et D,
d) le constituant C contenant un organopolysiloxane pourvu d'au moins deux groupes hydroxyle,
e) le constituant D contenant un ester d'acide silicique, un polyester d'acide silicique et/ou un organopolysiloxane pourvu d'au moins deux groupes alkoxy ainsi qu'un catalyseur de condensation, et
f) au moins un des constituants C et/ou D contenant un agent tensioactif non-ionique comportant du silicium et/ou un agent tensioactif fluoré de type non-ionique, et les constituants C et D présentant une composition telle que, suite au processus de mélange des constituants C et D, on obtient une combinaison de l'agent tensioactif non-ionique comportant du silicium et de l'agent tensioactif fluoré de type non-ionique.

16. Matériau d'empreinte dentaire selon l'une des revendications 1 à 14, **caractérisé en ce qu'**il s'agit d'un matériau d'empreinte dentaire à plusieurs constituants de type polyéther contenant des radicaux alcényle subissant une réticulation par réaction d'addition, lequel contient les constituants E et F,
g) le constituant E contenant un catalyseur de réticulation,
h) le constituant F contenant un polyéther susceptible d'être réticulé et comportant des radicaux alcényle, ainsi qu'un polysiloxane organohydrogéné et/ou un polyéther de type SiH, et
i) au moins un des constituants E et/ou F contenant un agent tensioactif non-ionique comportant du silicium et/ou un agent tensioactif fluoré de type non-ionique, et les constituants E et F présentant une composition telle que, suite au processus de mélange des constituants E et F, on obtient une combinaison de l'agent tensioactif non-ionique comportant du silicium et de l'agent tensioactif fluoré de type non-ionique.

17. Matériau d'empreinte dentaire selon l'une des revendications 1 à 14, **caractérisé en ce qu'**il s'agit d'un matériau d'empreinte dentaire à plusieurs constituants de type polyéther contenant des radicaux alkylsilyle, lequel contient les constituants G et H,
j) le constituant G contenant un polyéther susceptible d'être réticulé et comportant des radicaux alkoxysilyle,
k) le constituant H contenant de l'eau, et
1) au moins un des constituants G et/ou H contenant un catalyseur ainsi qu'un agent tensioactif non-ionique comportant du silicium et/ou un agent tensioactif fluoré de type non-ionique, et les constituants G et H présentant une composition telle que, suite au processus de mélange des constituants G et H, on obtient une combinaison de l'agent tensioactif non-ionique comportant du silicium et de l'agent tensioactif fluoré de type non-ionique.

18. Matériau d'empreinte dentaire contenant des polyéthers susceptibles d'être réticulés et présentant, en tant que groupes susceptibles d'être réticulés ou groupes susceptibles d'être réticulés par réaction de métathèse impliquant une ouverture de cycle, des radicaux alkoxysilyle, des radicaux aziridino, des radicaux dérivés d'un acide carboxylique éthyléniquement insaturés ou des radicaux alcényle, et un agent tensioactif fluoré de type non-ionique lequel présente au moins un radical hydrocarboné perfluoré ou partiellement fluoré qui est relié, à travers un atome d'oxygène, un groupe amino, un groupe céto, un groupe ester d'acide carboxylique, un groupe ester d'acide phosphorique, un groupe amide d'acide carboxylique et/ou un groupe amide d'acide phosphorique, à un radical de (poly)oxyde d'alkylène, un radical de glucide, un radical aliphatique polyhydroxylé ou à un radical hétérocyclique contenant de l'azote, ou lequel comporte au moins un radical hydrocarboné partiellement fluoré ou perfluoré et au moins un radical aminoxyde.

19. Matériau d'empreinte dentaire selon la revendication 18, **caractérisé en ce que** ledit agent tensioactif fluoré est un agent tensioactif fluoré de type non-ionique comportant au moins un radical (poly)alkoxylène et étant constitué d'un radical hydrocarboné partiellement fluoré ou perfluoré qui est relié, à travers un atome d'oxygène ou un groupe ester en tant que groupe de liaison, à un radical (poly)alkoxylène.

20. Matériau d'empreinte dentaire selon la revendication 18, **caractérisé en ce qu'**il comporte, en tant que groupes susceptibles d'être réticulés, des polyéthers susceptibles d'être réticulés et pourvus de radicaux alkoxysilyle ou de radicaux aziridino, ou qu'il comporte des polyéthers susceptibles d'être réticulés qui présentent des groupes susceptibles d'être réticulés par réaction de métathèse impliquant une ouverture de cycle.

21. Matériau d'empreinte dentaire selon les revendication 18 à 20, **caractérisé en ce qu'**il contient, en outre, au moins un agent tensioactif non-ionique comportant un groupe contenant du silicium, avec une masse moléculaire inférieure à 6 000 g/mol.

22. Matériau d'empreinte dentaire selon l'une des revendications 18 à 21, **caractérisé en ce qu'**il contient, en tant que constituants supplémentaires, des polyols et/ou des polyéthers contenant des radicaux alcényle et/ou des radicaux alcynyle, et/ou des polyéthers pourvus d'unités terminales de type hydroxyle et/ou alkoxy.

23. Matériau d'empreinte dentaire selon l'une des revendications 18 à 22, **caractérisé en ce qu'**il s'agit d'un matériau d'empreinte dentaire à plusieurs constituants, lequel contient les constituants I et J,
m) le constituant I contenant un polyéther susceptible d'être réticulé et comportant des radicaux alkoxysilyle ou des radicaux aziridino ou un polyéther contenant des groupes susceptibles d'être réticulés par réaction de métathèse impliquant une ouverture de cycle,
n) le constituant J contenant un catalyseur, et
o) au moins un des constituants I et/ou J contenant un agent tensioactif fluoré de type non-ionique et, le cas échéant, un agent tensioactif non-ionique comportant du silicium, les constituants I et J présentant une composition telle que, suite au processus de mélange des constituants I et J, on obtient une combinaison de l'agent tensioactif non-ionique comportant du silicium et de l'agent tensioactif fluoré de type non-ionique.

24. Matériau d'empreinte dentaire selon l'une des revendications 1 à 14, **caractérisé en ce qu'**il contient un éther de polyalkylène qui est susceptible d'être réticulé et qui comporte des radicaux dérivés d'acide acrylique et/ou d'acide méthacrylique, un initiateur qui peut être activé chimiquement ou par un rayonnement ainsi qu'un agent tensioactif non-ionique qui comporte du silicium et un agent tensioactif fluoré de type non-ionique.

25. Matériau d'empreinte dentaire selon l'une des revendications 1 à 14, **caractérisé en ce qu'**il s'agit d'un matériau d'empreinte dentaire à plusieurs constituants de type polyéther lequel contient des polyéthers, des polysiloxanes et/ou des caoutchoucs de synthèse comportant des groupes susceptibles d'être réticulés par une polymérisation de type métathèse impliquant une ouverture de cycle (ROMP), et lequel contient les constituants K et L,
p) le constituant K contenant des polyéthers, des polysiloxanes et/ou des caoutchoucs de synthèse comportant des groupes susceptibles d'être réticulés par ROMP,
q) le constituant L contenant un catalyseur de réticulation ROMP, et
r) au moins un des constituants K et/ou L contenant un agent tensioactif non-ionique comportant du silicium et/ou un agent tensioactif fluoré de type non-ionique, et les constituants K et L présentant une composition telle que, suite au processus de mélange des constituants K et L, on obtient une combinaison de l'agent tensioactif non-ionique comportant du silicium et de l'agent tensioactif fluoré de type non-ionique.

26. Matériau d'empreinte dentaire selon l'une des revendications 1 à 25, **caractérisé en ce qu'**il contient des charges, de préférence dans une proportion globale comprise entre 0,01 et 80 % en poids, avec une préférence particulière entre 0,05 et 75 % en poids et, avec une préférence toute particulière, entre 0,1 et 70 % en poids, par rapport à l'intégralité dudit matériau d'empreinte dentaire.

27. Matériau d'empreinte dentaire selon l'une des revendications 1 à 26, **caractérisé en ce qu'**il contient un ou plusieurs des additifs suivants : sels à effet tampon, agents d'absorption d'humidité, agents texturants pour pâtes, d'autres agents tensioactifs, principes actifs, plastifiants, substances permettant une lecture optique, arômes et/ou parfums, substances permettant de réaliser un diagnostic, agents de fluoration, agents blanchissants, agents de désensibilisation, agents de pontage d'adhésion, colorants, indicateurs, agents stabilisants (antioxydants) ainsi que des substances antibactériennes.

28. Mélanges pouvant être obtenus en mélangeant les matériaux d'empreinte dentaire selon l'une des revendications 1 à 27.

29. Matériau d'empreinte durci, pouvant être obtenu en soumettant les matériaux d'empreinte dentaire selon l'une des revendications 1 à 28 à un processus de durcissement.

30. Utilisation d'un mélange d'un agent tensioactif non-ionique comportant du silicium, lequel comporte au moins un radical de (poly)oxyde d'alkylène et présente une masse moléculaire inférieure à 6 000 g/mol, et d'un agent tensioactif fluoré de type non ionique lequel présente au moins un radical hydrocarboné partiellement fluoré ou perfluoré qui est relié, à travers un atome d'oxygène, à un radical de (poly)oxyde d'alkylène, pour préparer des matériaux d'empreinte dentaire élastomères, **caractérisée en ce que** l'on met en oeuvre, en tant qu'agent tensioactif fluoré de type non-ionique, un composé de la formule suivante :
F-(CF₂-CF₂)₁₋₇-CH₂-CH₂-(C)-CH₂-CH₂)₁₋₁₅-OH

31. Utilisation d'un mélange selon la revendication 30, **caractérisée en ce que** ce dernier contient, en outre, de l'éther de polyalkylène pourvu d'unités terminales d'alcényle, d'alcynyle, d'alkyloxy, d'aryloxy, d'arylalkyloxy et/ou d'hydroxyle.

32. Utilisation d'un mélange selon la revendication 30, **caractérisée en ce que** ce dernier contient en outre du polyol.
